# EUROPEAN PATENT APPLICATION

(11) **EP 3 489 687 A1**
(43) Date of publication of application: **29.05.2019**
(21) Application number: 17204073.5
(22) Date of filing: 28.11.2017
(51) Int. Cl.: G01N 33/68

(54) **METHOD FOR DETERMINING A TISSUE INJURY AND REPAIR (TIAR) PROCESS ASSOCIATED WITH ABNORMAL FORMATION OF ENDOMETRIAL TISSUE**

(71) Applicant: Leyendecker, Gerhard, 64287 Darmstadt (DE)
(72) Inventor: Leyendecker, Gerhard, 64287 Darmstadt (DE)
(74) Representative: Hertin und Partner Rechts- und Patentanwälte PartG mbB

(57) **Abstract**

The invention relates to a method for determining the presence of a tissue injury and repair (TIAR) process in the uterus of a subject as a marker for the presence of a preliminary stage or increased risk of developing a medical disorder associated with abnormal formation of endometrial tissue, comprising determining a level of CXCL12 and/or CXCR4 in a sample from a subject.

## Description

The invention relates to a method for determining the presence of a tissue injury and repair (TIAR) process in the uterus of a subject as a marker for the presence of a preliminary stage or increased risk of developing a medical disorder associated with abnormal formation of endometrial tissue, comprising determining a level of CXCL12 and/or CXCR4 in a sample from a subject.

### BACKGROUND

Endometriosis is a non-malignant disease that affects many women predominantly during the reproductive period of life. With the cardinal symptoms, such as pelvic pain, bleeding disorders, and infertility, the disease has a tremendous impact on women's health.

Laparoscopy and the histological examination of the suspicious tissue constitute the standard procedure for the diagnosis of peritoneal endometriosis. It is often refrained from this invasive diagnostic method if not considered necessary, such as in a final sterility work-up and in situations of extreme complaints. In any case, the diagnosis is often delayed. Recent research utilizing imaging techniques, such as magnetic resonance imaging (MRI) and high resolution transvaginal sonography (TVS) has elucidated that pelvic endometriosis is significantly associated with uterine adenomyosis (Hricak et al., 1983; Reinhold et al.. 1998; Leyendecker et al., 1998; Kunz et al., 2000; Leyendecker et al., 2000; Kunz et al., 2005; Leyendecker et al., 2009; Leyendecker et al., 2015; Van den Bosch et al., 2015) These imaging techniques could serve as non-invasive methods to identify uterine adenomyosis and thereby, indirectly, the probable presence of pelvic endometriosis in these women. However, as soon as the diagnosis of uterine adenomyosis can be based on these methods a considerable and irreversible destruction of the reproductive function of the utero-tubal system may have already been taken place.

Recent results from carefully taken histories of women with endometrioses revealed that the majority of them suffered from primary dysmenorrhea (Chapron et al., 2011; Leyendecker et al., 2015). There are, however, only scant data available with respect to the prevalence of the development of endometriosis and adenomyosis in a population of women with primary amenorrhea (Burnett et al., 2005). Thus, a simple, specific and non-invasive diagnostic test would help to identify these women at risk. Therefore, it is an urgent *desideratum* for research to establish a method that allows the diagnosis of the disease in a very early phase of development.

Uterine adenomyosis is caused by auto-traumatisation of the non-pregnant uterus by its genuine mechanical functions during the menstrual cycle (Leyendecker et al., 1998, 2009, 2015). It has also been described following iatrogenic trauma (Meyer, 1930; Kindermann, 1988). Moreover, some girls develop endometriosis and adenomyosis in the late puberty before menarche (Marsh and Laufer, 2005; Ebert et al., 2009; Janssen et al., 2013).

The term 'Tissue Injury and Repair' (TIAR) was coined to characterize the pathophysiological process that is operative in the processes of acute and chronic injury. It involves the local production and paracrine action of estradiol (Leyendecker et al., 2009) that, as shown in animal experiments, in turn results in an increased tissue expression of stromal cell-derived factor 1 (SDF 1), also known as the chemokine CXCL12 at the site of the lesion. By the binding to the corresponding receptor, CXCR4 that is expressed on mesenchymal stem cells (MSC), CXCL12 causes an increased attraction of MSC to the wound (Bouzaffour et al., 2009; Du and Taylor, 2997; Hufnagel et al., 2015).

The TIAR-process and the CXCL12/CXCR4 interaction are *non-organ specific* phenomena. They play essential roles in organogenesis and tissue regeneration, in inflammation and in wound healing as well as in benign and malignant proliferative processes (Dotan et al., 2010; Teicher and Fricker, 2010; Boudot et al., 2011; Lander et al., 2012).

Yet, as will be delineated below, these molecular processes can be utilized as a novel tool for the early diagnosis of uterine adenomyosis and peritoneal endometriosis in young women that are at a high risk to acquire this disease entity.

CXCL12 has been suggested as a biomarker for endometriosis (WO 2016/011377), although no suggestion has been provided in the art to date that CXCL12/CXCR4 could be employed for determination of a TIAR process, or in the prognosis or detection of the early stage of a medical disorder associated with abnormal formation of endometrial tissue.

As such, the field of reproductive health is in significant need of markers that may be employed as early as possible in order to identify pathogenic processes before endometriosis develops.

### SUMMARY OF THE INVENTION

In light of the prior art the technical problem underlying the present invention is to provide alternative and/or improved means for determining an early stage of abnormal formation of endometrial tissue and/or for determining subjects at risk of developing a medical disorder associated with abnormal formation of endometrial tissue.

This problem is solved by the features of the independent claims. Preferred embodiments of the present invention are provided by the dependent claims.

The invention therefore relates to a method for determining the presence of a tissue injury and repair (TIAR) process in the uterus of a subject, comprising:
a) providing a sample of said patient, and
b) determining a level of CXCL12 and/or CXCR4 in said sample,
c) wherein the level of CXCL12 and/or CXCR4 correlates with the presence of tissue injury and repair (TIAR) processes in the uterus of a subject.

The invention therefore relates further to a method for determining the presence of a tissue injury and repair (TIAR) process in the uterus of a subject as a marker for the presence of a preliminary stage or increased risk of developing a medical disorder associated with abnormal formation of endometrial tissue, comprising:
a) providing a sample of said patient, and
b) determining a level of CXCL12 and/or CXCR4 in said sample,
c) wherein the level of CXCL12 and/or CXCR4 correlates with the presence of tissue injury and repair (TIAR) processes in the uterus of a subject and/or the presence of a preliminary stage or increased risk of developing a medical disorder associated with abnormal formation of endometrial tissue.

As described in more detail below, the tissue injury and repair (TIAR) process is a non-organ specific biological phenomenon that is employed as a marker for the early stage or increased risk of developing abnormal formation of endometrial tissue. It was a surprising aspect of the invention that determination of CXCL12 or its receptor CXCR4 could be used to determine the TIAR process in a subject at an early stage of or prior to abnormal formation of endometrial tissue.

Although CXCL12 has been suggested as a biomarker for endometriosis (WO 2016/011377), no suggestion has been provided in the art to date that CXCL12/CXCR4 could be employed for determination of a TIAR process in the prognosis or detection of the early stage of a medical disorder associated with abnormal formation of endometrial tissue. The present invention enables the analysis to be conducted at time points earlier than were previously thought possible, thereby representing an improvement over the prior art.

The present invention therefore represents a novel and surprising solution to the problem of providing molecular markers for a preliminary stage or an increased risk of developing a medical disorder associated with abnormal formation of endometrial tissue.

The present invention therefore relates to a method for the prognosis of a medical disorder associated with abnormal formation of endometrial tissue.

The present invention therefore relates to a method for the diagnosis of an early or preliminary stage of a medical disorder associated with abnormal formation of endometrial tissue.

Endometriosis (archimetrose) is a benign proliferative disease associated with an increased accumulation of archimetral (endometrial) stem cells (eg progenitor cells or mesenchymal stem cells, MSC) (a) in the subbasal stroma of the eutopic endometrium and (b) in the stroma of ectopic sites of endometriotic growth. The increased attachment of stem cells represents an imitation of archimetric embryology, but represents a misguided healing process after acute and/or chronic mechanical trauma. This injury initially occurs either as auto-traumatization by one's own mechanical activity of the non-gravid uterus or as iatrogenic trauma on the level of the eutopic endometrial stroma (endometrial-myometrial junction). This chronic mechanical irritation maintains a local traumatization which leads to infiltrating processes. Auto-traumatization of the uterus and the attachment of stem cells requires the functional effects of estradiol, which is mediated via the estradiol receptor alpha (ER-alpha) and the estradiol receptor beta (ER-beta; activation of CXCL12).

In the meaning of the present invention, an "early" or "preliminary stage" of a medical disorder associated with abnormal formation of endometrial tissue is characterized by a uterine auto-traumatization.

Such a traumatization can be determined via increased levels of bone marrow stem cells being recruited to the uterus in comparison to healthy controls.

In some embodiments, an "early" or "preliminary stage" of a medical disorder associated with abnormal formation of endometrial tissue may also be defined by the presence of endometrial tissue growth outside the uterus, but before cysts (endometriomas) occur.

An auto-traumatization in an early stage of disease can also be determined by increased levels of estradiol, or the ER-alpha or ER-beta receptors, in the uterus of subjects compared to healthy controls.

The early or preliminary stage of such a disease can also be defined by increased levels of peristaltic activity in the uterus, without necessarily having increased pathologic endometriotic growth.

The method may therefore be carried out on samples from subjects who exhibit early indications of an oncoming endometriosis, as may be defined, for example, by the presence of a local "micro-traumatization" of a part of the endometrium, wherein the damage or traumatization may in some embodiments be localized to a sub-region of the uterus, or to a sub-group of cells of the endometrium.

In some embodiments, such additional parameters may be measured with CXCL12 and/or CXCR4 in combination with the method of the present invention, or such measurements may be replaced by the method of the present invention. Such parameters for the early or preliminary phase may also be used as a definition of the patient group, without limitation to necessarily having tested the patients of the method for these parameters.

Conducting an assay for CXCL12 and/or CXCR4 levels at such an early stage is advantageous, as it enables subsequent treatment before the disease has been established.

According to preferred embodiments of the invention, the patient or patient group to be analyzed represents a novel aspect of the invention. For example, the analysis of patients prior to developing endometriosis or other diseases associated with abnormal formation of endometrial tissue represents a preferred embodiment of the invention, which has not previously been suggested in the prior art.

In one embodiment, the recruitment of bone marrow-derived stem cells, preferably mesenchymal stem cells, is evident in the uterus of said subject. MSCs can be detected according to the markers described in more detail below. The MSC recruitment is one characteristic of the TIAR process that indicates the likely onset of a medical disorder associated with abnormal formation of endometrial tissue.

In one embodiment of the invention, the subject exhibits symptoms of dysmenorrhea. Symptoms of dysmenorrhea include but are not limited to painful periods, menstrual cramps, or pain during menstruation. Dysmenorrhea, typically occurs around the time that menstruation begins and symptoms typically last less than three days. The pain is usually in the pelvis or lower abdomen. Other symptoms may include back pain, diarrhea, or nausea.

Carrying out the method of the present invention in women with symptoms of dysmenorrhea is a surprising and beneficial aspect of the invention. The present invention enables this patient group to be assessed for risk of an endometriosis or adenomyosis at a very early stage, before other symptoms occur, or before fully developed disease occurs.

In one embodiment, the medical disorder associated with abnormal formation of endometrial tissue is endometriosis.

In one embodiment, the medical disorder associated with abnormal formation of endometrial tissue is adenomyosis.

The above two medical conditions are defined primarily by the abnormal formation of endometrial tissue. Endometriosis is a condition in which the tissue that normally lines the uterus grows outside the uterus. Its primary symptoms include pain and infertility. Most often pain is associated with the ovaries, fallopian tubes, and tissue around the uterus and ovaries, but can occur elsewhere. Adenomyosis is a medical condition characterized by the abnormal presence of endometrial tissue (the inner lining of the uterus) within the myometrium (the thick, muscular layer of the uterus). In contrast, when endometrial tissue is present entirely outside the uterus, it represents a similar but distinct medical condition called endometriosis. The two conditions are found together but may also occur independently.

In one embodiment of the invention, the level of CXCL12 and/or CXCR4 positively correlates with the presence of a preliminary stage or increased risk of developing a medical disorder associated with abnormal formation of endometrial tissue. The positive correlation refers to an increasing risk or likelihood of having or developing said disease with increasing levels of said markers. In this context a cut-off or threshold level may be employed, or comparisons may be made to reference levels of healthy subjects.

In one embodiment of the invention, the sample comprises or consists of blood.

In one embodiment of the invention, the sample comprises or consists of menstrual fluid.

In one embodiment of the invention, the sample comprises a cervical test specimen or cervical test smear.

In a preferred embodiment, the sample is a menstrual fluid sample and said levels of CXCL12 and/or CXCR4 are elevated in said menstrual blood sample. In some embodiments, the levels of CXCL12 and/or CXCR4 are elevated in comparison to a peripheral blood sample, or to levels obtained from a sample of a healthy control subject.

In one embodiment of the invention, the determination of an increased risk of developing a medical disorder associated with abnormal formation of endometrial tissue is conducted prior to the occurrence of endometriosis and/or adenomyosis in said subject. This represents a particularly preferred embodiment of the invention, in which the prognosis or risk assessment can be carried out, and represents an entirely novel method compared to previous methods described in the art.

In further embodiments of the invention, the subject has been menstruating for a period of 3 years or less, preferably 2 years or less, and is preferably of an age of 12, 13, 14, 15, 16 or 17. The method may therefore be characterized by the patient group to be analyzed. It is particularly beneficial to conduct the method in subjects of a relatively young age who have not been menstruating for long periods of time.

The present method therefore also enables early treatment of the disease, thereby preventing establishment of the disease and its severe effects, such as infertility. Treatments may include but are not limited to progesterone or progestins (which counteract estrogen and inhibit the growth of the endometrium), avoiding products with xenoestrogens (which have a similar effect to naturally produced estrogen and can increase growth of the endometrium), hormone contraception therapy (such as in the form of oral contraceptives), treatment with danazol (danocrine) or gestrinone (which are suppressive steroids with some androgenic activity) or gonadotropin-releasing hormone (GnRH) agonists (which may decrease hormone levels) with or without estrogen add-back.

These treatment modalities were based on the concept of endometriosis constituting an estrogen dependent disease, however, without having taken the mechanical functions of the non-gravid uterus into consideration. The estradiol dependence certainly also holds true for the new concept of tissue injury and repair (TIAR) described herein, because the traumatizing functions, such as uterine peristalsis and neometral menstrual contractions, are essentially estradiol driven. The uncovering of the TIAR-concept and its relationship to CXCL12, now enables alternative and novel treatment modalities that are directed against the uterine hypercontractility.

As such, the present invention is associated with the additional advantage of enabling a new line of therapeutic approaches directly targeting uterine peristalsis and neometral menstrual contractions in the treatment and/or prevention of medical conditions associated with the abnormal formation of endometrial tissue.

Such treatments encompass medical interference with the hypothalamic-pituitary-ovarian axis, resulting for example in low-grade hypothalamic amenorrhea with lowered but still sufficient peripheral estradiol levels that still allow for normal tissue regeneration, such as on the level of the mucosa of the genital tract and the bone.

Another option relates to the interference with the oxytocin-oxytocin-receptor (OT/OTR) system that is directly controlling the uterine contractility. For example, approaches may be employed based on contraction suppression, for example such as Atosiban, which is an inhibitor of the hormones oxytocin and vasopressin. It is used as an intravenous medication as a labour repressant (tocolytic) to halt premature labor. Other Tocolytics (also called anti-contraction medications or labor suppressants), such as Terbutaline (Brethine), Ritodrine (Yutopar), Salbutamol (INN) or albuterol, Hexoprenaline (Gynipral) or Nifedipine (Procardia, Adalat), are alternative potential medications used to suppress contractions, that may directly target a pathological TIAR-related uterine peristalsis and neometral menstrual contractions. Painful contractions of the uterine muscle (similar to labor pains) are triggered by increased endometrial synthesis of prostaglandins, which appear in elevated amounts in the plasma and menstrual fluid of women with dysmenorrhea. Non-steroidal anti-inflammatory drugs, which have been used for years in arthritis, are effective prostaglandin inhibitors. Therefore, prostaglandin inhibitors may relieve dysmenorrhea in the majority of cases and lead to the treatment and/or prevention of medical conditions associated with the abnormal formation of endometrial tissue.

In some embodiments, the invention relates to a method for the treatment of medical disorder associated with abnormal formation of endometrial tissue comprising the method described herein in addition to subsequent administration of one or more therapeutic agents to a subject in need thereof.

### DETAILED DESCRIPTION OF THE INVENTION

Further preferred and non-limiting embodiments of the invention are provided in the detailed description of the invention below.

### Historical overview

With the beginning of the clinical and scientific interest in adenomyosis and endometriosis (Von Rokitansky,1860) many theories have been presented to provide an understanding of the pathogenesis and pathophysiology of this enigmatic disease entity. The initial *Wolffian duct theory* put forward by von Recklinghausen had, already shortly after its formulation (Von Recklinghausen,1896), to be replaced by the *Müllerian duct theory* that is considered valid until today (Cullen,1896; Kossmann1897). While many clinicians and scientists considered adenomyosis and endometriosis as a disease entity, particularly based on the Cullen's publications (Cullen, 1896; 1903; 1908; 1920), the focus of interest shifted to its peritoneal variety. In a contribution to a textbook Robert Meyer (1930) stated, in contrast to Cullen's findings (1920), that uterine adenomyosis is rarely associated with peritoneal endometriosis. In a large review the prevalence of endometriosis in adenomyosis ranged from about 10 to 80 % (Emge, 1962). Most of the reported data were probably based upon casual findings during surgery and not upon data collected in a specific study focusing on that issue. In any case, peritoneal endometriosis was increasingly considered as a separate disease entity. This view was re-enforced by Robert Meyer's theory of *coelomic metaplasia* (Meyer 1919) that was later on extended by proposing a 'secondary Müllerian system'. Robert Meyer did not defend his theory after a seemingly better one was proposed.

This was the theory of J. A. Sampson. Initially believing that peritoneal endometriosis would result from the *rupture of ovarian endometrioma,* (Sampson, 1922) he later proposed that peritoneal endometriosis is primarily *due to the menstrual dissemination of endometrial tissue into the peritoneal cavity*.(Sampson, 1927). There is no doubt, as can be derived from his own arguments in support of his theory, that he in fact was convinced that during normal menstrual bleeding ("menstrual reaction") vital endometrial cells would be disseminated into the peritoneal cavity. He observed this "menstrual reaction" in studying the bleeding from a recto-vaginal endometriotic lesion, of which he erroneously thought to be identical with that during normal menstrual desquamation. His theory was considered as plausible and, although its validity was doubted by some authorities in that field (Counseller, 1938; Philipp and Huber,; 1939), his terminology was accepted. Without doubt, the theory of *transtubal transplantation,* meaning the transtubal route, is valid until today.

Sampson admitted that many of his patients with peritoneal endometriosis also presented with uterine adenomyoma. However, he did not consider a pathophysiological share between these lesions. He believed that uterine adenomyosis is caused by lymphatic transmission thus ignoring Cullen's work. Furthermore, his understanding of peritoneal endometriosis being composed of only endometrial epithelium and stroma, a view that is erroneously shared by many scientists up to today, was in contrast to the histological description of peritoneal adenomyoma (Cullen, 1920) irrespective whether they constitute superficial or deeply infiltrating lesions (Anaf et al., 2000; Leyendecker et al., 2002, Barcena de Arellano et al, 2011). Moreover, he did not take into account that the menstrual bleeding in women with endometriosis might differ from that of normal women. His theory of retrograde menstruation to cause peritoneal endometriosis is continued to be quoted in scientific work. It appears, however, that this refers more to the transtubal transplantation of endometrial tissue *with* the menstrual bleeding constituting merely the *vehicle* for this transport into the peritoneal cavity, rather than to a normal menstruation with transtubal reflux of the blood. In any way, definition and terminology often lack precision as already mentioned by Robert Meyer (1930). Moreover, premenarcheal peritoneal endometriosis (Marsh and Laufer, 2005; Ebert et al, 2009; Janssen et al., 2013) suggests that other factors than just bleeding might at least in addition also be responsible for the transtubal transport of endometrial fragments into the peritoneal cavity.

Sampson's theory had a strong impact on the understanding of endometriosis that was later-on re-enforced by laparoscopy (Batt, 2011). Peritoneal endometriosis and uterine adenomyosis were considered distinct disease entities without any pathophysiological shares (Parazzini et al., 1997). In the German literature, however, until today, uterine adenomyosis was still considered as a part of the disease process (Philipp and Huber, 1839; Kindermann, 1988). The basis of this understanding and the reconciliation of the divergent views was provided by Ridley's apodictic and, in fact, unproved view that endometrium, where ever located, is inclined to invade subjacent tissue' (Ridley, 1968). Thus, peritoneal endometriosis and uterine adenomyosis could co-exist in parallel. Ronald Batt (2016; personal communication) characterized the situation that he had realized during his medical work in the US: "With respect to endometriosis, in research, teaching and clinical management adenomyosis did not play any role". Uterine adenomyosis that had initially addressed the interest to this disease entity fell into oblivion (Benagiano and Brosens, 2006).

During the last five decades, the clinical and scientific interest in endometriosis rose substantially. This is certainly owed to new methods available in the clinical management of the disease, such as laparoscopy and imaging techniques. The recognition of this disease, however, increased considerably in consequence of the dramatic change in the reproductive behavior of the society during this time. While in parous women the disease was often not diagnosed, with the postponing of child bearing endometriosis emerged as a disease of young non-parous women with an enormous impact on their lives.

Following the demonstration that transtubal 'retrograde' bleeding probably constitutes a normal phenomenon (Blumenkrantz et al., 1981; Halme et al., 1984) but only about 15 % of young women acquire peritoneal endometriosis further theories have been proposed that were still based on the concept of retrograde menstruation, such as menstrual outflow obstruction, early menarche resulting in an increased number of menstruations and menstrual cycle irregularities (Mahmood and Templeton, 1990).

Newer concepts were increasingly based on laboratory data, such as those obtained in histological and immunological studies and in molecular biology. It was proposed that peritoneal endometriosis constitutes *three different disease entities* with ovarian endometriosis resulting from retrograde menstruation, superficial peritoneal endometriosis from metaplasia and deeply infiltrating recto-vaginal endometriosis constituting Müllerian remnants (Nisolle and Donnez, 1997). Brosens and Brosens (2000) distinguished superficial from deeply infiltrating endometriosis. Superficial endometriosis would arise from retrograde menstruation and the deeply infiltrating variety would constitute adenomyosis.

Cellular and molecular phenomena known from immunological diseases suggested that immunological defects on the level of the peritoneum were responsible for the growth of endometrial tissue on peritoneal surfaces (Halme et al, 1984; Bartosik et al., 1984; Leiva et al., 1994; Han et al, 2015). Peritoneal endometriotic lesions result from auto-transplantation of endometrial fragments and thus should, primarily, not cause immunological reactions. The immunological phenomena observed may, therefore, not be directed against the transplanted tissue but may rather constitute a reaction towards the cyclic changes of the implanted tissue, such as bleeding and cellular debris resulting from cyclic phenomena and cannot be externalized. It is therefore not justified to consider endometriosis primarily as an *immunological disease.*

In maintaining the view of retrograde menstruation, it was proposed, on the basis of results obtained in molecular biology, that peritoneal endometriosis resulted from the desquamation of *intrinsically or epigenetically altered endometrium* (Aghajanova et al., 2009; Bulun, 2009). These alterations in molecular biology observed, both, on the level of the endometrium and on the endometriotic lesions were of importance in the further understanding of the disease process. These views, however, did not take structural components in the disease process into consideration, such as the topography of the lesions within the uterine cavity as well as the zonal layers of the endometrium involved. This also holds true for recent publications, in that findings in biopsies taken from eutopic endometrium of affected women are considered as representative for the whole endometrium. Moreover, no causal relationship of uterine (auto)-traumatisation to the development of adenomyosis as the primary lesion is considered (Sakr et al., 2014; Wang et al., 2015; Plucino and Taylor, 2016; Moridi et al., 2017).

During the last two decades, the view had been advanced that peritoneal endometriosis developed primarily on the level of the uterus (Leyendecker et al., 1998, 2009). It can, without doubt, be considered as a *paradigmatic change* (Alabiso et al., 2016), first, to relate the development of the disease to basic processes of wound healing and, second, that the injury is caused by essentially physiological mechanical functions of the non-pregnant uterus (Leyendecker et al, 1998, 2009, 2015;). For the understanding of such processes, however, the normal morphology of the non-pregnant uterus and its physiological mechanical functions had first to be elucidated and defined.

### Uterine morphology and the mechanical functions of the non-pregnant uterus

The uterus was long considered to be a quiescent organ that becomes mechanically active only during the expulsion of the conceptus. With the advent of high resolution transvaginal ultrasound (TVS), hysterosalpingoscintigraphy (HSSG) and measurement of intrauterine pressure it became apparent that the uterus is a mechanically very active organ throughout the menstrual cycle. These functions consist of the archimyometrial peristaltic activity for directed sperm transport as well as of the neometral contractions for the discharge of the menstrual debris at the end of a non-conception cycle (Leyendecker et al., 2012; 2015).

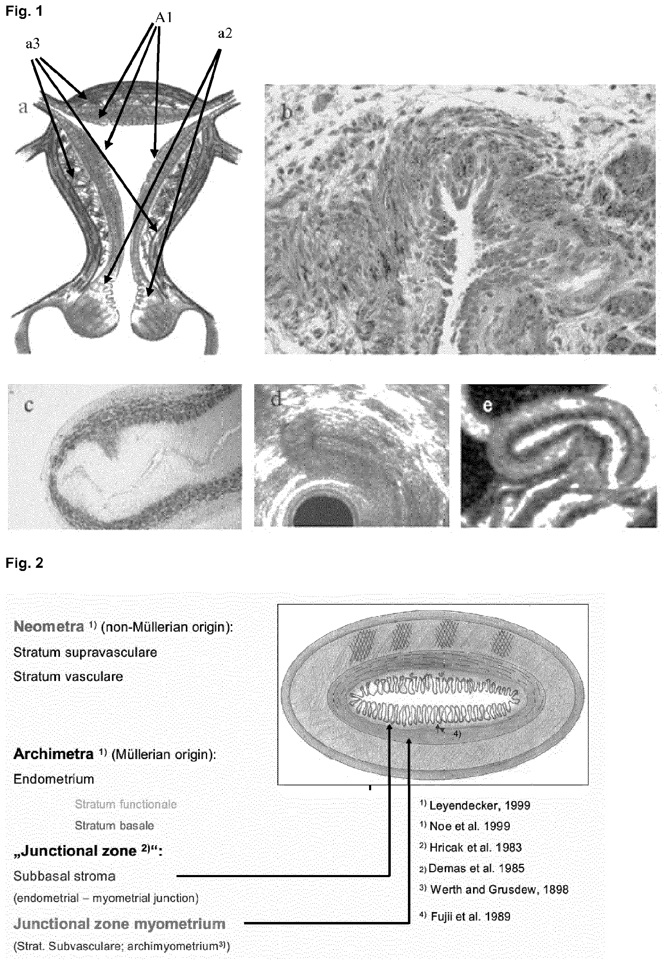

The uterine corpus is composed of two organs, the neometra and the archimetra, that differ from each other with respect to embryology, structure and functions during the reproductive process (Werth and Grusdew, 1898; Leyendecker et al., 1998; Leyendecker et al. 199; Noe et al. 1999) (Figure 1).

The archimetra is phylogenetically and ontologically the oldest uterine structure. It is derived from the Müllerian ducts and it composed of the glandular endometrium and the glandular as well as the sub basal stroma, also termed the endometrial-myometrial junction, and the stratum subvasculare of the uterus as the muscular layer. This *primordial uterus* develops early during pregnancy. The archimetra constitutes the adult representation of the primordial uterus (Werth and Grusdew, 1898; Leyendecker et al., 1999; Noe et al., 1999).

The neometra is phylogenetically a younger uterine structure (Noe et al, 1999) and develops late during pregnancy, sometimes even after birth (Werth and Grusdew, 1898). The neometra is composed of two muscular layers, the stratum supravasculare with a longitudinal direction of muscular fibers and the stratum vasculare of the myometrium with a three-dimensional network of short muscular bundles. The neometra is of non-Müllerian origin. The two muscular layers develop from the connective tissue of the peritoneal serosa and the connective tissue of the vessels of the uterine stratum vasculare for example present in the rodents, respectively.

Because the stratum subvasculare is the ontological oldest muscular structure of the uterus, Werth and Grusdew (1898) coined the denomination archimyometrium. This prompted us to suggest the terms *Archimetra* and *Neometra* for the Müllerian and non-Müllerian parts of the human uterus, respectively (Leyendecker et a., 1998; Leyendecker et al., 1999, Noe at al., 1999) (Figure 2).

The archimetra is, more than the neometra, characterized by morphological and functional changes during the menstrual cycle. These changes pertain to all structures of the archimetra, such as the glandular epithelium and the stroma of the endometrium, the endometrial-myometrial junction as well as the archimyometrium.

### Endometrium

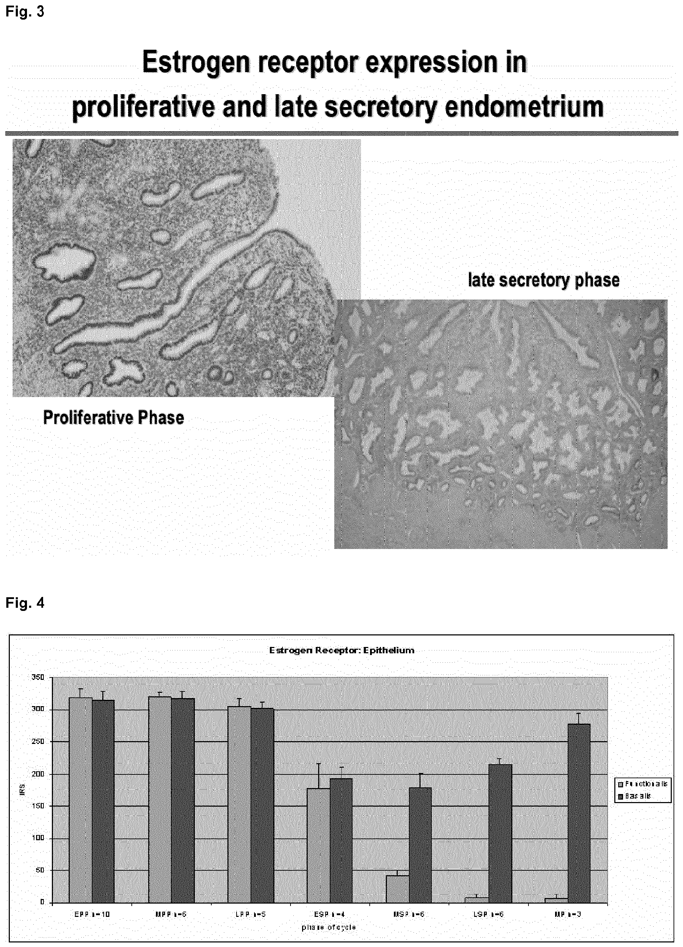

A tripartite or quadripartite horizontal zonation exists in the human endometrium and in that of menstruating subhuman primates. The endometrial zones are microenvironments that differ by position, ultra structural differentiation and mitotic activity during the cycle (Kaiserman-Abramof and Padykula, 1987; Padykula *et al.,* 1989). During the secretory phase of the rhesus menstrual cycle the functionalis and the spongiosa are characterised by progesterone induced mitotic inhibition, while the basalis not only escapes from that inhibition but rather exhibits increasing mitotic activity towards the end of the secretory phase (Padykula *et al.,* 1989). It is reasonable to assume that these data are also pertinent to the human endometrium. Our data show that, in the human, proliferative phase ER and PR expression persist, though each at different levels, in the epithelium and/or the stroma of the basalis during the whole secretory phase even with an increase of the ER and PR expression during the late secretory phase. In the functionalis and in the spongiosa, however, the immunoreactive scores (IRS) of ER and PR expression decline progressively towards the end of the cycle. Thus, the basalis appears to constitute a highly vital endometrial compartment throughout the menstrual cycle, while most of the other layers are destined to cell death concomitant with the late luteal progesterone decline (Padykula *et al.,* 1989; Leyendecker et al., 2002) (Figure 3 and 4).

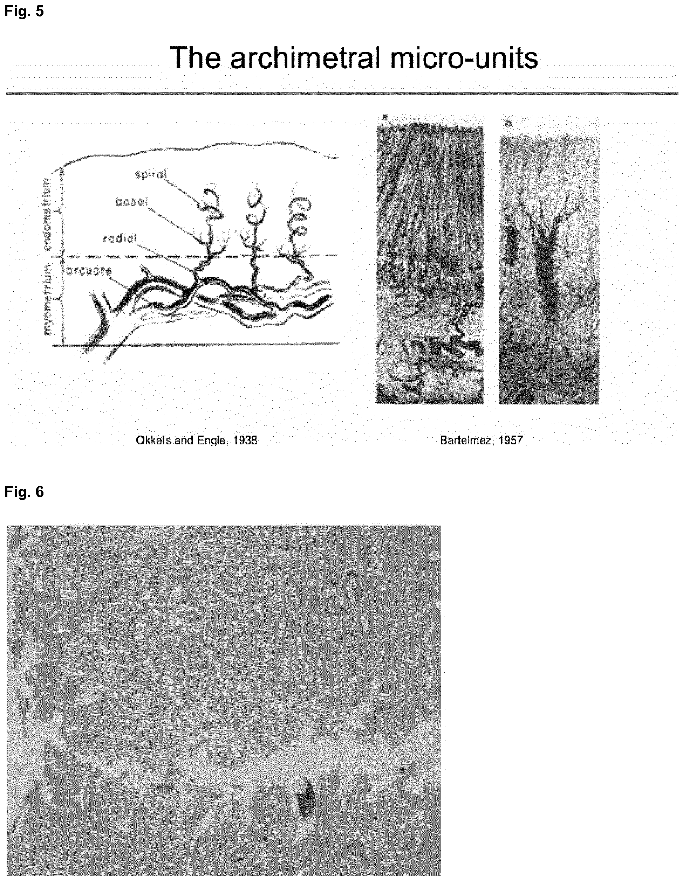

The blood supply of the endometrium is pertinent to its cyclic changes and function (Okkels and Engle, 1938; Bartelmez, 1957; Ramsey, 1989; Rogers, 1996) (Figure 5). The radial arteries branch off from the arcuate ones and perforate the archimyometrium to reach the basal layer of the endometrium. Basal arteries branching off from the radial artery supply the archimyometrium and the basal endometrium before the latter is coiled to form the spiral artery that extends from the upper basalis through the spongiosa into the lower functionalis. The spiral artery passes over into small arteries that supply the upper functionalis. This blood supply is of utmost importance for the level of endometrial desquamation at the end of the secretory phase: The earlier the small arteries branch off from the radial artery and the lower part of the spiral artery the less is the supply affected and reduced during the process of shrinkage of the functional endometrium.

It is of interest to note that only a single radial artery branching off from the arcuate artery provides, as a terminal vessel, the blood supply for a circumscribed segment that covers on the luminal surface an area of around 4 - 9 mm². There is no horizontal communication of the vascular systems between such neighbouring segments (Rogers, 1996). Therefore, such a segment extending from the archimyometrium to the luminal surface and supplied by only one radial artery could be denominated the *Hoxa-10-regulated archimetra micro- unit (AMU)* (Figure 5). Thus the archimetra is composed of many of such AMU. Further research has to elucidate and define the roles of these units in physiology and pathology, such as the propagation of the archimyometrial peristaltic activity in directed sperm transport, normal implantation and the regeneration of the endometrium after expulsion of the placenta (Guo et al., 2010) as well regenerative medicine (Liu et al., 2017) There is no doubt that these units are of importance in the development of uterine adenomyosis and its sequels, such as archimyometrial dysperistalsis (Leyendecker et al., 1996) and increased rates of miscarriage following artificial reproductive technology (Martinez-Conjure et al, 2011).

In healthy women the level of desquamation is localized within the spongiosa and probably controlled by MMPs that are up-regulated following ischemia of the functionalis in consequence of the compression of the spiral artery by the process of endometrial shrinking (Rogers, 1996) (Figure 6). This highly controlled process ensures that only functionalis is desquamated during menstruation (Brenner and Slayden, 1994; Osteen et al., 1994; Rudolph-Owen et al., 1998). As judged from histological criteria and the negative results obtained from attempts to culture menstrual debris of healthy women the desquamated tissue has to be considered as non-vital (Philipp und Huber. 1939; Leyendecker et al., 2002).

Due to the special blood supply that is derived from the radial arteries the lower spongiosa and all of the basalis escape menstrual desquamation. In addition, the estradiol receptors are not fully down regulated by progesterone. In contrast, the immunoreactive scores (IRS) increase after a short postovulatory decline to nearly late proliferative phase levels at the end of the luteal phase and during menstruation. Thus, the basalis constitutes, during menstruation and the beginning of the secretory phase, a highly vital tissue (Leyendecker et al., 2002) (Figure. 4).

The regeneration (proliferation) of the functionalis after menstruation does not arise from bone marrow derived mesenchymal *stem cells,* although such cells are certainly involved in the normal and continuous process of endometrial tissue regeneration. In all of the menstrual basalis the epithelial cells are stained positive for estradiol receptor (ER.) (Figure 3) Thus, these cells are already differentiated into Müllerian epithelial cells and therefore in a strict sense no longer endometrial or archimetral stem cells (ESC; ASC) that still could differentiate in all three archimetral components. Basal endometrium is constitutively positive for ER and PR; they more or less escape, other than the receptors of the functionalis layer progesterone-driven down-regulation (Leyendecker et al., 2002)). Recent studies have shown that CXCL12-tissue levels do not change significantly across the menstrual cycle. Around menstruation there was only a slight increase in CXCR labelled mesenchymal stem cells (MSC) (Laird et al., 2011). This concurs with the finding that a cyclic pattern imposed on rodents did not alter the content of stem cells in the basal endometrium (Gargett et al.,2016)

### Endometrial-myometrial junction

This thin layer of the archimetra is interposed between the endometrium and the archimyometrium and essentially constitutes the sub basal endometrial stroma. At the myometrial interface of the endometrial-myometrial junction cyclical metaplastic changes take place in that the stroma cells develop into fibro-muscular and muscular cells under the influence of progesterone and back into stromal cells during the proliferative phase of the cycle (Fujii et al., 1989).

The sub basal endometrial stroma and the stroma in the apical region of the endometrial glands are homing the MSC (Ibrahim et al., 2015). They are attracted via the vascular system to these sites because of the expression of CXCL12 in the glandular epithelium and they are the sources of the highly estrogen dependent continuous process of tissue regeneration (Ibrahim et al., 2015; Gargett et al., 2016).

### Archimyometrium

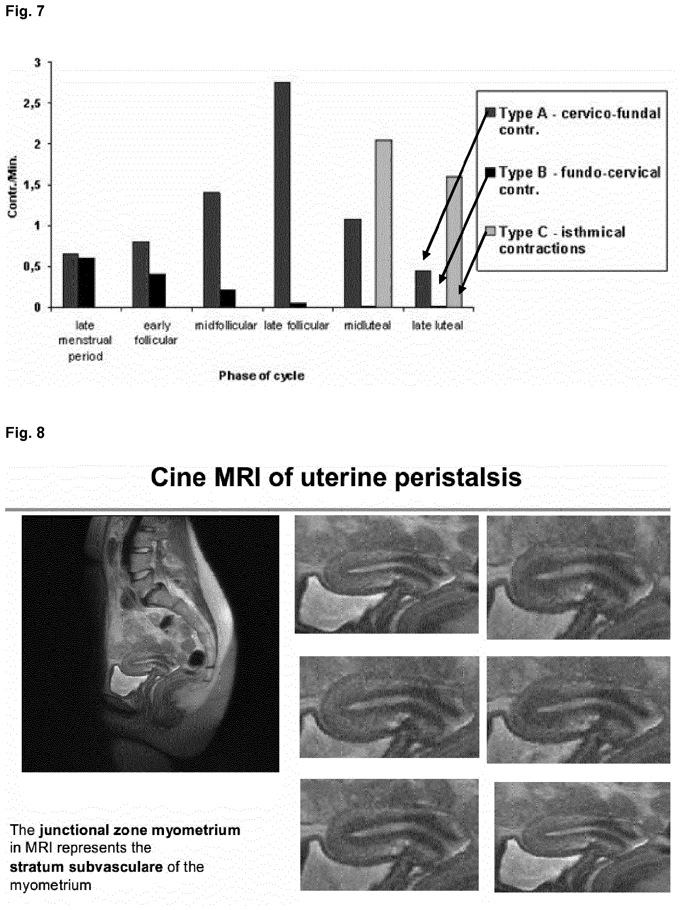

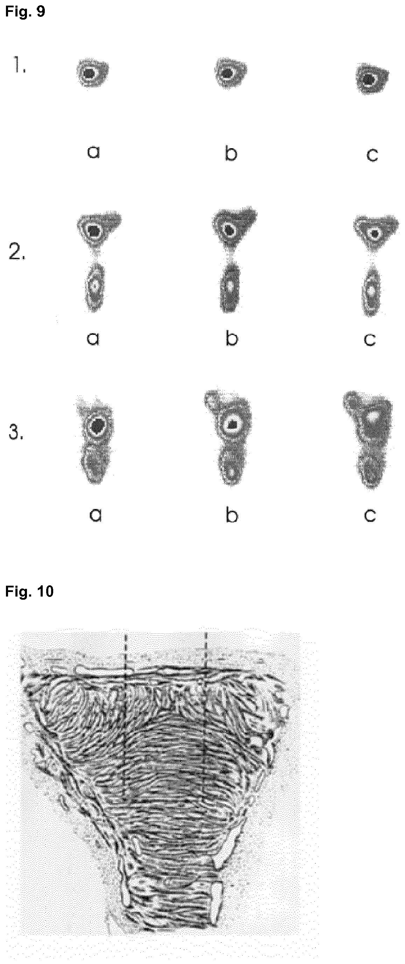

The archimyometrium develops from the Müllerian mesenchyme early during ontology (Werth and Grusdew, 1898). Data from TVS and HSSG have shown that the archimyometrium serves directed sperm transport from the external cervical os into the isthmical part of the tube on the side of the dominant ovarian structure, the mature follicle bearing the egg, particularly during the late and immediate preovulatory phase of the menstrual cycle (Birnholz, 1984; Oike et al., 1988; Abramovicz and Archer, 1990; Devries et al., 1990; Lyons et al., 1991; Kunz et al., 1996). This function, the peristaltic activity of the archimyometrium, is under the control of the main ovarian steroids, estradiol and progesterone (Kunz et al., 1998a) (Figure 7), and is made possible by the specific structure of this myometrial layer. The myocytes of the archimyometrium are densely packed with little interstitial tissue (Schwalm and Dubrauszky, 1966). In cinematographic MRI the peristaltic waves start near the internal os of the cervical canal and rapidly move in fundal direction (Figure 8). Probably by the activation of both, the circular and short longitudinal fibers (Werth and Grusdew, 1898) a muscular package is built up as the wave moves in fundal direction providing the pressure and power that enables this peristaltic pump in the late follicular phase to inject sperm into the beginning of the ampullary part of the tube leading to the dominant follicle (Kunz et al., 1996; Leyendecker et al., 1996) (Figure 9). Thus, most probably, the highest intrauterine pressure that is built up by the archimetral peristaltic pump occurs in the fundo-cornual part of the uterine cavity.

In the sub human primate and in the human the two Müllerian ducts fuse early during embryonic development to form the unpaired uterus. The circular fibers of the archimyometrium separate on the mid-uterine level and continue on both sides as the circular muscular fibers of the uterine cornua and of the tubes. This separation forms, in the upper part of the uterus, a fundo-cornual-raphe that constitutes the gross morphological basis of directed sperm transport (Leyendecker et al., 1998; Leyendecker, 2000) (Figure 10). The utero-ovarian counter-current system (Einer-Jensen, 1988) provides the vascular basis for the ovarian endocrine control that ensures sperm transport into the tube on the side of the dominant follicle (Kunz et al., 1998b; Wildt 1998; Zervomanolakis et al., 2009). Thus, with respect to sperm transport, the unpaired uterus is still functioning as a paired organ. It is reasonable to assume that the archimetral micro-units play a significant role in this endocrine control-system. It is likely that for the fast propagation of the waves in addition to the endocrine control a neural or excitatory one exists. The short longitudinal fibers of the archimyometrium may be involved in the rapid propagation of the peristaltic waves within this network of archimetral micro-units. Dysperistalsis in uterine adenomyosis may be caused by focal and diffuse destruction of these morphological and functional units (*vide infra*).

### Neometra

The muscular layers of the neometra, particularly the stratum vasculare, subserve the expulsion of the conceptus and in non-conception cycles, during menstruation, the discharge of the menstrual debris. During an ovulatory cycle the oxytocin receptors (OTR) accumulate in the stratum vasculare of the myometrium. Their formation is stimulated by follicular estrogen and by the subsequent increase of luteal progesterone (Maggi et al., 1992). Following the decline of progesterone in blood the OTRs are activated presumably by endometrial oxytocin (OT) (Zingg et al., 1995). A gradient of OTR concentration along the longitudinal uterine axis with highest density of the OTR in the fundal part of the uterus (Fuchs et al., 1998) ensures the orthograde discharge of menstrual debris and at the same time these contractions may occlude the intramural part of the tube, thus presumably minimizing the efflux of menstrual debris into the peritoneal cavity. The muscular mass of the neometra that is highest in the fundal region of the uterus (Wetzstein, 1965; Schwalm and Dubrauszky, 1966) and the increased concentration of OTR strongly suggests that the power of the neometral contractions during menstruation is highest in the fundal region of the uterus.

### Evidence for a uterine role in the disease process

On the uterine level significant alterations are observed in women with endometriosis that pertain to the molecular biology of the endometrium and to morphological and functional alterations and dysfunctions of the muscular layers, respectively.

In the past two decades evidence was accumulating that the eutopic endometrium of women affected with endometriosis shares cellular and biochemical alterations with endometriotic lesions that are not or at least to a lesser extent found in the eutopic endometrium of healthy women. The endometrium of women with endometriosis is to a higher extent colonized with macrophages than that of women without the disease (Takebayashi et al., 2015). Furthermore, it was realized that, equipped with the COX2-enzyme and the P450 aromatase, the endometriotic lesions as well as the eutopic endometrium of affected women is, in contrast to healthy women, capable of producing estradiol from cholesterole (Fazleabas et al., 2003; Attar et al., 2009) In fact, it was shown that in women with endometriosis the concentration of estradiol in menstrual blood is higher than in peripheral blood. This difference does not exist in healthy women (Takahashi et a., 1989). Estradiol is produced by endometriotic and adenomyotic lesions and acts locally in a paracrine fashion. Recently, it was demonstrated that the chemotactic cytokine CRCI12 is significantly up-regulated in the epithelium of endometriotic lesions as well as in the eutopic endometrium of these women (Hufnagel et al., 2015).

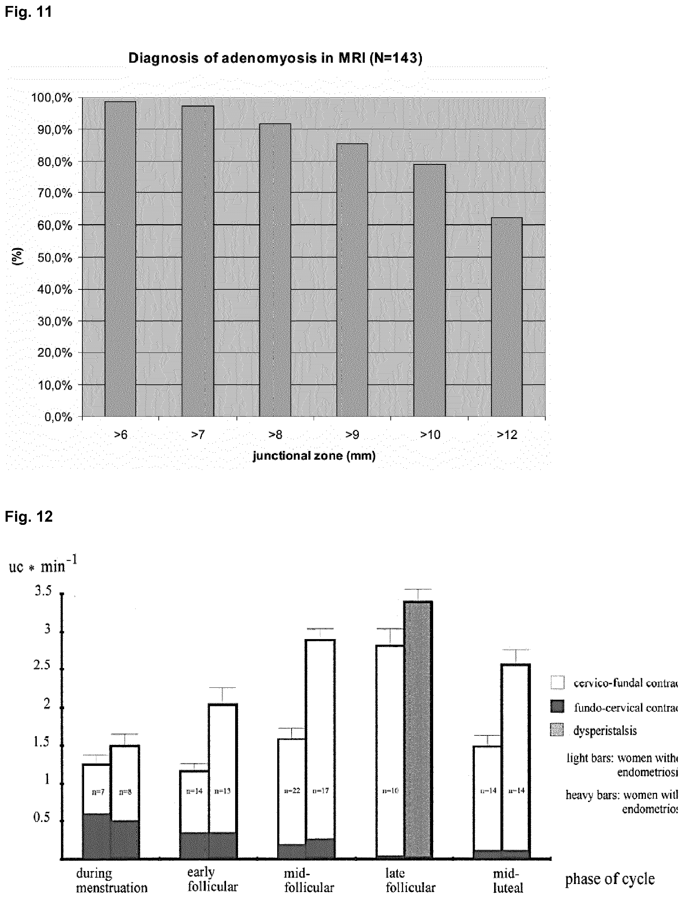

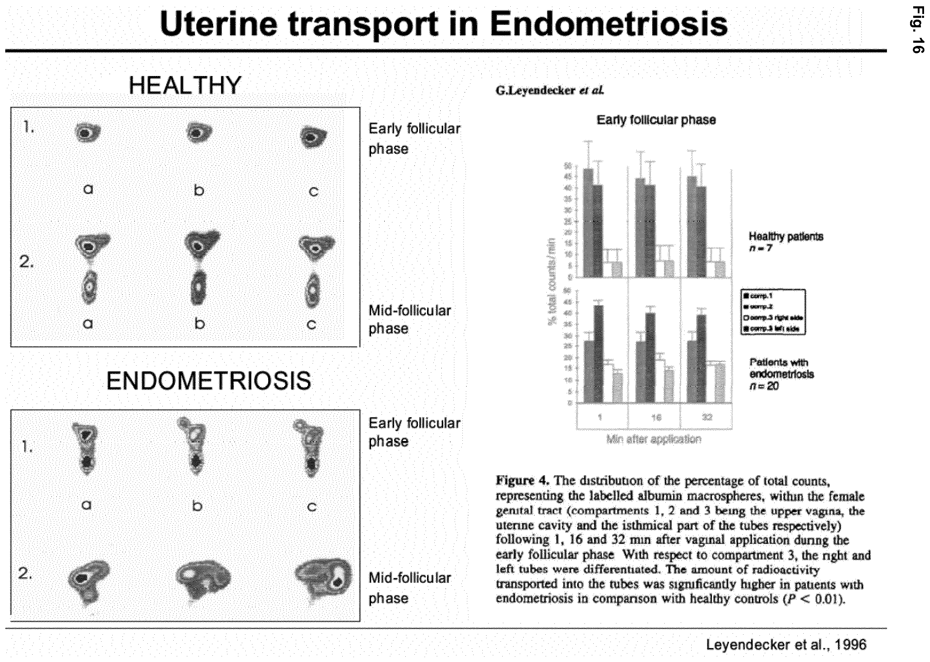

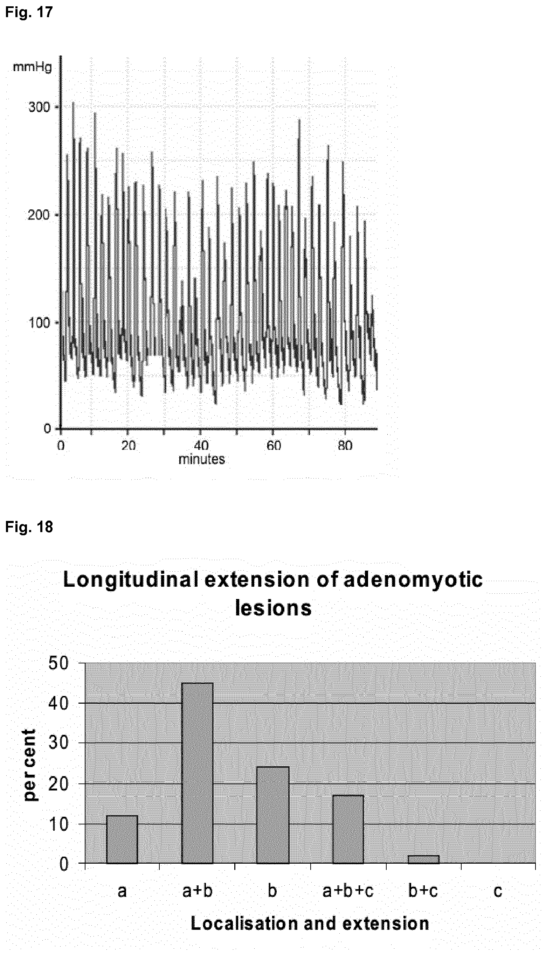

There are several lines of evidence for the notion that dysfunctions of the uterus play a crucial role in the pathophysiology of endometriosis that may be summarized as follows:
1. Fragments of basal endometrium were found in the menstrual effluent with a higher prevalence in women with endometriosis than in controls. On the basis of these and other findings it was suggested that pelvic endometriosis results from the transtubal dislocation of fragments of basal endometrium (Leyendecker et al., 2002).
2. There is a significant association of pelvic endometriosis with uterine adenomyosis in women and in the baboon with life-long infertility (Barrier et al., 2004; Kunz et al 2005; Li and Guo, 2014; Leyendecker et al., 2015). In women, the reported prevalence, however, differs according to the study population chosen and to the criteria applied to the interpretation of MRI findings (Figure 11). About 80% of the adenomyotic lesions are localized in the upper two thirds of the uterine corpus. They may extend over the whole length of the uterine corpus. They rarely present in the lower two thirds and never in the lower third alone (Figure 12) (Larsen et al., 2011; Leyendecker et al., 2015).
3. The uterine function of rapid and directed sperm transport into the 'dominant tube' is dysfunctional in women with endometriosis and is characterized by hyper- and dysperistalsis (Leyendecker et al, 1996) (Figures 12, 13, 14, 15).
4. In comparison to normal controls and in contrast to peripheral blood estradiol levels are elevated in menstrual blood of women with endometriosis and adenomyosis (Takahashi et al., 1989).
5. The expression of the P450 aromatase is increased in adenomyotic tissue and in the ectopic and eutopic endometrium of women with endometriosis (Yamamato et al., 1993; Kitiwaki et al., 1997; Review: Leyendecker and Wildt, 2011).
3. Highly estrogen-dependent genes, such as VEGF and Cyr61, are up-regulated in eutopic endometrium of women with endometriosis and also in ectopic lesions as well as in experimental endometriosis (Absenger et al., 2005; Gashaw et al., 2006).
5. The peristaltic activity of the subendometrial myometrium can be dramatically increased by elevated peripheral levels of estradiol as they are observed during controlled ovarian hyperstimulation. The intensity of uterine peristaltic activity in women with endometriosis resembles that of women during controlled ovarian hyperstimulation although the peripheral estradiol levels are within the normal range (Leyendecker et al., 1998) (Figure. 16).
6. There is an increased intra uterine pressure in these women (Mäkäräinen, 1988; Bulletti et al., 2002). Histories taken of these women reveal a high prevalence of primary dysmenorrhea (Leyendecker et al., 2015). Primary dysmenorrhea is caused by an abnormally increased strength of the neometral contractions during menstruation (Dawood, 2006) (Figure 17).

### Mechanism of disease

### Uterine auto-traumatisation

During the whole reproductive period of a woman's life, inevitably, the uterus is subjected to chronic mechanical strain due to its genuine mechanical functions, such as uterine peristalsis for directed sperm transport into the tube ipsilateral to the dominant follicle and due to rhythmic neometral contractions during menstruation for the externalization of endometrial debris. This results in chronic trauma. In autopsy, in about more than 60 % of women uterine adenomyosis can be demonstrated. Following Sampson's theory, particularly under the influence of the American Society of Reproductive Medicine (ASRM) (American Fertility Society, 1985) and the European Society of Human Reproduction and Embryology (ESHRE) (Kennedy et al., 2005), it was long maintained that premenopausal uterine adenomyosis and peritoneal endometriosis constitute two different disease entities. A careful analysis of the available literature suggests that throughout the reproductive period of life uterine adenomyosis of perimenopausal and younger women is significantly associated with pelvic endometriosis (Moen and Muus, 1991; Muus, 1991; Kunz et al., 2005; Leyendecker et al., 2015)

The mechanical strain to the uterus is considerably increased in women who acquire disease early in their reproductive life. This is indicated by the fact that the intrauterine pressure during menstruation and the peristaltic activity are significantly enhanced over controls (Mäkäräinen, 1988; Salamanca and Beltran, 1995; Bulett et al., 2002). The intrauterine pressure exerted by neometral contractions during menstruation may exceed the blood pressure of arterioles not only during the contractions themselves but also between single contractions resulting in localized ischemia within archimetral tissue in addition to mechanical injury (Figure 17) (Leyendecker et al.,2015).

The peristaltic activity is increased with the doubling of the cervico-fundal peristaltic waves per minute. This pertains particularly to the early and mid-follicular phases of the menstrual cycle (Figure 12) (Leyendecker et al., 1996)

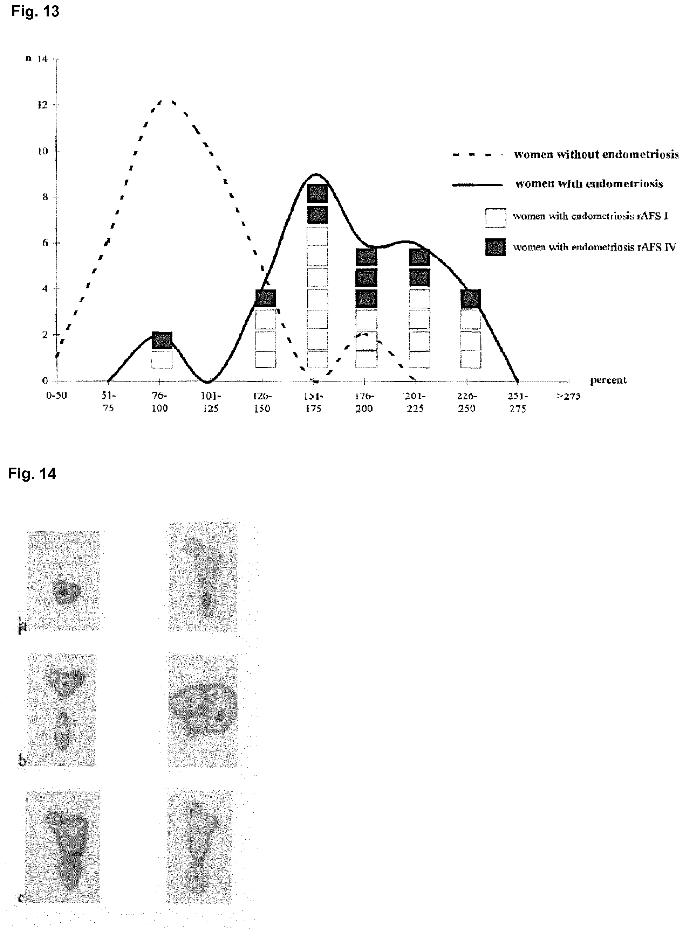

The archimyometrial peristaltic activities attain, like the neometral contractile activity, their strongest power physiologically in the more fundal part of the uterine corpus. In HSSG, it can be shown that the labeled macrospheres are injected deeply into the tubes and also into the peritoneal cavity in women affected from the disease (Figure 14).

Thus, in women with an exaggeration of both of these mechanical functions a massive mechanical strain is imposed on the more fundal part of the uterus chronically. This concurs with the finding that uterine adenomyosis primarily and predominantly develops in the more fundal part of the uterus (Figure 18) (Leyendecker et al., 2015).

The TIAR-mechanism results on the levels of the lesions in an increased tissue concentration of estradiol (Leyendecker et al., 2009). Because both mechanical functions of the uterus are controlled by ovarian estradiol, these exaggerated functions are further re-enforced by the paracrine action of the locally produced estradiol.

Because archimyometrial hyperperistalsis and neometral hypercontractility display various grades of severity it is justified to assume that, with respect to the exaggeration of the mechanical functions (and possibly also with respect to the susceptibility to mechanical strain), we are not dealing with a defined disease category completely separated from normal but rather with a 'pathophysiological continuum' ranging from mild to severe dysfunctions, such as in hypothalamic ovarian insufficiency (Leyendecker and Wildt, 1983). The development of premenopausal adenomyosis along a time axis in nearly all women supports this view. The disease development appears to be dependent on strength and chronicity of the mechanical injury. This renders the disclosure of a genetic background of adenomyosis in young women extremely difficult, in spite of clearly existing clinical and historical hints that suggest a hereditary component of endometriosis (Simpson et al., 1980; Malinak et al., 1980; Hadfield et al., 1997; Montgomery et al, 2008). Interestingly, dysmenorrhea and its severity are associated with increased contractility and over-expression of oxytocin receptors in women with symptomatic adenomyosis, which, however, could also be a sequel of local production and paracrine action of estradiol in adenomyosis (Guo et al., 2013).

### Archimyometrial hyperperistalsis versus neometral hypercontractility and compression of the archimetra as the primary causative factors

In young women affected by adenomyosis both mechanical functions of the non-pregnant uterus are intensified.

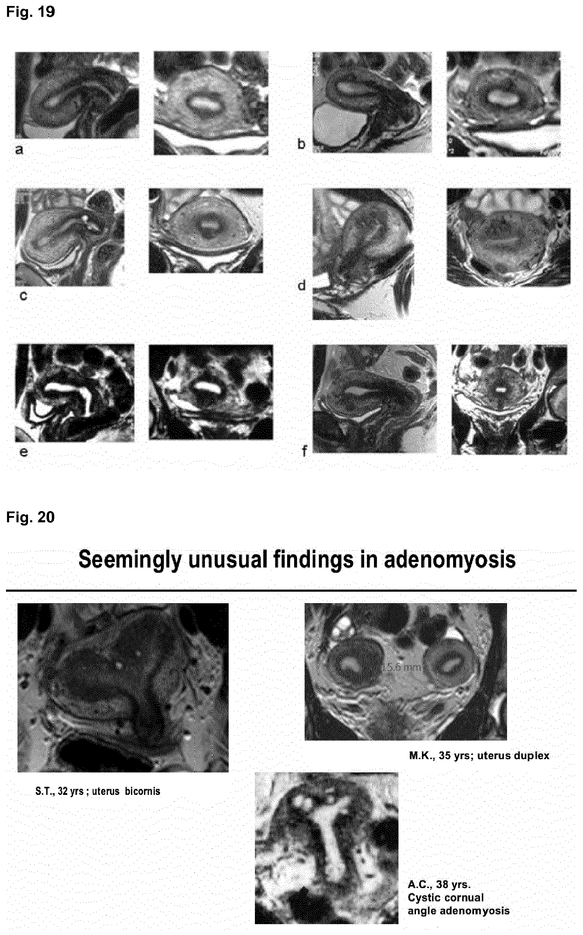

With a broadened 'junctional zone', In MRI early signs of the development of uterine adenomyosis can be identified close to the sagittal midline of the upper part of the uterus. This is the region of the 'fundo-cornual raphe, where the Müllerian ducts have fused and where, in directed sperm transport, hyperperistalsis may impose increased mechanical strain on stromal cells and myofibroblasts. This preponderance of the lesions near the uterine midline can still be observed in more advanced stages of uterine adenomyosis (Figure 19) (Leyendecker et al., 2009).

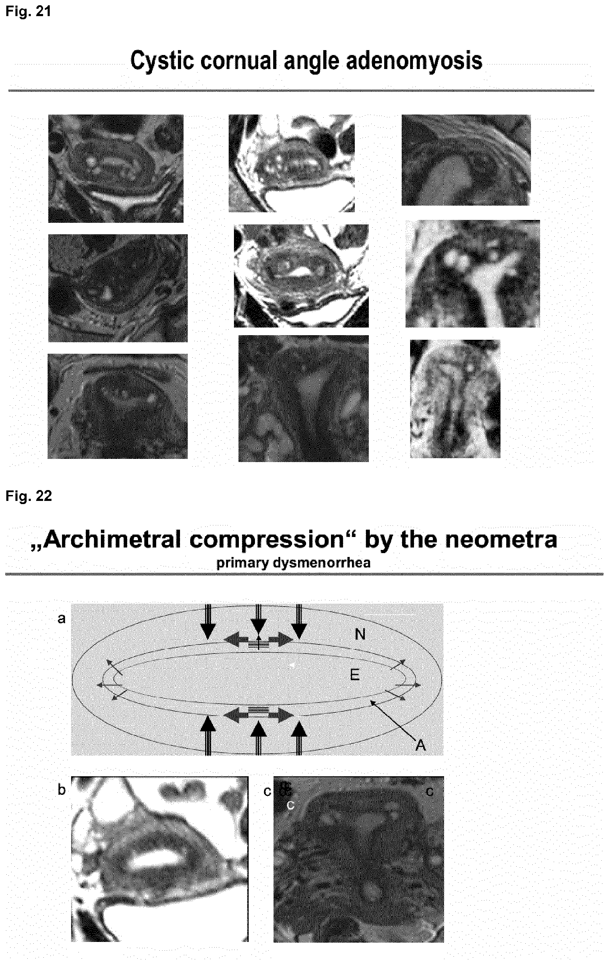

Further support for a role of hyperperistalsis in the development of adenomyosis comes from the finding of endometriosis in premenarcheal girls (Marsh and Laufer, 2005; Ebert et al., 2009; Janssen et al., 2013). With increasing ovarian function during puberty as demonstrated by rising estradiol levels in blood and the beginning growth of follicles up to the pre-ovulatory size (Peters, 1977) increased cervico-fundal peristalsis might abrade and transport cells and fragments of endometrium into the peritoneal cavity, where they might implant and cause endometriotic lesions Adenomyosis does also develop under conditions without a fundo-cornual raphe, such as congenital malformations (Hansen et al., 2006; Su et al., 2005) suggesting that an additional mechanism inducing strain on the archimetra is operative (Figure 20). Strong support for a role of neometral compression of the archimetra was derived from the finding that primary dysmenorrhea is reported in the history of the majority of young girls who develop endometriosis and adenomyosis at a young age (Chapron et al., 2011; Leyendecker et al., 2015). In extreme primary dysmenorrhea resulting in absenteeism from school and work cystic cornual angle adenomyosis may developed, which can be considered an extensive adenomyotic lesion (Leyendecker et al., 2015) (Figure 21). The mechanism proposed for the development of such lesions in extreme primary dysmenorrhea does not contradict a development of the lesions also in the uterine midline, because the strong anterior-posterior compression of the uterus in its upper part may also cause distracting the tissue and cells, such as the myofibroblasts and the stromal cells, at the sagittal midline of the endometrial-myometrial junction (Figure 22).

Thus, the available data support the view that, both, archimyometrial hyperperistalsis and neometral compression are to a variable degree involved in the process of uterine auto-traumatisation.

### The microscopical level (Microscopy)

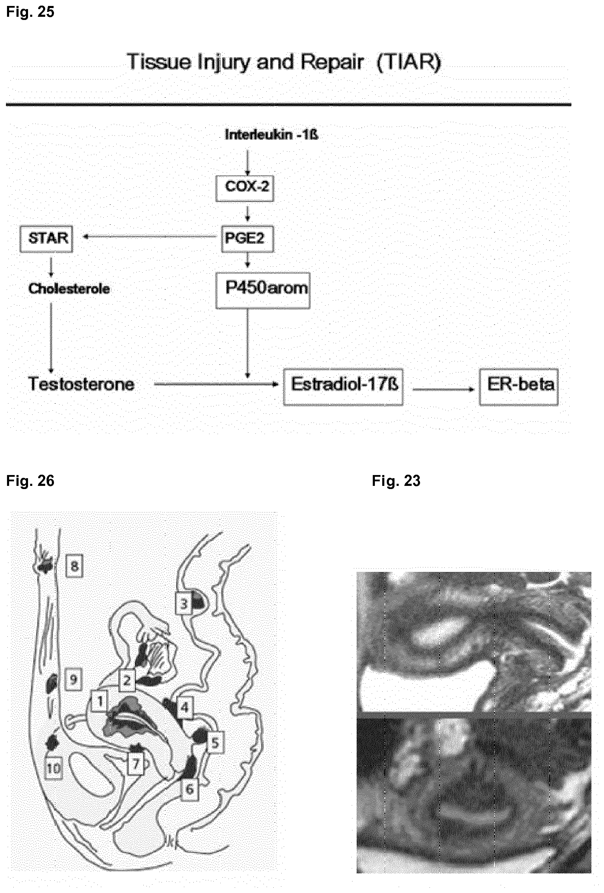

The variable interaction of both hyper-activated mechanical functions may be demonstrated by the morphology of uterine adenomyotic lesions as they are frequently described in literature. Cullen (1903) pointed out that it was often difficult to demonstrate the Müllerian origin of the lesions in that they resulted from the proliferation of endometrial glands into the depth of the myometrium. Multiple microscopic sections had to be examined with scrutiny in order to demonstrate the glandular continuity between the lesions and the epithelial surface of the endometrium (Cullen, 1903; Otto. 1957) The lesions exhibited a cauliflower-like appearance with the stalk representing the primary proliferation from the apical zone of endometrial glands into the archimyometrium and the heads representing the bulk of the adenomyotic lesion extending in various directions within the myometrial wall. Cullen refrained from providing an explanation for this type of proliferation. On the basis of the knowledge accumulated now, however, a plausible explanation of the growth pattern of such a lesion may be attempted:
The primary archimetral injury may occur focally on the stromal level of several adjacent archimetral micro-units. The area affected by the trauma may be small and can be estimated by the size of the defect of the 'junctional zone' in TVS and MRI and, as can be seen under the microscope, by the amount of proliferating glandular systems each representing an archimetral micro-unit with apical glandular growth. Because there is no vascular communication between the units, the proliferative growth of the glands that depends on the increased attraction of MSC follows the blood supply provided by the radial artery of each archimetral micro-unit. With growing into the archimyometrium with hyperperistalsis of this muscular layer further mechanical strain, although this may also constitute the primary mechanical stress, is imposed on the proliferating lesions. With entering into the stratum vasculare of the neometra, in addition to further mechanical strain resulting from the neometral contractions during menstruation, a new vascular system is tapped allowing the proliferation of the lesion in various directions (Cullen, 1908; Goodall, 1944) (Figure 23).

### Molecular Biology

### Estradiol receptor ßeta (ER-beta); chemokines and mesenchymal stem cells (MSC)

ER-ßeta, chemokines and bone marrow derived mesenchymal stem cells are the main components in the disease process. Following identification of these genes and cellular elements many scientific efforts have been undertaken, to disclose their physiological roles and those in various disease processes.

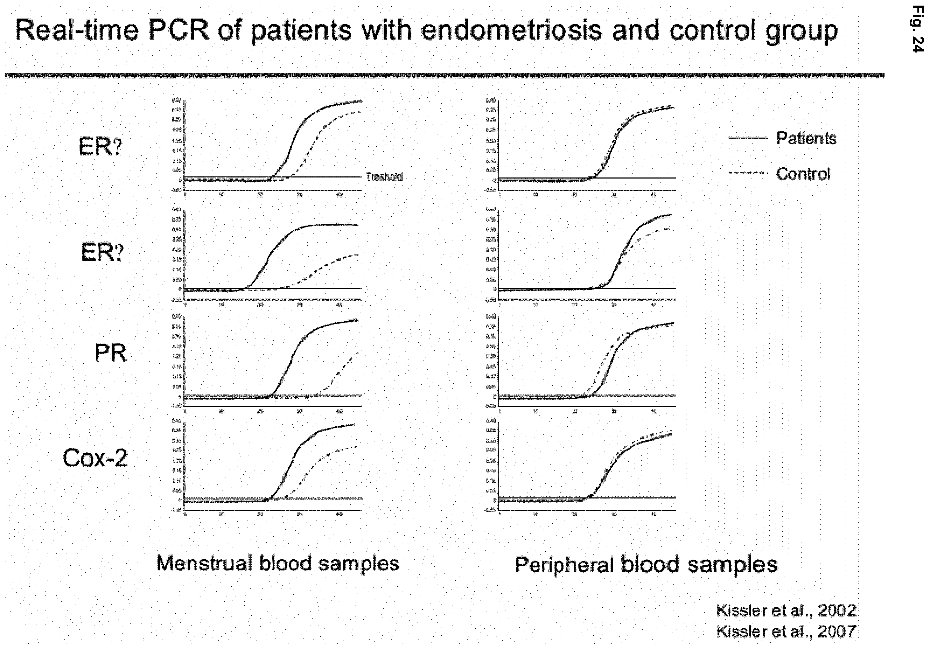

With the identification of ER-β after that of ER-alpha the dual existence of estradiol receptors, their distribution in various tissues, their physiological roles and their possible interaction are only incompletely understood (Hapangama et al., 2015). Both receptors have been shown to be expressed in many tissues of the body (Taylor and Al-Azzawi, 2000). With respect to the theme here for discussion it might be generalized that the preponderant role of ER-alpha constitutes for example in the correct functioning of the reproductive system, such as the hypothalamic-pituitary-ovarian axis and the utero-tubal complex (Franceschini et al. 2006), while ER-ßeta, concurring with the strong morphogenetic role of estradiol, is operative in embryonal morphogenesis, tissue regeneration and wound healing. In the basal endometrium, for example in patients with endometriosis and adenomyosis, both receptors are expressed as demonstrated by real-time PCR of menstrual effluent (Kissler et al., 2005, 2007; Bombail et al., 2008) (Figure 24).

Chemotactic cytokines (chemokines) are involved in cell trafficking during embryogenesis, in tissue regeneration and in wound healing (Wu et al., 2007; Stappenmbeck et al., 2009; Garbern et al., 2017). They are functionally closely related to the ER-beta. Three families of chemokines are identified with the CXC family being the preponderant one, such as CXCL12.

Bone marrow-derived MSC are a heterogeneous population of plastic adherent cells that represent only up to 0.01 % of total marrow. They are defined to following criteria: (i) plastic adherent, (ii) positive for stromal cell surface markers, while negative for hematopoietic lineage markers and HLA-DR; and (iii) able to differentiate into bone, fat, and cartilage *in vitro.* These MSC express on their surface the receptor CXCR4 (Hocking, 2015).

These three components, ER-β, CXCL12 and its receptor, CXCR4, on MSC surfaces can be termed *the basic morphogenic complex* that is acting within the archimetral micro-units and is playing a crucial role in the pathogenesis of endometriosis and adenomyosis.

### Stromal cell-derived factor 1 (CXCL12)

Stromal cell-derived factor 1 (SDF-1 or CXCL12) is a chemotactic cytokine (chemokine) that plays a predominant role in embryology, organogenesis, oncology, normal continuous tissue regeneration of virtually all tissues of the body such as the epidermis and intestinal mucosa, as well as in wound healing after injury and inflammation. It is highly expressed in tissues with a high cellular turnover, such as the intestinal mucosa and chronic skin disease such as psoriasis. CXCL12 sub-serves the attraction of bone marrow derived mesenchymal stem cells (MSC) that are equipped with the corresponding receptor CXCR4. The CXCL12/CXCR4-system regulates the need of various tissues for and the adequate supply with mesenchymal stem cells and it appears to be balanced in the healthy body with respect to normal tissue regeneration (Laird et al., 2011).

### The permissive role of estrogen in normal tissue regeneration

Estrogens play a predominant role in tissue regeneration. The expression of CXCL12 is stimulated by estrogen via the estrogen-receptor β (ER-β) (Ruiz et al., 2010; Zhou et al-. 2β15; Wang et al., 2015). In hypoestrogenic states such as the late postmenopause or following ovariectomy and in states of severe hypothalamic ovarian failure the expression of CXCL12 is reduced impairing the normal tissue regeneration that is critically dependent upon a normal incorporation of MSC into the tissue. Also the stem cells themselves appear to be of reduced quality. (Review: Gargett et al., 2016)

### The role of estradiol in tissue injury and repair (TIAR)

The local production of estrogen, both, on the level of the uterus in adenomyosis and of the ectopic peritoneal lesions is, undoubtedly, central to the understanding of the pathophysiology of the disease (Matsusaki et al., 2001). Recent studies have increasingly shown that estradiol is of utmost importance in the process of wound healing (Gilliver et al., 2007; Mowa et al., 2008). This action of estrogens appears to be mainly mediated by the estrogen receptor-beta (ER2). Animal experiments with chemotoxic and mechanic stress to astroglia (Garcia-Segura, 2008; Sierra et al., 2003; Lavaque et al., 2006) and urinary bladder tissue as well as studies with isolated connective tissue such as fibroblasts and cartilage (Yang et al., 2005; Jeffrey et al., 2007; Shioyama et al., 2008) have revealed that tissue injury and inflammation with subsequent healing is associated with a specific physiological process that involves the local production of estrogen from its precursors. Interleukin-1 induced activation of the cyclooxygenase-2 enzyme (COX-2) results in the production of prostaglandin E2 (PGE2), which in turn activates STAR (steroidogenic acute regulatory protein) and the P450 aromatase. Thus, with the increased transport of cholesterole to the inner mitochondrial membrane testosterone can be formed and aromatized into estradiol that exerts its proliferative and healing effects via the ER2. In studies with fibroblast it was surprising that the first steps of this cascade could be activated by seemingly minor biophysical strain) (Yang et al., 2005). Following termination of unphysiological strain and healing this process is down-regulated and the local production of estrogen or up-regulation of estrogen dependent genes ceases (Yang et al., 2005; Hadjiargyrou et al., 2000). This cascade can even be activated in tissue that normally does not express the P450aromatase indicating the basic physiological significance of the local production of estrogen in tissue injury and repair (TIAR) (Garcia-Segura et al., 1999). The similarity of the molecular biology of TIAR in various tissues with that described in endometriosis (Hudelist et al., 2007; Aghajanova et al., 2009; Bulun, 2009; Gurates and Bulun, 2003; Kissler et al., 2005, 2007; Attar et al., 2009) strongly suggests that this represents the common underlying mechanisms of both processes (Figure 26).).

### TIAR as an emergency system for the attraction of MSC to the site of injury

The TIAR mechanism provides 'estrogen-rich niches' at the site of tissue injury (Lander et al., 2012). In these niches MSC are accumulated to induce and accelerate the healing process. The molecular biology is similar to that of normal tissue regeneration. While the latter is dependent on the permissive action of systemic estradiol and constitutes an endocrine effect of estradiol, in the TIAR system the strong morphogenic effect of estradiol is utilized in that it is produced at the site of the lesion and acting in a paracrine way to result in an increased expression of CXCL12 that in turn attracts CXCR4 marked MSC. In the urodele it could be demonstrated that the healing of experimental heart injuries could be accelerated by local injections of estradiol into the lesion and that this effect was mediated by the increased expression of CXCL12. At the site of the lesion the MSC differentiate into the tissue specific progenitor cells. This mechanism seems also to be operative in limb regeneration of the axolotl. After amputation of a limb a blastema of stem cells is formed that covers the wound. By cell-to-cell interaction the stem cells are programmed to form the various tissues of a limb. This mechanism constitutes a re-activation of embryonic morphogenesis (Garbern et al., 2013).

In the normal endometrium CXCL12 is expressed in the glandular epithelium. Bone marrow derived stem cells marked by the CXCR4 are attracted by the chemokine and homed in the stroma of the endometrial-myometrial junction to substitute continuously for the loss of apoptotic endometrial cells. Isolated and transplanted endometrial stem cells (ESC; better: archimetral stem cells; ASC) have been shown to differentiate in all Müllerian tissue elements, such as endometrial glandular epithelium, endometrial stroma and metaplastic myometrium (Review: Gargett et al., 2016). In endometriosis, in consequence of the local production and the paracrine effect of estradiol the expression of CXCL12 is and the attraction of MSC are dramatically increased. Locally they differentiate into archimetral stem cells and form *hoxa-10 regulated archimetral micro-units,* such as "uteri en miniature" in adenomyotic (Cullen, 1903) and "mini-primordial uteri" in endometriotic lesions (Leyendecker et al., 2002).

The attraction MSC to the lesions in uterine adenomyosis does not result in healing. Because the causal trauma and, therefore, the attraction of MSC continue, the proliferative process is resulting with the adenomyotic lesion in aberrant Müllerian morphological structures of variable size, within the uterine wall.

### Peritoneal endometriosis and peripheral adenomyosis

There is ample evidence that basal endometrial fragments or only single viable cells of the basal endometrium or even only endometrial or archimetral stem cells (ESC; ASC) may be transported into the periphery of the body by the vascular system as indicated by vital Müllerian tissue in lymphnodes (Mechsner et al., 2008).

The transtubal transmission, however, certainly constitutes the preponderant route of dissemination (Sampson, 1927; Leyendecker 2000). Highly vital basal endometrial tissue elements are transported into the abdominal cavity, where they implant on peritoneal surfaces. Accounting his work Cullen (1920) describes the sites, where endometriotic/adenomyotic lesions persist. These are sites of enduring mechanical strain (Figure 26). Thus, the TIAR-system is also operative on the level of external lesions forming "micro-primordial uteri" (Figure 1) (Leyendecker et al., 2002). Lesions at peritoneal sites that are not subjected to chronic mechanical strain undergo transformation into white fibrotic scars and finally disappear, because the TIAR system is not operative and the proliferative process is not supported by paracrine estradiol

### Impairment of fertility

Already Freund (in: Recklinghausen, 1897) mentioned that sterility was one of the main symptoms of patients suffering from uterine adenomyosis. With the advent of laparoscopy it was realized that not only severe pelvic endometriosis with an overt impairment of ovarian and utero-tubal function but also minimal and mild forms of the disease with no involvement of the tubo-ovarian complex were associated with infertility. Surgical and medical eradication of these lesions did not result in a significant improvement of the conception rates in these patients (Hull et al., 1987; Adamson and Pasta, 1994; Marcoux et a., 1997).

Because no overt cause of the sterility could be identified in these patients the term "unexplained infertility" was introduced (Tummon et al., 1988; Cahil et al., 1995). At that time, the possible presence of uterine adenomyosis was not yet taken into consideration in these studies. That uterine adenomyosis could be a factor of sterility independent of peritoneal lesion was shown in couples with uterine adenomyosis constituting the sole identified factor of sterility, in MRI, the "junctional zone" was significantly broader than in sterile couples with an additional male factor of sterility (Kunz et al., 2005). It was assumed that uterine hyper- and dysperistalsis of the female with an impairment of directed sperm transport were the causative factors.

Already in small adenomyotic lesions hyper- and dysperistalsis can be observed. These lesions destroy the cervico-fundal propagation of the peristaltic waves. The correct propagation of these waves to ensure directed sperm transport into the dominant tube presumably requires the undisturbed interaction of the archimetral micro-units. Furthermore, the local production and paracrine action of estradiol in the TIAR process interferes with the endocrine regulation of directed sperm transport. As seen in real-time TVS, in women with endometriosis, the long cervico-fundal waves as seen in normal controls are replaced by a more convulsive action of the adenomyotic uterus (Kunz et al., 2000).

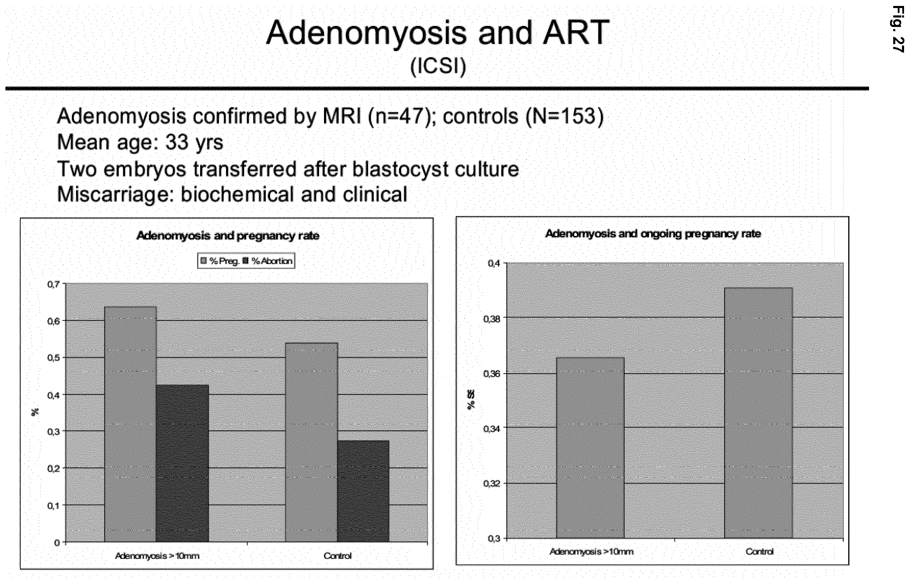

The level of HOXA 10 and CXCL12 have been described to be lowered in endometrium of women with endometriosis in comparison to controls. It was therefore suggested that the reduced implantation rates in endometriosis may be caused by the decrease of these levels (Moridi et al., 2017). This, however, does no concur with the clinical experience. In women with endometriosis and adenomyosis, following artificial reproductive technology (ART), such as IVF and ICSI, the implantation rate is at least as high as in normal women. The miscarriage rate, however, is increased (Figure 27). Primary dysmenorrhea is considered a sign of normal ovulatory function. Moreover, as judged from ART, there is no evidence that the oocytes of women with endometriosis and adenomyosis are of reduced quality.

Thus, taken together, there is strong direct and indirect evidence that the destruction of the functional morphology of the archimetra by mechanical strain constitutes a major cause of the development of infertility in young women affected by endometriosis.

### Nosological categorization of adenomyosis and endometriosis

### Injury due to mechanical strain followed by wound healing with the resumption of embryonal morphogenesis

There is ample evidence provided that the disease is caused by auto-traumatisation of the non-pregnant uterus by its genuine mechanical functions during the normal menstrual cycle and/or by iatrogenic trauma. Chronic mechanical strain is the causative factor in many diseases, such as atherosclerosis and arthrosis. With the TIAR-system resulting in the local production of estradiol and the chemo-attraction of stem cells by CXCL12 the disease utilizes the basic, evolutionarily highly conserved molecular biology of cell trafficking in embryology (Wierman et al., 2011), continuous tissue regeneration, such as that of the intestinal mucosa (Konstatzinopoulos et al., 2003; Wada-Hareike et al., 2006), chronic proliferative diseases, such as psoriasis (Zraggen et al., 2004) and wound healing following various kinds of injury (Hocking, 2015) such as inflammation i. e. of the pulpa of the teeth (Zhang et al., 2015) and other tissues as well as mechanical trauma, of virtually all organs and tissues of the body (Wu et al., 2007). Thus, the pathophysiology of adenomyosis is essential that of tissue injury and repair (TIAR) (Leyendecker et al., 2009). The proliferative process in endometriosis and adenomyosis consists in the attempt to reconstruct, following injury, archimetral micro-units in order to re-build the archimetra as a whole organ. It is reasonable to assume that there is a physiological background of this phenomenon.

With implantation, decidualization and hemochorial placentation the structure of the archimetra changes dramatically. The trophoblast invades the decidua deeply into the layer that had constituted the archimyometrium before pregnancy. One to two weeks after delivery the junctional zone myometrium (archimyometrium) can be, in MRI, identified again. The wound is quickly re-epithelialized and following termination of the lochia the archimetra presents with non proliferated endometrium as observed in hypoestrogenic states. In cases of breastfeeding this hypoestrogenic status persists for a longer period of time. In a recent publication it was estimated that endometrial stem cells would constitute about 30 % of the cellular mass of the decidua. Endometrial stem cells are programmed to form all tissue elements of the archimetra, such as endometrial epithelium, endometrial stroma and metaplastic muscular fibers. Thus, decidual ESC would reconstruct the archimetra, guided by the system of archimetral micro-units that persists during pregnancy and ensures the correct positioning of these morphological and functional elements (Guo et al., 2010; Huang et al., 2012).

In the axolotl it was shown that experimental interference with the limb reconstruction resulted in disordered growth (Zhu et al, 2012). Clinically, in not properly performed postpartum and miscarriage-curettages a similar event might happen. Such curettages are the cause of most of the cases of iatrogenic uterine adenomyosis (Kindermann, 1988).

In the post-partum period the reconstruction of the archimetra is controlled by the genetic program (Hoxa-10) that is also operative in the morphogenetic program of this organ during ontology. Chronic traumatisation and the permanent attraction of stem cells in consequence of the enduring paracrine action of locally elevated estradiol (TIAR), however, results in a proliferative process that destroys the functional morphology of the archimetra. Thus, it is the aim of the test to identify this destructive process as early as possible.

### Principle and mechanism of the test

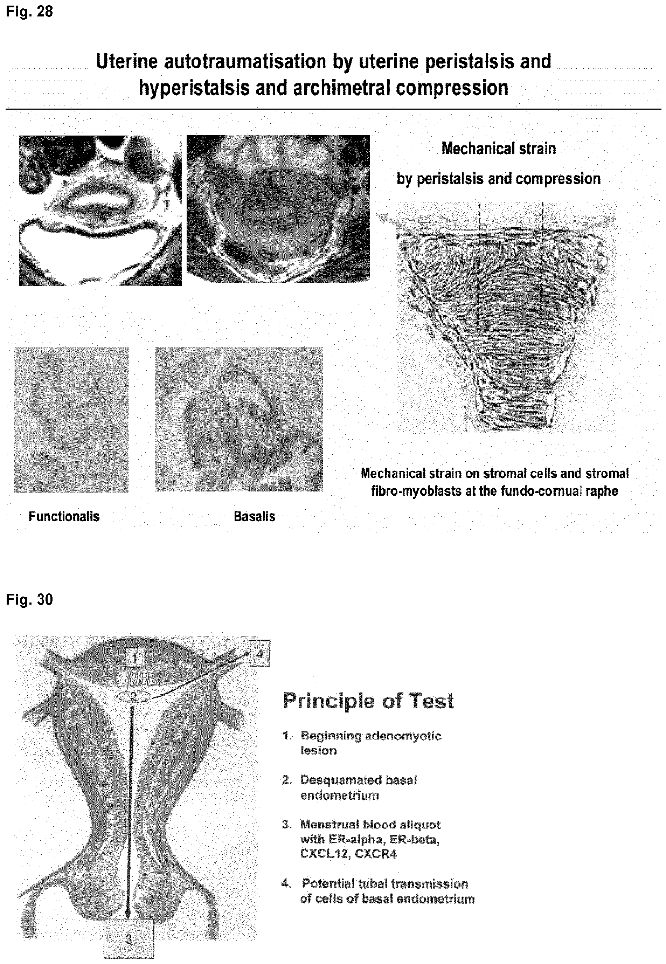

At the site of the archimetral lesion within the uterine cavity MMPs are up-regulated and the level of desquamation is horizontally moved from the spongiosa layer into the basalis layer. Thus, in contrast to healthy women, fragments of basalis are also desquamated and shed with the menstrual blood. With immunohistochemistry basal endometrial fragments can be identified in menstrual blood because the glandular epithelium of the basalis layer is stained estrogen receptor positive (Leyendecker et al., 2002). Moreover, these cells are vital in contrast to the cells of the shed functionalis layer. Some of the basal endometrial fragments may be disseminated via the tubes within the peritoneal cavity, where they have their further own fate of development (Figure 28). Some of them might implant and form peritoneal endometriosis. These lesions persist at sites of chronic mechanical stress. It has to be concurred with Sampson's clinical judgment that primary tubal dissemination of basal endometrial fragments may result in more or less scant lesions and that diffuse peritoneal endometriosis may in general result from secondary dissemination following rupture of ovarian hematoma. On the other side, the findings of biochemical alterations that correspond to parts of the TIAR cascade in endometrial biopsies of women with endometriosis clearly correlated with the grade of endometriosis (Aghajanova et al., 2009). Thus, the likelihood of transtubal dissemination increases with the extent of the archimetral lesion, but only as long the tubes are patent (Philipp and Huber, 1939).

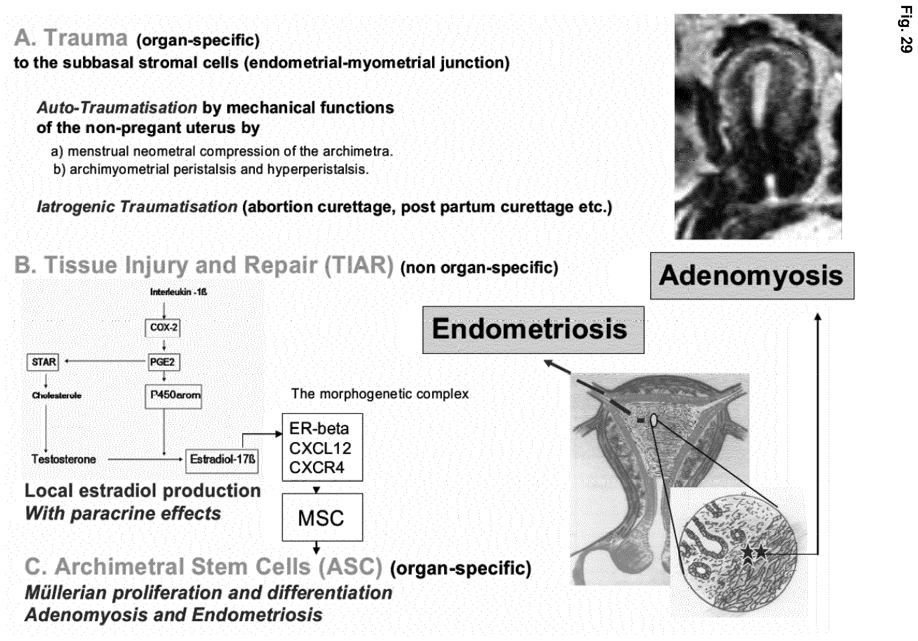

Trauma results in an attraction of macrophages to the site of the lesion. Interleukin 1β released by the macrophages initiates the TIAR process in the stromal cells of the basal endometrium and of the endometrial myometrial junction. The locally produced estradiol, by binding to the ERβ, dramatically increases the expression of CXCR12 in the basal endometrial epithelium, which in turn attracts MSC marked with CXCR4. They differentiate by cell-to-cell contact in archimetral (endometrial) stem cells that initiate the adenomyotic proliferative process (Figure 29).

The test identifies this process by the measurement of CXCL12 and/or CXCR4, preferably in an aliquot of menstrual blood. In women without the onset of the disease process this test will be negative, because no basal endometrium is desquamated during menstruation. The magnitude of the measured CXCL12 and CXCR4 levels in the menstrual blood aliquot will depend upon the extent of the archimetral lesion, the amount of basal endometrial fragments shed and collected with the test procedure. ER-alpha and ER-beta are highly expressed in the epithelium of the basal endometrium. Their parallel estimation in the menstrual blood aliquot would provide an estimation of the amount of basal endometrial fragments subjected to the test procedure (Figure 30).

The test utilizes non-organ specific processes of molecular biology. However, in that the test is performed in young women with the risk for the development of adenomyosis and the material is taken from the menstrual blood of these women high degrees of disease- and organ-specificity of the test are ensured.

### Summary

The concept of uterine auto-traumatisation of the non-pregnant uterus during the reproductive period of life (Tissue Injury and Repair, TIAR) created a completely new basis for the understanding of the pathophysiology of adenomyosis and endometriosis and provides the perspective of a new rational approach to the prevention of the disease. The new insights are based upon a new understanding of the morphology of the non-pregnant uterus, its various functions in the early process of reproduction and their endocrine and paracrine regulation. They are furthermore based on new imaging techniques, such as hysterosalpingoscintigraphy (HSS, magnetic resonance imaging (MRI) and transvaginal sonography (TVS), as well as on new data obtained in molecular biology, not only with respect to the diseases under study but also with respect to more general principles of molecular biology regarding embryology, tissue injury and organ repair. Of utmost importance was, in the understanding of the pathophysiology, to relate the findings of the individual patient to her history. While uterine peristalsis for directed sperm transport had initially been considered the principal mechanical function of the non-pregnant uterus to result in lesions at the upper midline of the archimetra (fundo-cornual raphe), new data suggest that 'neometral compression of the archimetra' for orthograde menstrual discharge should be considered as another important cause of uterine auto-traumatisation. While about 60-80% of all women develop perimenopausal adenomyosis/endometriosis, about 10-15% being affected by an early onset of the disease. Uterine auto-traumatisation pertains to both forms. Genetic variations, such as an increased local production and paracrine action of estradiol following trauma (COX-2; P450arom) and/or hyperactivity of the oxytocin (OT)/OT-receptor system controlling the mechanical functions of the non-pregnant uterus, may be responsible for the increased uterine auto-traumatisation in young women affected by the disease.

The pathophysiology is characterized by three intertwined processes:
1. The organ-specific auto-traumatisation by uterine hyperperistalsis for directed sperm transport and by the neometral compression of the archimetra during menstruation. Both contractile mechanisms of the uterus are controlled by ovarian endocrine function.
2. The non-organ specific TIAR process that results in the production of estrogen at the site of traumatisation. The attraction of mesenchymal stem cells (MSC) to the site of the lesion is utilizing the estrogen-enhanced CXCL12/CXCR4 system.
3. The differentiation of the MSC at the site of the lesion into endometrial (ESC) or archimetral stem cells (ASC) with the genetic program that results in the proliferation of Müllerian tissue being composed of all layers of the archimetra such as endometrium, sub basal stroma and metaplastic muscular tissue ("uteri en miniature", Cullen, 1903).
Iatrogenic adenomyosis develops principally in the same way.

Focal and diffuse proliferations of Müllerian tissue destroy the functional architecture of the 'junctional zone myometrium' (archimyometrium) resulting in dysperistalsis and impaired fertility. In MRI and TVS these proliferations can be visualized as permanent irregularities and expansions of the junctional zone (JZ) and "halo", respectively.

There is a high association between adenomyosis and pelvic endometriosis. Indirect evidence suggests that in women with adenomyosis fragments of basal endometrium and stroma are disseminated within the abdominal cavity where they implant on peritoneal surfaces. They persist at sites of chronic mechanical strain. Such lesions are composed of all archimetral elements ("mini primordial uteri", Leyendecker et al., 2002) and can therefore be regarded as external adenomyosis.

The test of the present invention aims at identifying these processes on the level of the uterus in an early stage of development in young women with primary dysmenorrhea that are at special risk to acquire the disease. With the test procedure, the appearances and the levels of CXCL12 and CXCR4 are determined preferably in menstrual blood. CXCL12 and CXCR4 are expressed in the endometrial epithelium and adjacent stroma of the basal endometrium, respectively. Because estradiol receptor (ER) is always expressed in the epithelium of the basal endometrium, the parallel measurement of estradiol receptor (ER) allows estimating whether or not basal endometrium is desquamated at all during menstruation. The levels of both, CXCL12 and CXCR4, preferably relative to the levels of ER, indicate initiation and extent of the attraction of MSC and thus of the proliferative process. An early diagnosis of the disease process allows taking adequate measures against a further progression of the proliferation in order to prevent the detrimental sequels of the disease such as causing infertility.

### Methods for detecting CXCL12 and/or CXCR4

The detection reagents for determining the level of CXCL12 and/or CXCR4 or fragment(s) thereof, are preferably selected from those necessary to perform the method, for example antibodies directed to CXCL12 and/or CXCR4, suitable labels, or alternative diagnostic reagents for molecular determination and preferably quantification of CXCL12 and/or CXCR4.

In one embodiment of the method described herein the level of CXCL12 and/or CXCR4 or fragment(s) thereof is determined using a method selected from the group consisting of an immunoassay (IA), nucleic acid amplication reaction, or mass spectrometry (MS). Non-limiting examples relate to a luminescence immunoassay (LIA), radioimmunoassay (RIA), chemiluminescence- and fluorescence-immunoassays, enzyme immunoassay (EIA), Enzyme-linked immunoassays (ELISA), luminescence-based bead arrays, magnetic beads based arrays, protein microarray assays, rapid test formats such as for instance immunochromatographic strip tests and automated systems/analyzers.

Antibodies against CXCL12 or CXCR4 are known to a skilled person, for example those obtained from ThermoFischer or Abcam.

The method according to the present invention can furthermore be embodied as a homogeneous immunoassay method, wherein the sandwich complexes formed by the antibody/antibodies and the marker, e.g., the CXCL12 and/or CXCR4 or a fragment thereof, which is to be detected, remains suspended in the liquid phase. In this case it is preferred, that when two antibodies are used, both antibodies are labelled with parts of a detection system, which leads to generation of a signal or triggering of a signal if both antibodies are integrated into a single sandwich.

In further embodiments of the method described herein, the method additionally comprises a molecular analysis of a sample from said patient. The sample used for the molecular analysis for detecting an infection preferably is a blood sample, more preferably menstrual blood or menstrual fluid sample.

According to some embodiments, CXCL12 and/or CXCR4 are detected by one or more methods for analysis of biomolecules selected from the group comprising nucleic acid amplification methods such as PCR, qPCR, RT-PCR, qRT-PCR or isothermal amplification, mass spectrometry, detection of enzymatic activity and immunoassay based detection methods.

Suitable primers and/or probes can be designed by a skilled person based on the nucleic acid sequences of CXCL12 and/or CXCR4, as described below. Primers may be designed to amplify nucleic acids corresponding to mRNA transcripts of CXCL12 and/or CXCR4, as is commonly employed in molecular diagnostic approaches. Further methods of molecular analysis are known to the person skilled in the art and are comprised by the method of the present invention.

The term "nucleic acid amplification" refers to any method comprising an enzymatic reaction, which allows the amplification of nucleic acids. One preferred embodiment of the invention relates to a polymerase chain reaction (PCR). Another preferred embodiment relates to real time PCR (RT-PCR) or quantitative RT-PCR (qRT-PCR), as it allows the quantification of the amplified target in real-time. The term "real-time PCR" is intended to mean any amplification technique which makes it possible to monitor the progress of an ongoing amplification reaction as it occurs (i.e. in real time). Data is therefore collected during the exponential phase of the PCR reaction, rather than at the end point as in conventional PCR. Measuring the kinetics of the reaction the early phases of PCR provides distinct advantages over traditional PCR detection. In real-time PCR, reactions are characterized by the point in time during cycling when amplification of a target is first detected rather than the amount of target accumulated after a fixed number of cycles. The higher the starting copy number of the nucleic acid target, the sooner a significant increase in fluorescence is observed. Traditional PCR methods may also be applied, and use separation methods, such as agarose gels, for detection of PCR amplification at the final phase of or end-point of the PCR reaction. For qRT-PCR no post-PCR processing of the unknown DNA sample is necessary as the quantification occurs in real-time during the reaction. Furthermore, an increase in reporter fluorescent signal is directly proportional to the number of amplicons generated.

Real-time PCR technique can be classified by the chemistry used to detect the PCR product, specific or non-specific fluorochromes. A non-specific DNA-binding dye binds to all double-stranded (ds) DNA in PCR, causing fluorescence of the dye. An increase in DNA product during PCR therefore leads to an increase in fluorescence intensity measured at each cycle.

Specific detection of PCR production can be achieved by using fluorescent reporter probes, which detect only the DNA containing the sequence complementary to the probe. Therefore, use of the reporter probe significantly increases specificity, and enables performing the technique even in the presence of other dsDNA. Using different-colored labels, fluorescent probes can be used in multiplex assays for monitoring several target sequences in the same tube. The specificity of fluorescent reporter probes also prevents interference of measurements caused by primer dimers, which are undesirable potential by-products in PCR. The method relies on a DNA-based probe with a fluorescent reporter at one end and a quencher of fluorescence at the opposite end of the probe. The close proximity of the reporter to the quencher prevents detection of its fluorescence; breakdown of the probe through hydrolysis by the 5' to 3' exonuclease activity of the polymerase used for the amplification reaction breaks the reporter-quencher proximity and thus allows unquenched emission of fluorescence, which can be detected after excitation with a laser. An increase in the product targeted by the reporter probe at each PCR cycle therefore causes a proportional increase in fluorescence due to the breakdown of the probe and release of the reporter. Due to the fact that this kind of assay is based on the 5'-exonuclease activity of the polymerase it is also called "5'-exonuclease assay".

In a preferred embodiment, the invention relates to the detection of CXCL12 and/or CXCR4 mRNA transcripts obtained from menstrual blood samples via molecular diagnostics techniques.

In particular, quantitative or semi-quantitative PCR-based methods, such as those described above, may be applied using primers that hybridize with CXCL12 and/or CXCR4-encoding nucleic acid molecules.

In one embodiment of the method described herein, the method additionally comprises comparing the determined level of CXCL12 and/or CXCR4 thereof to a reference level, threshold value and/or a population average corresponding to CXCL12 and/or CXCR4 in patients who have been diagnosed as healthy, wherein said comparing may be carried out in a computer processor using computer executable code.

The methods of the present invention may in part be computer-implemented. For example, the step of comparing the detected level of a marker, e.g. the CXCL12 and/or CXCR4, with a reference level can be performed in a computer system.

The computer-system can be directly at the point-of-care (e.g. primary care, ICU or ED) or it can be at a remote location connected via a computer network (e.g. via the internet, or specialized medical cloud-systems, optionally combinable with other IT-systems or platforms such as hospital information systems (HIS)). Typically, the computer-system will store the values (e.g. marker level or parameters such as age, blood pressure, weight, sex, etc.) on a computer-readable medium and conduct a comparison in values based-on pre-defined and/or pre-stored reference levels or reference values, leading to a score. The resulting score will be displayed and/or printed for the user (typically a health professional such as a physician). Alternatively or in addition, the associated prognosis, diagnosis, assessment, treatment guidance, patient management guidance or stratification will be displayed and/or printed for the user (typically a health professional such as a physician).

Cut-off values and other reference levels of CXCL12 and/or CXCR4 thereof in patients who have been diagnosed as being healthy may be determined by previously described methods.

As used herein, "diagnosis" in the context of the present invention relates to the recognition and (early) detection of a clinical condition of a subject. Also, the assessment of the severity of the disease may be encompassed by the term "diagnosis".

"Prognosis" relates to the prediction of an outcome or a specific risk for a subject based on a disease. This may also include an estimation of the chance of recovery or the chance of an adverse outcome for said subject.

The invention may also relate to risk assessment and/or risk stratification. In the present invention, the terms "risk assessment" and "risk stratification" relate to the grouping of subjects into different risk groups according to their further prognosis. Risk assessment also relates to stratification for applying preventive and/or therapeutic measures.

It is understood that in the context of the present invention "CXCL12 and/or CXCR4" or the like refers to determining any biological representation of CXCL12 and/or CXCR4. Included are measurement on the protein and/or nucleic acid level. Fragments of such molecules may also be employed in the analysis.

The CXCL12 gene encodes a stromal cell-derived alpha chemokine member of the intercrine family. The encoded protein functions as the ligand for the G-protein coupled receptor, chemokine (C-X-C motif) receptor 4, and plays a role in many diverse cellular functions, including embryogenesis, immune surveillance, inflammation response, tissue homeostasis, and tumor growth and metastasis. Mutations in this gene are associated with resistance to human immunodeficiency virus type 1 infections.

Multiple protein sequences have been detected for CXCL12, for example those recorded as stromal cell-derived factor 1 isoform 5 precursor [Homo sapiens], 103 aa protein, Accession: NP_001264919.1, GI: 489406390, stromal cell-derived factor 1 isoform delta precursor [Homo sapiens], 140 aa protein, Accession: NP_001171605.1, GI: 296011023, stromal cell-derived factor 1 isoform gamma precursor [Homo sapiens], 119 aa protein, Accession: NP_001029058.1, GI: 76563933, stromal cell-derived factor 1 isoform alpha precursor [Homo sapiens], 89 aa protein, Accession: NP_954637.1, GI: 40316924, stromal cell-derived factor 1 isoform beta precursor [Homo sapiens], 93 aa protein, Accession: NP_000600.1, GI: 10834988.
SEQ ID NO:1:
   Amino acid sequence of the longest identified 140 aa protein sequence of CXCL12 (NCBI; NP_001171605): 
MNAKVVVVLVLVLTALCLSDGKPVSLSYRCPCRFFESHVARANVKHLKILNTPNCALQIVARLKNNNRQVCIDPKLKWI
QEYLEKALNNLISAAPAGKRVIAGARALHPSPPRACPTARALCEIRLWPPPEWSWPSPGDV
   Additional fragments are:
      SEQ ID NO 2:
         sp|P48061|1-21 (signal peptide)
         MNAKVVVVLVLVLTALCLSDG
      SEQ ID NO 3:
         sp|P48061|22-93 (Stromal cell-derived factor 1)
         KPVSLSYRCPCRFFESHVARANVKHLKILNTPNCALQIVARLKNNNRQVCIDPKLKWIQEYLEKALNKRFKM
      SEQ ID NO 4:
         sp|P48061|24-93 (SDF-1-beta(3-72))
         VSLSYRCPCRFFESHVARANVKHLKILNTPNCALQIVARLKNNNRQVCIDPKLKWIQEYLEKALNKRFKM
      SEQ ID NO 5:
         sp|P48061|24-88 (SDF-1-alpha(3-67))
         VSLSYRCPCRFFESHVARANVKHLKILNTPNCALQIVARLKNNNRQVCIDPKLKWIQEYLEKALN

Processed forms SDF-1-beta(3-72) and SDF-1-alpha(3-67) are produced after secretion by proteolytic cleavage of isoforms Beta and Alpha, respectively. The N-terminal processing is probably achieved by DPP4. Isoform Alpha is first cleaved at the C-terminus to yield a SDF-1-alpha(1-67) intermediate before being processed at the N-terminus. The C-terminal processing of isoform Alpha is reduced by binding to heparin and, probably, cell surface proteoglycans.

The corresponding nucleic acid sequences, of the encoding CXCL12 gene and mRNA transcripts for molecular determination, may be obtained from Gene ID: 6387 (NCBI).

CXCR4 is the receptor for the C-X-C chemokine CXCL12/SDF-1 that transduces a signal by increasing intracellular calcium ion levels and enhancing MAPK1/MAPK3 activation. Acts as a receptor for extracellular ubiquitin; leading to enhanced intracellular calcium ions and reduced cellular cAMP levels. Involved in hematopoiesis and in cardiac ventricular septum formation. Also plays an essential role in vascularization of the gastrointestinal tract, probably by regulating vascular branching and/or remodeling processes in endothelial cells. Involved in cerebellar development. In the CNS, could mediate hippocampal-neuron survival.
SEQ ID NO 6: amino acid sequence of CXCR4:
   sp|P61073|1-352 
MEGISIYTSDNYTEEMGSGDYDSMKEPCFREENANFNKIFLPTIYSIIFLTGIVGNGLVILVMGYQKKLRSMTDKYRLH
LSVADLLFVITLPFWAVDAVANWYFGNFLCKAVHVIYTVNLYSSVLILAFISLDRYLAIVHATNSQRPRKLLAEKVVYV
GVWIPALLLTIPDFIFANVSEADDRYICDRFYPNDLWVVVFQFQHIMVGLILPGIVILSCYCIIISKLSHSKGHQKRKA
LKTTVILILAFFACWLPYYIGISIDSFILLEIIKQGCEFENTVHKWISITEALAFFHCCLNPILYAFLGAKFKTSAQHA
LTSVSRGSSLKILSKGKRGGHSSVSTESESSSFHSS
   Additional isoforms of this protein are also known, such as:
SEQ ID NO 7: 
MSIPLPLLQIYTSDNYTEEMGSGDYDSMKEPCFREENANFNKIFLPTIYSIIFLTGIVGNGLVILVMGYQKKLRSMTDK
YRLHLSVADLLFVITLPFWAVDAVANWYFGNFLCKAVHVIYTVNLYSSVLILAFISLDRYLAIVHATNSQRPRKLLAEK
VVYVGVWIPALLLTIPDFIFANVSEADDRYICDRFYPNDLWVVVFQFQHIMVGLILPGIVILSCYCIIISKLSHSKGHQ
KRKALKTTVILILAFFACWLPYYIGISIDSFILLEIIKQGCEFENTVHKWISITEALAFFHCCLNPILYAFLGAKFKTS
AQHALTSVSRGSSLKILSKGKRGGHSSVSTESESSSFHSS
 Multiple transcript variants encoding different isoforms of CXCL12 have been found for this gene. Reference: Gene ID: 6387, NCBI.
SEQ ID NO 8: NM_199168.3 Homo sapiens C-X-C motif chemokine ligand 12 (CXCL12), transcript variant 1, mRNA 
GCCGCACTTTCACTCTCCGTCAGCCGCATTGCCCGCTCGGCGTCCGGCCCCCGACCCGCGCTCGTCCGCCCGCCCGCCC
GCCCGCCCGCGCCATGAACGCCAAGGTCGTGGTCGTGCTGGTCCTCGTGCTGACCGCGCTCTGCCTCAGCGACGGGAAG
CCCGTCAGCCTGAGCTACAGATGCCCATGCCGATTCTTCGAAAGCCATGTTGCCAGAGCCAACGTCAAGCATCTCAAAA
TTCTCAACACTCCAAACTGTGCCCTTCAGATTGTAGCCCGGCTGAAGAACAACAACAGACAAGTGTGCATTGACCCGAA
GCTAAAGTGGATTCAGGAGTACCTGGAGAAAGCTTTAAACAAGTAAGCACAACAGCCAAAAAGGACTTTCCGCTAGACC
CACTCGAGGAAAACTAAAACCTTGTGAGAGATGAAAGGGCAAAGACGTGGGGGAGGGGGCCTTAACCATGAGGACCAGG
TGTGTGTGTGGGGTGGGCACATTGATCTGGGATCGGGCCTGAGGTTTGCCAGCATTTAGACCCTGCATTTATAGCATAC
GGTATGATATTGCAGCTTATATTCATCCATGCCCTGTACCTGTGCACGTTGGAACTTTTATTACTGGGGTTTTTCTAAG
AAAGAAATTGTATTATCAACAGCATTTTCAAGCAGTTAGTTCCTTCATGATCATCACAATCATCATCATTCTCATTCTC
ATTTTTTAAATCAACGAGTACTTCAAGATCTGAATTTGGCTTGTTTGGAGCATCTCCTCTGCTCCCCTGGGGAGTCTGG
GCACAGTCAGGTGGTGGCTTAACAGGGAGCTGGAAAAAGTGTCCTTTCTTCAGACACTGAGGCTCCCGCAGCAGCGCCC
CTCCCAAGAGGAAGGCCTCTGTGGCACTCAGATACCGACTGGGGCTGGGCGCCGCCACTGCCTTCACCTCCTCTTTCAA
CCTCAGTGATTGGCTCTGTGGGCTCCATGTAGAAGCCACTATTACTGGGACTGTGCTCAGAGACCCCTCTCCCAGCTAT
TCCTACTCTCTCCCCGACTCCGAGAGCATGCTTAATCTTGCTTCTGCTTCTCATTTCTGTAGCCTGATCAGCGCCGCAC
CAGCCGGGAAGAGGGTGATTGCTGGGGCTCGTGCCCTGCATCCCTCTCCTCCCAGGGCCTGCCCCACAGCTCGGGCCCT
CTGTGAGATCCGTCTTTGGCCTCCTCCAGAATGGAGCTGGCCCTCTCCTGGGGATGTGTAATGGTCCCCCTGCTTACCC
GCAAAAGACAAGTCTTTACAGAATCAAATGCAATTTTAAATCTGAGAGCTCGCTTTGAGTGACTGGGTTTTGTGATTGC
CTCTGAAGCCTATGTATGCCATGGAGGCACTAACAAACTCTGAGGTTTCCGAAATCAGAAGCGAAAAAATCAGTGAATA
AACCATCATCTTGCCACTACCCCCTCCTGAAGCCACAGCAGGGTTTCAGGTTCCAATCAGAACTGTTGGCAAGGTGACA
TTTCCATGCATAAATGCGATCCACAGAAGGTCCTGGTGGTATTTGTAACTTTTTGCAAGGCATTTTTTTATATATATTT
TTGTGCACATTTTTTTTTACGTTTCTTTAGAAAACAAATGTATTTCAAAATATATTTATAGTCGAACAATTCATATATT
TGAAGTGGAGCCATATGAATGTCAGTAGTTTATACTTCTCTATTATCTCAAACTACTGGCAATTTGTAAAGAAATATAT
ATGATATATAAATGTGATTGCAGCTTTTCAATGTTAGCCACAGTGTATTTTTTCACTTGTACTAAAATTGTATCAAATG
TGACATTATATGCACTAGCAATAAAATGCTAATTGTTTCATGGTATAAACGTCCTACTGTATGTGGAATTTATTTACC
TGAAATAAAATTCATTAGTTGTTAGTGATGGAGCTTAAAAAAAA
SEQ ID NO 9: NM_000609.6 Homo sapiens C-X-C motif chemokine ligand 12 (CXCL12), transcript variant 2, mRNA 
GCCGCACTTTCACTCTCCGTCAGCCGCATTGCCCGCTCGGCGTCCGGCCCCCGACCCGCGCTCGTCCGCCCGCCCGCCC
GCCCGCCCGCGCCATGAACGCCAAGGTCGTGGTCGTGCTGGTCCTCGTGCTGACCGCGCTCTGCCTCAGCGACGGGAAG
CCCGTCAGCCTGAGCTACAGATGCCCATGCCGATTCTTCGAAAGCCATGTTGCCAGAGCCAACGTCAAGCATCTCAAAA
TTCTCAACACTCCAAACTGTGCCCTTCAGATTGTAGCCCGGCTGAAGAACAACAACAGACAAGTGTGCATTGACCCGAA
GCTAAAGTGGATTCAGGAGTACCTGGAGAAAGCTTTAAACAAGAGGTTCAAGATGTGAGAGGGTCAGACGCCTGAGGAA
CCCTTACAGTAGGAGCCCAGCTCTGAAACCAGTGTTAGGGAAGGGCCTGCCACAGCCTCCCCTGCCAGGGCAGGGCCCC
AGGCATTGCCAAGGGCTTTGTTTTGCACACTTTGCCATATTTTCACCATTTGATTATGTAGCAAAATACATGACATTTA
TTTTTCATTTAGTTTGATTATTCAGTGTCACTGGCGACACGTAGCAGCTTAGACTAAGGCCATTATTGTACTTGCCTTA
TTAGAGTGTCTTTCCACGGAGCCACTCCTCTGACTCAGGGCTCCTGGGTTTTGTATTCTCTGAGCTGTGCAGGTGGGGA
GACTGGGCTGAGGGAGCCTGGCCCCATGGTCAGCCCTAGGGTGGAGAGCCACCAAGAGGGACGCCTGGGGGTGCCAGGA
CCAGTCAACCTGGGCAAAGCCTAGTGAAGGCTTCTCTCTGTGGGATGGGATGGTGGAGGGCCACATGGGAGGCTCACCC
CCTTCTCCATCCACATGGGAGCCGGGTCTGCCTCTTCTGGGAGGGCAGCAGGGCTACCCTGAGCTGAGGCAGCAGTGTG
AGGCCAGGGCAGAGTGAGACCCAGCCCTCATCCCGAGCACCTCCACATCCTCCACGTTCTGCTCATCATTCTCTGTCTC
ATCCATCATCATGTGTGTCCACGACTGTCTCCATGGCCCCGCAAAAGGACTCTCAGGACCAAAGCTTTCATGTAAACTG
TGCACCAAGCAGGAAATGAAAATGTCTTGTGTTACCTGAAAACACTGTGCACATCTGTGTCTTGTTTGGAATATTGTCC
ATTGTCCAATCCTATGTTTTTGTTCAAAGCCAGCGTCCTCCTCTGTGACCAATGTCTTGATGCATGCACTGTTCCCCCT
GTGCAGCCGCTGAGCGAGGAGATGCTCCTTGGGCCCTTTGAGTGCAGTCCTGATCAGAGCCGTGGTCCTTTGGGGTGAA
CTACCTTGGTTCCCCCACTGATCACAAAAACATGGTGGGTCCATGGGCAGAGCCCAAGGGAATTCGGTGTGCACCAGGG
 
TTGACCCCAGAGGATTGCTGCCCCATCAGTGCTCCCTCACATGTCAGTACCTTCAAACTAGGGCCAAGCCCAGCACTGC
TTGAGGAAAACAAGCATTCACAACTTGTTTTGGTTTTAAAACCCAGTCCACAAAATAACCAATCCTGGACATGAAGA
TTCTTTCCCAATTCACATCTAACCTCATCTTCTTCACCATTTGGCAATGCCATCATCTCCTGCCTTCCTCCTGGGCCCT
CTCTGCTCTGCGTGTCACCTGTGCTTCGGGCCCTTCCCACAGGACATTTCTCTAAGAGAACAATGTGCTATGTGAAGAG
TAAGTCAACCTGCCTGACATTTGGAGTGTTCCCCTTCCACTGAGGGCAGTCGATAGAGCTGTATTAAGCCACTTAAAAT
GTTCACTTTTGACAAAGGCAAGCACTTGTGGGTTTTTGTTTTGTTTTTCATTCAGTCTTACGAATACTTTTGCCCTTTG
ATTAAAGACTCCAGTTAAAAAAAATTTTAATGAAGAAAGTGGAAAACAAGGAAGTCAAAGCAAGGAAACTATGTAACAT
GTAGGAAGTAGGAAGTAAATTATAGTGATGTAATCTTGAATTGTAACTGTTCTTGAATTTAATAATCTGTAGGGTAATT
AGTAACATGTGTTAAGTATTTTCATAAGTATTTCAAATTGGAGCTTCATGGCAGAAGGCAAACCCATCAACAAAAATTG
TCCCTTAAACAAAAATTAAAATCCTCAATCCAGCTATGTTATATTGAAAAAATAGAGCCTGAGGGATCTTTACTAGTTA
TAAAGATACAGAACTCTTTCAAAACCTTTTGAAATTAACCTCTCACTATACCAGTATAATTGAGTTTTCAGTGGGGCAG
TCATTATCCAGGTAATCCAAGATATTTTAAAATCTGTCACGTAGAACTTGGATGTACCTGCCCCCAATCCATGAACCAA
GACCATTGAATTCTTGGTTGAGGAAACAAACATGACCCTAAATCTTGACTACAGTCAGGAAAGGAATCATTTCTATTTC
TCCTCCATGGGAGAAAATAGATAAGAGTAGAAACTGCAGGGAAAATTATTTGCATAACAATTCCTCTACTAACAATCAG
CTCCTTCCTGGAGACTGCCCAGCTAAAGCAATATGCATTTAAATACAGTCTTCCATTTGCAAGGGAAAAGTCTCTTGTA
ATCCGAATCTCTTTTTGCTTTCGAACTGCTAGTCAAGTGCGTCCACGAGCTGTTTACTAGGGATCCCTCATCTGTCCCT
CCGGGACCTGGTGCTGCCTCTACCTGACACTCCCTTGGGCTCCCTGTAACCTCTTCAGAGGCCCTCGCTGCCAGCTCTG
TATCAGGACCCAGAGGAAGGGGCCAGAGGCTCGTTGACTGGCTGTGTGTTGGGATTGAGTCTGTGCCACGTGTTTGTGC
TGTGGTGTGTCCCCCTCTGTCCAGGCACTGAGATACCAGCGAGGAGGCTCCAGAGGGCACTCTGCTTGTTATTAGAGAT
TACCTCCTGAGAAAAAAGGTTCCGCTTGGAGCAGAGGGGCTGAATAGCAGAAGGTTGCACCTCCCCCAACCTTAGATGT
TCTAAGTCTTTCCATTGGATCTCATTGGACCCTTCCATGGTGTGATCGTCTGACTGGTGTTATCACCGTGGGCTCCCTG
ACTGGGAGTTGATCGCCTTTCCCAGGTGCTACACCCTTTTCCAGCTGGATGAGAATTTGAGTGCTCTGATCCCTCTACA
GAGCTTCCCTGACTCATTCTGAAGGAGCCCCATTCCTGGGAAATATTCCCTAGAAACTTCCAAATCCCCTAAGCAGACC
ACTGATAAAACCATGTAGAAAATTTGTTATTTTGCAACCTCGCTGGACTCTCAGTCTCTGAGCAGTGAATGATTCAGTG
TTAAATGTGATGAATACTGTATTTTGTATTGTTTCAATTGCATCTCCCAGATAATGTGAAAATGGTCCAGGAGAAGGCC
AATTCCTATACGCAGCGTGCTTTAAAAAATAAATAAGAAACAACTCTTTGAGAAACAACAATTTCTACTTTGAAGTCAT
ACCAATGAAAAAATGTATATGCACTTATAATTTTCCTAATAAAGTTCTGTACTCAAATGTAGCCACCAACAGT
SEQ ID NO 10: NM_001033886.2 Homo sapiens C-X-C motif chemokine ligand 12 (CXCL12), transcript variant 3, mRNA 
GCCGCACTTTCACTCTCCGTCAGCCGCATTGCCCGCTCGGCGTCCGGCCCCCGACCCGCGCTCGTCCGCCCGCCCGCCC
GCCCGCCCGCGCCATGAACGCCAAGGTCGTGGTCGTGCTGGTCCTCGTGCTGACCGCGCTCTGCCTCAGCGACGGGAAG
CCCGTCAGCCTGAGCTACAGATGCCCATGCCGATTCTTCGAAAGCCATGTTGCCAGAGCCAACGTCAAGCATCTCAAAA
TTCTCAACACTCCAAACTGTGCCCTTCAGATTGTAGCCCGGCTGAAGAACAACAACAGACAAGTGTGCATTGACCCGAA
GCTAAAGTGGATTCAGGAGTACCTGGAGAAAGCTTTAAACAAGGGGCGCAGAGAAGAAAAAGTGGGAAAAAAGAAAAG
ATAGGAAAAAAGAAGCGACAGAAGAAGAGAAAGGCTGCCCAGAAAAGGAAAAACTAGTTATCTGCCACCTCGAGATGGA
CCACAGTTCACTTGCTCTCGGCGCTTTGTAAATTTGCTCGATC
SEQ ID NO 11: NM_001178134.1 Homo sapiens C-X-C motif chemokine ligand 12 (CXCL12), transcript variant 4, mRNA 
GCCGCACTTTCACTCTCCGTCAGCCGCATTGCCCGCTCGGCGTCCGGCCCCCGACCCGCGCTCGTCCGCCCGCCCGCCC
GCCCGCCCGCGCCATGAACGCCAAGGTCGTGGTCGTGCTGGTCCTCGTGCTGACCGCGCTCTGCCTCAGCGACGGGAAG
CCCGTCAGCCTGAGCTACAGATGCCCATGCCGATTCTTCGAAAGCCATGTTGCCAGAGCCAACGTCAAGCATCTCAAAA
TTCTCAACACTCCAAACTGTGCCCTTCAGATTGTAGCCCGGCTGAAGAACAACAGACAAGTGTGCATTGACCCGAA
GCTAAAGTGGATTCAGGAGTACCTGGAGAAAGCTTTAAACAACCTGATCAGCGCCGCACCAGCCGGGAAGAGGGTGATT
GCTGGGGCTCGTGCCCTGCATCCCTCTCCTCCCAGGGCCTGCCCCACAGCTCGGGCCCTCTGTGAGATCCGTCTTTGGC
CTCCTCCAGAATGGAGCTGGCCCTCTCCTGGGGATGTGTAATGGTCCCCCTGCTTACCCGCAAAAGACAAGTCTTTACA
GAATCAAATGCAATTTTAAATCTGAGAGCTCGCTTTGAGTGACTGGGTTTTGTGATTGCCTCTGAAGCCTATGTATGCC
ATGGAGGCACTAACAAACTCTGAGGTTTCCGAAATCAGAAGCGAAAAAATCAGTGAATAAACCATCATCTTGCCACTAC
CCCCTCCTGAAGCCACAGCAGGGTTTCAGGTTCCAATCAGAACTGTTGGCAAGGTGACATTTCCATGCATAAATGCGAT
CCACAGAAGGTCCTGGTGGTATTTGTAACTTTTTGCAAGGCATTTTTTTATATATATTTTTGTGCACATTTTTTTTTAC
GTTTCTTTAGAAAACAAATGTATTTCAAAATATATTTATAGTCGAACAATTCATATATTTGAAGTGGAGCCATATGAAT
GTCAGTAGTTTATACTTCTCTATTATCTCAAACTACTGGCAATTTGTAAAGAAATATATATGATATATAAATGTGATTG
CAGCTTTTCAATGTTAGCCACAGTGTATTTTTTCACTTGTACTAAAATTGTATCAAATGTGACATTATATGCACTAGCA
ATAAAATGCTAATTGTTTCATGGTATAAACGTCCTACTGTATGTGGGAATTTATTTACCTGAAATAAAATTCATTAGTT
GTTAGTGATGGAGCTTAAAAAAAACTCCTCC
SEQ ID NO 12: NM_001277990.1 Homo sapiens C-X-C motif chemokine ligand 12 (CXCL12), transcript variant 5, mRNA

GCCGCACTTTCACTCTCCGTCAGCCGCATTGCCCGCTCGGCGTCCGGCCCCCGACCCGCGCTCGTCCGCCCGCCCGCCC
GCCCGCCCGCGCCATGAACGCCAAGGTCGTGGTCGTGCTGGTCCTCGTGCTGACCGCGCTCTGCCTCAGCGACGGGAAG
CCCGTCAGCCTGAGCTACAGATGCCCATGCCGATTCTTCGAAAGCCATTATTGTACTTGCCTTATTAGAGTGTCTTTCC
ACGGAGCCACTCCTCTGACTCAGGGCTCCTGGGTTTTGTATTCTCTGAGCTGTGCAGGTGGGGAGACTGGGCTGAGGGA
GCCTGGCCCCATGGTCAGCCCTAGGGTGGAGAGCCACCAAGAGGGACGCCTGGGGGTGCCAGGACCAGTCAACCTGGGC
AAAGCCTAGTGAAGGCTTCTCTCTGTGGGATGGGATGGTGGAGGGCCACATGGGAGGCTCACCCCCTTCTCCATCCACA
TGGGAGCCGGGTCTGCCTCTTCTGGGAGGGCAGCAGGGCTACCCTGAGCTGAGGCAGCAGTGTGAGGCCAGGGCAGAGT
GAGACCCAGCCCTCATCCCGAGCACCTCCACATCCTCCACGTTCTGCTCATCATTCTCTGTCTCATCCATCATCATGTG
TGTCCACGACTGTCTCCATGGCCCCGCAAAAGGACTCTCAGGACCAAAGCTTTCATGTAAACTGTGCACCAAGCAGGAA
ATGAAAATGTCTTGTGTTACCTGAAAACACTGTGCACATCTGTGTCTTGTTTGGAATATTGTCCATTGTCCAATCCTAT
GTTTTTGTTCAAAGCCAGCGTCCTCCTCTGTGACCAATGTCTTGATGCATGCACTGTTCCCCCTGTGCAGCCGCTGAGC
GAGGAGATGCTCCTTGGGCCCTTTGAGTGCAGTCCTGATCAGAGCCGTGGTCCTTTGGGGTGAACTACCTTGGTTCCCC
CACTGATCACAAAAACATGGTGGGTCCATGGGCAGAGCCCAAGGGAATTCGGTGTGCACCAGGGTTGACCCCAGAGGAT
TGCTGCCCCATCAGTGCTCCCTCACATGTCAGTACCTTCAAACTAGGGCCAAGCCCAGCACTGCTTGAGGAAAACAAGC
ATTCACAACTTGTTTTTGGTTTTAAAACCCAGTCCACAAAATAACCAATCCTGGACATGAAGATTCTTTCCCAATTCA
CATCTAACCTCATCTTCTTCACCATTTGGCAATGCCATCATCTCCTGCCTTCCTCCTGGGCCCTCTCTGCTCTGCGTGT
CACCTGTGCTTCGGGCCCTTCCCACAGGACATTTCTCTAAGAGAACAATGTGCTATGTGAAGAGTAAGTCAACCTGCCT
GACATTTGGAGTGTTCCCCTTCCACTGAGGGCAGTCGATAGAGCTGTATTAAGCCACTTAAAATGTTCACTTTTGACAA
AGGCAAGCACTTGTGGGTTTTGTTTTGTTTTTCATTCAGTCTTACGAATACTTTTGCCCTTTGATTAAAGACTCCAGT
TAAAAAAAATTTTAATGAAGAAAGTGGAAAACAAGGAAGTCAAAGCAAGGAAACTATGTAACATGTAGGAAGTAGGAAG
TAAATTATAGTGATGTAATCTTGAATTGTAACTGTTCTTGAATTTAATAATCTGTAGGGTAATTAGTAACATGTGTTAA
GTATTTTCATAAGTATTTCAAATTGGAGCTTCATGGCAGAAGGCAAACCCATCAACAAAAATTGTCCCTTAAACAAAAA
TTAAAATCCTCAATCCAGCTATGTTATATTGAAAAAATAGAGCCTGAGGGATCTTTACTAGTTATAAAGATACAGAACT
CTTTCAAAACCTTTTGAAATTAACCTCTCACTATACCAGTATAATTGAGTTTTCAGTGGGGCAGTCATTATCCAGGTAA
TCCAAGATATTTTAAAATCTGTCACGTAGAACTTGGATGTACCTGCCCCCAATCCATGAACCAAGACCATTGAATTCTT
GGTTGAGGAAACAAACATGACCCTAAATCTTGACTACAGTCAGGAAAGGAATCATTTCTATTTCTCCTCCATGGGAGAA
AATAGATAAGAGTAGAAACTGCAGGGAAAATTATTTGCATAACAATTCCTCTACTAACAATCAGCTCCTTCCTGGAGAC
TGCCCAGCTAAAGCAATATGCATTTAAATACAGTCTTCCATTTGCAAGGGAAAAGTCTCTTGTAATCCGAATCTCTTTT
TGCTTTCGAACTGCTAGTCAAGTGCGTCCACGAGCTGTTTACTAGGGATCCCTCATCTGTCCCTCCGGGACCTGGTGCT
GCCTCTACCTGACACTCCCTTGGGCTCCCTGTAACCTCTTCAGAGGCCCTCGCTGCCAGCTCTGTATCAGGACCCAGAG
GAAGGGGCCAGAGGCTCGTTGACTGGCTGTGTGTTGGGATTGAGTCTGTGCCACGTGTTTGTGCTGTGGTGTGTCCCCC
TCTGTCCAGGCACTGAGATACCAGCGAGGAGGCTCCAGAGGGCACTCTGCTTGTTATTAGAGATTACCTCCTGAGAAAA
AAGGTTCCGCTTGGAGCAGAGGGGCTGAATAGCAGAAGGTTGCACCTCCCCCAACCTTAGATGTTCTAAGTCTTTCCAT
TGGATCTCATTGGACCCTTCCATGGTGTGATCGTCTGACTGGTGTTATCACCGTGGGCTCCCTGACTGGGAGTTGATCG
CCTTTCCCAGGTGCTACACCCTTTTCCAGCTGGATGAGAATTTGAGTGCTCTGATCCCTCTACAGAGCTTCCCTGACTC
ATTCTGAAGGAGCCCCATTCCTGGGAAATATTCCCTAGAAACTTCCAAATCCCCTAAGCAGACCACTGATAAAACCATG
TAGAAAATTTGTTATTTTGCAACCTCGCTGGACTCTCAGTCTCTGAGCAGTGAATGATTCAGTGTTAAATGTGATGAAT
ACTGTATTTTGTATTGTTTCAATTGCATCTCCCAGATAATGTGAAAATGGTCCAGGAGAAGGCCAATTCCTATACGCAG
CGTGCTTTAAAAAATAAATAAGAAACAACTCTTTGAGAAACAACAATTTCTACTTTGAAGTCATACCAATGAAAAAATG
TATATGCACTTATAATTTTCCTAATAAAGTTCTGTACTCAAATGTAGCCACCAACAGT

The corresponding nucleic acid sequences for CXCR4, of the encoding gene and mRNA transcripts for molecular determination, may be obtained from Gene ID: 7852 (NCBI).
SEQ ID NO 13: NM_001008540.2 Homo sapiens C-X-C motif chemokine receptor 4 (CXCR4), transcript variant 1, mRNA 
CTTCCCTCTAGTGGGCGGGGCAGAGGAGTTAGCCAAGATGTGACTTTGAAACCCTCAGCGTCTCAGTGCCCTTTTGTTC
TAAACAAAGAATTTTGTAATTGGTTCTACCAAAGAAGGATATAATGAAGTCACTATGGGAAAAGATGGGGAGGAGAGTT
GTAGGATTCTACATTAATTCTCTTGTGCCCTTAGCCCACTACTTCAGAATTTCCTGAAGAAAGCAAGCCTGAATTGGTT
TTTTAAATTGCTTTAAAAATTTTTTTAACTGGGTTAATGCTTGCTGAATTGGAAGTGAATGTCCATTCCTTTGCCTCT
TTTGCAGATATACACTTCAGATAACTACACCGAGGAAATGGGCTCAGGGGACTATGACTCCATGAAGGAACCCTGTTTC
CGTGAAGAAAATGCTAATTTCAATAAAATCTTCCTGCCCACCATCTACTCCATCATCTTCTTAACTGGCATTGTGGGCA
ATGGATTGGTCATCCTGGTCATGGGTTACCAGAAGAAACTGAGAAGCATGACGGACAAGTACAGGCTGCACCTGTCAGT
GGCCGACCTCCTCTTTGTCATCACGCTTCCCTTCTGGGCAGTTGATGCCGTGGCAAACTGGTACTTTGGGAACTTCCTA
TGCAAGGCAGTCCATGTCATCTACACAGTCAACCTCTACAGCAGTGTCCTCATCCTGGCCTTCATCAGTCTGGACCGCT
ACCTGGCCATCGTCCACGCCACCAACAGTCAGAGGCCAAGGAAGCTGTTGGCTGAAAAGGTGGTCTATGTTGGCGTCTG
GATCCCTGCCCTCCTGCTGACTATTCCCGACTTCATCTTTGCCAACGTCAGTGAGGCAGATGACAGATATATCTGTGAC
CGCTTCTACCCCAATGACTTGTGGGTGGTTGTGTTCCAGTTTCAGCACATCATGGTTGGCCTTATCCTGCCTGGTATTG
TCATCCTGTCCTGCTATTGCATTATCATCTCCAAGCTGTCACACTCCAAGGGCCACCAGAAGCGCAAGGCCCTCAAGAC
CACAGTCATCCTCATCCTGGCTTTCTTCGCCTGTTGGCTGCCTTACTACATTGGGATCAGCATCGACTCCTTCATCCTC
CTGGAAATCATCAAGCAAGGGTGTGAGTTTGAGAACACTGTGCACAAGTGGATTTCCATCACCGAGGCCCTAGCTTTCT
 
TCCACTGTTGTCTGAACCCCATCCTCTATGCTTTCCTTGGAGCCAAATTTAAAACCTCTGCCCAGCACGCACTCACCTC
TGTGAGCAGAGGGTCCAGCCTCAAGATCCTCTCCAAAGGAAAGCGAGGTGGACATTCATCTGTTTCCACTGAGTCTGAG
TCTTCAAGTTTTCACTCCAGCTAACACAGATGTAAAAGACTTTTTTTTATACGATAAATAACTTTTTTTTAAGTTACAC
ATTTTTCAGATATAAAAGACTGACCAATATTGTACAGTTTTATTGCTTGTTGGATTTTTGTCTTGTGTTTCTTTAGTT
TTTGTGAAGTTTAATTGACTTATTTATATAAATTTTTTTTGTTTCATATTGATGTGTGTCTAGGCAGGACCTGTGGCCA
AGTTCTTAGTTGCTGTATGTCTCGTGGTAGGACTGTAGAAAAGGGAACTGAACATTCCAGAGCGTGTAGTGAATCACGT
AAAGCTAGAAATGATCCCCAGCTGTTTATGCATAGATAATCTCTCCATTCCCGTGGAACGTTTTTCCTGTTCTTAAGAC
GTGATTTTGCTGTAGAAGATGGCACTTATAACCAAAGCCCAAAGTGGTATAGAAATGCTGGTTTTTCAGTTTTCAGGAG
TGGGTTGATTTCAGCACCTACAGTGTACAGTCTTGTATTAAGTTGTTAATAAAAGTACATGTTAAACTTAAAAAAAAAA
AAAAAAAA
SEQ ID NO 14: NM_001348056.1 Homo sapiens C-X-C motif chemokine receptor 4 (CXCR4), transcript variant 3, mRNA 
AACTTCAGTTTGTTGGCTGCGGCAGCAGGTAGCAAAGTGACGCCGAGGGCCTGAGTGCTCCAGTAGCCACCGCATCTGG
AGAACCAGCGGTTACCATGGAGGGGATCAGTGAAAATGCCCCGCTCCCTAACGTCCCAAACGCGCCAAGTGATAAACAC
GAGGATGGCAAGAGACCCACACACCGGAGGAGCGCCCGCTTGGGGGAGGAGGTGCCGTTTGTTCATTTTCTGACACTCC
CGCCCAATATACCCCAAGCACCGAAGGGCCTTCGTTTTAAGACCGCATTCTCTTTACCCACTACAAGTTGCTTGAAGCC
CAGAATGATATACACTTCAGATAACTACACCGAGGAAATGGGCTCAGGGGACTATGACTCCATGAAGGAACCCTGTTTC
CGTGAAGAAAATGCTAATTTCAATAAAATCTTCCTGCCCACCATCTACTCCATCATCTTCTTAACTGGCATTGTGGGCA
ATGGATTGGTCATCCTGGTCATGGGTTACCAGAAGAAACTGAGAAGCATGACGGACAAGTACAGGCTGCACCTGTCAGT
GGCCGACCTCCTCTTTGTCATCACGCTTCCCTTCTGGGCAGTTGATGCCGTGGCAAACTGGTACTTTGGGAACTTCCTA
TGCAAGGCAGTCCATGTCATCTACACAGTCAACCTCTACAGCAGTGTCCTCATCCTGGCCTTCATCAGTCTGGACCGCT
ACCTGGCCATCGTCCACGCCACCAACAGTCAGAGGCCAAGGAAGCTGTTGGCTGAAAAGGTGGTCTATGTTGGCGTCTG
GATCCCTGCCCTCCTGCTGACTATTCCCGACTTCATCTTTGCCAACGTCAGTGAGGCAGATGACAGATATATCTGTGAC
CGCTTCTACCCCAATGACTTGTGGGTGGTTGTGTTCCAGTTTCAGCACATCATGGTTGGCCTTATCCTGCCTGGTATTG
TCATCCTGTCCTGCTATTGCATTATCATCTCCAAGCTGTCACACTCCAAGGGCCACCAGAAGCGCAAGGCCCTCAAGAC
CACAGTCATCCTCATCCTGGCTTTCTTCGCCTGTTGGCTGCCTTACTACATTGGGATCAGCATCGACTCCTTCATCCTC
CTGGAAATCATCAAGCAAGGGTGTGAGTTTGAGAACACTGTGCACAAGTGGATTTCCATCACCGAGGCCCTAGCTTTCT
TCCACTGTTGTCTGAACCCCATCCTCTATGCTTTCCTTGGAGCCAAATTTAAAACCTCTGCCCAGCACGCACTCACCTC
TGTGAGCAGAGGGTCCAGCCTCAAGATCCTCTCCAAAGGAAAGCGAGGTGGACATTCATCTGTTTCCACTGAGTCTGAG
TCTTCAAGTTTTCACTCCAGCTAACACAGATGTAAAAGACTTTTTTTTATACGATAAATAACTTTTTTTTAAGTTACAC
ATTTTTCAGATATAAAAGACTGACCAATATTGTACAGTTTTTATTGCTTGTTGGATTTTTGTCTTGTGTTTCTTTAGTT
TTTGTGAAGTTTAATTGACTTATTTATATAAATTTTTTTTGTTTCATATTGATGTGTGTCTAGGCAGGACCTGTGGCCA
AGTTCTTAGTTGCTGTATGTCTCGTGGTAGGACTGTAGAAAAGGGAACTGAACATTCCAGAGCGTGTAGTGAATCACGT
AAAGCTAGAAATGATCCCCAGCTGTTTATGCATAGATAATCTCTCCATTCCCGTGGAACGTTTTTCCTGTTCTTAAGAC
GTGATTTTGCTGTAGAAGATGGCACTTATAACCAAAGCCCAAAGTGGTATAGAAATGCTGGTTTTTCAGTTTTCAGGAG
TGGGTTGATTTCAGCACCTACAGTGTACAGTCTTGTATTAAGTTGTTAATAAAAGTACATGTTAAACTTAAAAAAAAAA
AAAAAAAA
SEQ ID NO 15: NM_001348059.1 Homo sapiens C-X-C motif chemokine receptor 4 (CXCR4), transcript variant 4, mRNA 
AACTTCAGTTTGTTGGCTGCGGCAGCAGGTAGCAAAGTGACGCCGAGGGCCTGAGTGCTCCAGTAGCCACCGCATCTGG
AGAACCAGCGGTTACCATGGAGGGGATCAGTGAAAATGCCCCGCTCCCTAACGTCCCAAACGCGCCAAGTGATAAACAC
GAGGATGGCAAGAGACCCACACACCGGAGGAGCGCCCGCTTGGGGGAGGAGATATACACTTCAGATAACTACACCGAGG
AAATGGGCTCAGGGGACTATGACTCCATGAAGGAACCCTGTTTCCGTGAAGAAAATGCTAATTTCAATAAAATCTTCCT
GCCCACCATCTACTCCATCATCTTCTTAACTGGCATTGTGGGCAATGGATTGGTCATCCTGGTCATGGGTTACCAGAAG
AAACTGAGAAGCATGACGGACAAGTACAGGCTGCACCTGTCAGTGGCCGACCTCCTCTTTGTCATCACGCTTCCCTTCT
GGGCAGTTGATGCCGTGGCAAACTGGTACTTTGGGAACTTCCTATGCAAGGCAGTCCATGTCATCTACACAGTCAACCT
CTACAGCAGTGTCCTCATCCTGGCCTTCATCAGTCTGGACCGCTACCTGGCCATCGTCCACGCCACCAACAGTCAGAGG
CCAAGGAAGCTGTTGGCTGAAAAGGTGGTCTATGTTGGCGTCTGGATCCCTGCCCTCCTGCTGACTATTCCCGACTTCA
TCTTTGCCAACGTCAGTGAGGCAGATGACAGATATATCTGTGACCGCTTCTACCCCAATGACTTGTGGGTGGTTGTGTT
CCAGTTTCAGCACATCATGGTTGGCCTTATCCTGCCTGGTATTGTCATCCTGTCCTGCTATTGCATTATCATCTCCAAG
CTGTCACACTCCAAGGGCCACCAGAAGCGCAAGGCCCTCAAGACCACAGTCATCCTCATCCTGGCTTTCTTCGCCTGTT
GGCTGCCTTACTACATTGGGATCAGCATCGACTCCTTCATCCTCCTGGAAATCATCAAGCAAGGGTGTGAGTTTGAGAA
CACTGTGCACAAGTGGATTTCCATCACCGAGGCCCTAGCTTTCTTCCACTGTTGTCTGAACCCCATCCTCTATGCTTTC
CTTGGAGCCAAATTTAAAACCTCTGCCCAGCACGCACTCACCTCTGTGAGCAGAGGGTCCAGCCTCAAGATCCTCTCCA
AAGGAAAGCGAGGTGGACATTCATCTGTTTCCACTGAGTCTGAGTCTTCAAGTTTTCACTCCAGCTAACACAGATGTAA
AAGACTTTTTTTTATACGATAAATAACTTTTTTTTAAGTTACACATTTTTCAGATATAAAAGACTGACCAATATTGTAC
AGTTTTTATTGCTTGTTGGATTTTTGTCTTGTGTTTCTTTAGTTTTTGTGAAGTTTAATTGACTTATTTATATAAATTT
TTTTTGTTTCATATTGATGTGTGTCTAGGCAGGACCTGTGGCCAAGTTCTTAGTTGCTGTATGTCTCGTGGTAGGACTG
 
TAGAAAAGGGAACTGAACATTCCAGAGCGTGTAGTGAATCACGTAAAGCTAGAAATGATCCCCAGCTGTTTATGCATAG
ATAATCTCTCCATTCCCGTGGAACGTTTTTCCTGTTCTTAAGACGTGATTTTGCTGTAGAAGATGGCACTTATAACCAA
AGCCCAAAGTGGTATAGAAATGCTGGTTTTTCAGTTTTCAGGAGTGGGTTGATTTCAGCACCTACAGTGTACAGTCTTG
TATTAAGTTGTTAATAAAAGTACATGTTAAACTTAAAAAAAAAAAAAAAAAA
SEQ ID NO 16: NM_001348060.1 Homo sapiens C-X-C motif chemokine receptor 4 (CXCR4), transcript variant 5, mRNA 
GAGTTACATTGTCTGAATTTAGAGGCGGAGGGCGGCGTGCCTGGGCTGAGTTCCCAGGAGGAGATTGCGCCCGCTTTAA
CTTCGGGGTTAAGCGCCTGGTGACTGTTCTTGACACTGGATATACACTTCAGATAACTACACCGAGGAAATGGCTCAG
GGACTATGACTCCATGAAGGAACCCTGTTTCCGTGAAGAAAATGCTAATTTCAATAAAATCTTCCTGCCCACCATCTA
CTCCATCATCTTCTTAACTGGCATTGTGGGCAATGGATTGGTCATCCTGGTCATGGGTTACCAGAAGAAACTGAGAAGC
ATGACGGACAAGTACAGGCTGCACCTGTCAGTGGCCGACCTCCTCTTTGTCATCACGCTTCCCTTCTGGGCAGTTGATG
CCGTGGCAAACTGGTACTTTGGGAACTTCCTATGCAAGGCAGTCCATGTCATCTACACAGTCAACCTCTACAGCAGTGT
CCTCATCCTGGCCTTCATCAGTCTGGACCGCTACCTGGCCATCGTCCACGCCACCAACAGTCAGAGGCCAAGGAAGCTG
TTGGCTGAAAAGGTGGTCTATGTTGGCGTCTGGATCCCTGCCCTCCTGCTGACTATTCCCGACTTCATCTTTGCCAACG
TCAGTGAGGCAGATGACAGATATATCTGTGACCGCTTCTACCCCAATGACTTGTGGGTGGTTGTGTTCCAGTTTCAGCA
CATCATGGTTGGCCTTATCCTGCCTGGTATTGTCATCCTGTCCTGCTATTGCATTATCATCTCCAAGCTGTCACACTCC
AAGGGCCACCAGAAGCGCAAGGCCCTCAAGACCACAGTCATCCTCATCCTGGCTTTCTTCGCCTGTTGGCTGCCTTACT
ACATTGGGATCAGCATCGACTCCTTCATCCTCCTGGAAATCATCAAGCAAGGGTGTGAGTTTGAGAACACTGTGCACAA
GTGGATTTCCATCACCGAGGCCCTAGCTTTCTTCCACTGTTGTCTGAACCCCATCCTCTATGCTTTCCTTGGAGCCAAA
TTTAAAACCTCTGCCCAGCACGCACTCACCTCTGTGAGCAGAGGGTCCAGCCTCAAGATCCTCTCCAAAGGAAAGCGAG
GTGGACATTCATCTGTTTCCACTGAGTCTGAGTCTTCAAGTTTTCACTCCAGCTAACACAGATGTAAAAGACTTTTTTT
TATACGATAAATAACTTTTTTTTAAGTTACACATTTTTCAGATATAAAAGACTGACCAATATTGTACAGTTTTTATTGC
TTGTTGGATTTTTGTCTTGTGTTTCTTTAGTTTTTGTGAAGTTTAATTGACTTATTTATATAAATTTTTTTTGTTTCAT
ATTGATGTGTGTCTAGGCAGGACCTGTGGCCAAGTTCTTAGTTGCTGTATGTCTCGTGGTAGGACTGTAGAAAAGGGAA
CTGAACATTCCAGAGCGTGTAGTGAATCACGTAAAGCTAGAAATGATCCCCAGCTGTTTATGCATAGATAATCTCTCCA
TTCCCGTGGAACGTTTTTCCTGTTCTTAAGACGTGATTTTGCTGTAGAAGATGGCACTTATAACCAAAGCCCAAAGTGG
TATAGAAATGCTGGTTTTTCAGTTTTCAGGAGTGGGTTGATTTCAGCACCTACAGTGTACAGTCTTGTATTAAGTTGTT
AATAAAAGTACATGTTAAACTTAAAAAAAAAAAAAAAAAA
SEQ ID NO 17: NM_003467.2 Homo sapiens C-X-C motif chemokine receptor 4 (CXCR4), transcript variant 2, mRNA

AACTTCAGTTTGTTGGCTGCGGCAGCAGGTAGCAAAGTGACGCCGAGGGCCTGAGTGCTCCAGTAGCCACCGCATCTGG
AGAACCAGCGGTTACCATGGAGGGGATCAGTATATACACTTCAGATAACTACACCGAGGAAATGGGCTCAGGGGACTAT
GACTCCATGAAGGAACCCTGTTTCCGTGAAGAAAATGCTAATTTCAATAAAATCTTCCTGCCCACCATCTACTCCATCA
TCTTCTTAACTGGCATTGTGGGCAATGGATTGGTCATCCTGGTCATGGGTTACCAGAAGAAACTGAGAAGCATGACGGA
CAAGTACAGGCTGCACCTGTCAGTGGCCGACCTCCTCTTTGTCATCACGCTTCCCTTCTGGGCAGTTGATGCCGTGGCA
AACTGGTACTTTGGGAACTTCCTATGCAAGGCAGTCCATGTCATCTACACAGTCAACCTCTACAGCAGTGTCCTCATCC
TGGCCTTCATCAGTCTGGACCGCTACCTGGCCATCGTCCACGCCACCAACAGTCAGAGGCCAAGGAAGCTGTTGGCTGA
AAAGGTGGTCTATGTTGGCGTCTGGATCCCTGCCCTCCTGCTGACTATTCCCGACTTCATCTTTGCCAACGTCAGTGAG
GCAGATGACAGATATATCTGTGACCGCTTCTACCCCAATGACTTGTGGGTGGTTGTGTTCCAGTTTCAGCACATCATGG
TTGGCCTTATCCTGCCTGGTATTGTCATCCTGTCCTGCTATTGCATTATCATCTCCAAGCTGTCACACTCCAAGGGCCA
CCAGAAGCGCAAGGCCCTCAAGACCACAGTCATCCTCATCCTGGCTTTCTTCGCCTGTTGGCTGCCTTACTACATTGGG
ATCAGCATCGACTCCTTCATCCTCCTGGAAATCATCAAGCAAGGGTGTGAGTTTGAGAACACTGTGCACAAGTGGATTT
CCATCACCGAGGCCCTAGCTTTCTTCCACTGTTGTCTGAACCCCATCCTCTATGCTTTCCTTGGAGCCAAATTTAAAAC
CTCTGCCCAGCACGCACTCACCTCTGTGAGCAGAGGGTCCAGCCTCAAGATCCTCTCCAAAGGAAAGCGAGGTGGACAT
TCATCTGTTTCCACTGAGTCTGAGTCTTCAAGTTTTCACTCCAGCTAACACAGATGTAAAAGACTTTTTTTTATACGAT
AAATAACTTTTTTTTAAGTTACACATTTTTCAGATATAAAAGACTGACCAATATTGTACAGTTTTTATTGCTTGTTGGA
TTTTTGTCTTGTGTTTCTTTAGTTTTTGTGAAGTTTAATTGACTTATTTATATAAATTTTTTTTGTTTCATATTGATGT
GTGTCTAGGCAGGACCTGTGGCCAAGTTCTTAGTTGCTGTATGTCTCGTGGTAGGACTGTAGAAAAGGGAACTGAACAT
TCCAGAGCGTGTAGTGAATCACGTAAAGCTAGAAATGATCCCCAGCTGTTTATGCATAGATAATCTCTCCATTCCCGTG
GAACGTTTTTCCTGTTCTTAAGACGTGATTTTGCTGTAGAAGATGGCACTTATAACCAAAGCCCAAAGTGGTATAGAAA
TGCTGGTTTTTCAGTTTTCAGGAGTGGGTTGATTTCAGCACCTACAGTGTACAGTCTTGTATTAAGTTGTTAATAAAAG
TACATGTTAAACTTAAAAAAAAAAAAAAAAAA

In some embodiments, the method comprises a polymerase chain reaction (PCR) to determine a level of CXCL12 and/or CXCR4 in said sample, wherein said PCR comprises primers that hybridize with CXCL12 and/or CXCR4-encoding nucleic acid molecules according to one or more of SEQ ID NO 8-17.

In some embodiments, the method comprises an immunoassay using one or more antibodies that bind CXCL12 and/or CXCR4 according to one or more of SEQ ID NO 1-7 to determine a level of CXCL12 and/or CXCR4 in said sample.

As used herein, the term "sample" is a biological sample that is obtained or isolated from the patient or subject. "Sample" as used herein may, e.g., refer to a sample of bodily fluid or tissue obtained for the purpose of diagnosis, prognosis, or evaluation of a subject of interest, such as a patient. Preferably herein, the sample is a sample of a bodily fluid, such as blood, serum, plasma, cerebrospinal fluid, urine, saliva, sputum, pleural effusions, cells, a cellular extract, a tissue sample, a tissue biopsy, a stool sample and the like. Particularly, the sample is a sample of menstrual fluid, also known as menstrual blood.

In certain aspects of the invention, the method of determining CXCL12 and/or CXCR4 is one or more immunoassays comprising the steps of:
a) contacting the sample with
   i. a first antibody or an antigen-binding fragment or derivative thereof specific for a first epitope of said CXCL12 or CXCR4, and
   ii. a second antibody or an antigen-binding fragment or derivative thereof specific for a second epitope of said CXCL12 or CXCR4; and
b) detecting the binding of the two antibodies or antigen-binding fragments or derivates thereof to said CXCL12or CXCR4.

Preferably, one of the antibodies can be labeled and the other antibody can be bound to a solid phase or can be bound selectively to a solid phase. In a particularly preferred aspect of the assay, one of the antibodies is labeled while the other is either bound to a solid phase or can be bound selectively to a solid phase.

The level of the marker of the present invention, e.g. CXCL12 or CXCR4, can also be determined by a mass spectrometric (MS) based methods. Such a method may comprise detecting the presence, amount or concentration of one or more modified or unmodified fragment peptides of e.g. CXCL12 or CXCR4 in said biological sample or a protein digest (e.g. tryptic digest) from said sample, and optionally separating the sample with chromatographic methods, and subjecting the prepared and optionally separated sample to MS analysis. For example, selected reaction monitoring (SRM), multiple reaction monitoring (MRM) or parallel reaction monitoring (PRM) mass spectrometry may be used in the MS analysis, particularly to determine the amounts of ADM or fragments thereof. Herein, the term "mass spectrometry" or "MS" refers to an analytical technique to identify compounds by their mass. The skilled person is aware how quantify the level of a marker in the sample by mass spectrometric methods. For example, relative quantification "rSRM" or absolute quantification can be employed as described above.

The invention further relates to kits, the use of the kits and methods wherein such kits are used. The invention relates to kits for carrying out the herein above and below provided methods. The herein provided definitions, e.g. provided in relation to the methods, also apply to the kits of the invention.

In particular, the invention relates to kits for determining the presence of a tissue injury and repair (TIAR) process in the uterus of a subject as a marker for the presence of a preliminary stage or increased risk of developing a medical disorder associated with abnormal formation of endometrial tissue, wherein said kit comprises
- detection reagents for determining the level of CXCL12 and/or CXCR4 in a sample from a subject, and
- reference data, such as a reference level, corresponding to levels of CXCL12 and/or CXCR4, wherein said reference data is preferably stored on a computer readable medium and/or employed in the form of computer executable code configured for comparing the determined levels of CXCL12 and/or CXCR4, to said reference data.

The kit may additionally comprise items useful for obtaining a sample, such as a blood or menstrual fluid sample, for example the kit may comprise a container, wherein said container comprises a device for attachment of said container to a canula or syringe, is a syringe suitable for blood isolation, exhibits an internal pressure less than atmospheric pressure, such as is suitable for drawing a predetermined volume of sample into said container, and/or comprises additionally detergents, chaotropic salts, ribonuclease inhibitors, chelating agents, such as guanidinium isothiocyanate, guanidinium hydrochloride, sodium dodecylsulfate, polyoxyethylene sorbitan monolaurate, RNAse inhibitor proteins, and mixtures thereof, and/or A filter system containing nitro-cellulose, silica matrix, ferromagnetic spheres, a cup retrieve spill over, trehalose, fructose, lactose, mannose, poly-ethylen-glycol, glycerol, EDTA, TRIS, limonene, xylene, benzoyl, phenol, mineral oil, anilin, pyrol, citrate, and mixtures thereof.

The term "method" refers to manners, means, techniques and procedures for accomplishing a given task including, but not limited to, those manners, means, techniques and procedures either known to, or readily developed from known manners, means, techniques and procedures by practitioners of the chemical, biological and biophysical arts.

The present invention is further described by reference to the following non-limiting figures and examples.

### FIGURES

The invention is further described by the following figures. These are not intended to limit the scope of the invention, but represent preferred embodiments of aspects of the invention provided for greater illustration of the invention described herein.

### Detailed description of the figures:

**Figure 1****:**
   a) A schematic representation of the endometrial-subendometrial unit ("archimetra") within the human uterus based on immunocytochemical results as well as morphological and ontogenetic data. The endometrial-subendometrial unit is composed of the glandular (a1), the stromal part of the endometrium and the stratum subvasculare of the myometrium with predominantly circular muscular fibers (a2). Ontogenetically, the endometrial-subendometrial unit is derived from the paramesonephric ducts (a1) and their surrounding mesenchyme (a2). The bulk of the human myometrium does not originate from the paramesonephric ducts (a3). It consists of the stratum vasculare with a three dimensional meshwork of short muscular bundles and the stratum supravasculare with predominantly longitudinal muscular fibers. The stratum vasculare is the phylogenetically most recent acquisition and, in contrast to the endometrial-subendometrial unit, both, the stratum vasculare and supravasculare develop late during ontogeny. The stratum vasculare and supravasculare surround the uterine corpus and extend caudally only to the uterine isthmus. There is a transitory zone within the stratum vasculare adjacent to the stratum subvasculare where muscular fibers of the two layers blend (yellow margin of the stratum vasculare). The endocervical mucoasa is the most caudal structure derived from the paramesonephric ducts. The underlying circular muscular fibers, which are progressively diminishing in caudal direction, and the accompanying connective tissue blend with vaginal tissue elements (red) to form the vaginal portion of the cervix.
   b) a peritoneal endometriotic lesion (x400) as an ectopic "microarchimetra". With endometrial glands. endometrial stroma and peristromal muscular tissue the lesion is composed of all elements of the archimetra.
   c.) The primordial uterus of the 23^{rd} week of pregnancy (x50) is composed of the elements of the archimetra, such as endometrium and archimyometrium (specific actin staining) (top right). The archimetra is essentially the adult representation of the primordial uterus.
   d.) The "halo" in transvaginal sonography represents the archimyometrium as does e.) the "junctional zone" in MR imaging. Transvaginal sonography (TVS) and magnetic resonance imaging (MRI) of the uterus of a 29 years old woman unaffected with endometriosis and adenomyosis. Sagittal scans of the uterine midline are shown. The myometrial-endometrial lining is sharp and smooth; the "halo" in TVS and the "junctional zone" in MRI are unaltered; there is symmetry with respect to the anterior and posterior myometrial walls and the texture of the myometrium in TVS appears to be homogenous. Modified from Leyendecker et al. (2004, Annals of the New York Academy of Sciences 1034:338-355).
**Figure 2****:** Schematic representation of the neometra and the archimetra
**Figure 3****:** Immunohistochemistry of estrogen receptor (ER) expression during the late secretory phase (x 100) (left) and late secretory phase (x 50) (right) of the cycle. During the secretory phase of the cycle the positive ER staining is restricted to a small fringe representing basal endometrium.
**Figure 4****:** Immunoreactive scores of estradiol receptor alpha expression in the functional (blue) and basalis (red), respectively, during the menstrual cycle. Modified from Leyendecker et al. (2002, Human Reproduction 17: 2725-2736).
**Figure 5****:** Archimetral micro-unit. Left: Schematic representation of uterine arteries. After Okkels and Engle (1938). Right: Cross section of the uterine wall of a rhesus monkey on day 12 (a) and on day 17 (b) of the menstrual cycle. Bartelmez (1957)
**Figure 6****:** Section of the uterus of a woman unaffected from endometriosis/adenomyosis during menstruation. Immunohistochemistry of estrogen receptor. The level of endometrial desquamation is within the lower functionalis.
**Figure 7****:** Histogram demonstrating the frequency of the uterine peristaltic waves during menstruation, the early, mid- and late follicular and mid- and late luteal phases of the cycle, respectively., as obtained from video sonography of uterine peristalsis (VSUP) in healthy women. The relative distribution of cervico-fundal (type A) versus fundo-cervical (type B) and isthmical (type C) contractions is also shown. The graph clearly demonstrates the increase of the frequency of type A contractions with the progression of the follicular phase reaching a maximum during the late follicular phase and the decrease during the luteal phase of the cycles, respectively. With the progression of the menstrual cycle type B contraction waves almost disappear. Type C contractions prevail during the luteal phase. These contractions do not extend beyond the isthmical or lower corporal part of the uterus rendering the fundo-cornual part of the uterus a zone of relative peristaltic quiescence during the period of embryo implantation. Modified from Kunz et al. (2000, In Filicori, M. (ed) Endocrine Basis of Reproductive Function. Monduzzi Editore, Bologna, Italy).
**Figure 8****:** The course of a peristaltic wave of the archimyometrium as shown by a sequence of MRI scans obtained from cinematographic MRI scan in a healthy woman in the late follicular phase. Initially, the archimyometrium appears to be relaxed indicated by a thin JZ with a less marked hypointensity (a). The peristaltic wave starts with tension of the archimyometrium in the lower half of the uterine corpus indicated by marked hypointensity of the JZ (b). The zone of increased tension (marked hypointensity) moves in fundal direction. A muscular package is built up indicated by the rapid increase of the JZ as the wave moves in fundal direction (c-e) followed by a rapid relaxation (f).
**Figure 9****:** Representative colour prints obtained by hysterosalpingoscintigraphy in three different patients (panel 1: early follicular phase; panel 2: mid-follicular phase and panel 3: late follicular phase), respectively. In each patient, scintigrams were performed at one to two minute intervals. In this figure only the scintigrams following one minute (a), 16 minutes (b) and 32 minutes (c) after the vaginal application of the labelled macrospheres are depicted. In the patient of the mid-follicular phase, the dominant follicle was in the right ovary, while the macrospheres enter the left tube. In the patient of the late follicular phase the dominant follicle was situated in the left ovary, while the macrospheres tended to enter the right tube.
**Figure 10****:** Modified original drawing from Werth and Grusdew showing the architecture of the subendometrial myometrium (archimyometrium) in a human fetal uterus. The specific orientation of the circular fibers of the archimyometrium results from the fusion of the two paramesonephric ducts forming a fundo-cornual raphe in the midline (dashed rectangle). The peristaltic pump of the uterus, which is continuously active during the menstrual cycle, is driven by coordinated contractions of these muscular fibers. Directed sperm transport into the dominant tube is made possible by differential activation of these fibers. The region of the fundo-cornual raphe is considered the predominant site of mechanical strain. Modified from Werth and Grusdew (1898, Archiv für Gynäkologie 55: 325-409).
**Figure 11****:** Percentage of patients with the diagnosis of adenomyosis on the basis arbitrary limits of detection with respect to the thickness of the junctional zone ranging from ≥ 6 to ≥ 12 mm in the sagittal plane of the anterior or posterior wall of the uterus in 143 patients.
**Figure 12****:** A graphical demonstration of the frequency of the subendometrial uterine peristaltic waves during menstruation, the early, mid- and late follicular and mid-luteal phases of the cycle, respectively, as determined by vaginal ultrasonography (contractions/min ± SEM) in women with and without endometriosis. The graph shows also the relative distribution of fundo-cervical contractions versus cervico-fundal contractions during these different phases of the cycle. During the early and midfollicular as well as the mid-luteal phase, respectively, the peristaltic activity differs significantly between the two groups of patients (P< 0.05). During the late follicular phase the increased peristaltic activity in patients with endometriosis in comparison to the healthy controls (P=0.06) has attained the character of dysperistalsis.
**Figure 13****:** The distribution pattern of uterine peristalsis with respect to the absence (dotted line) (n=36) or presence (solid line) (n=31) of endometriosis. Data of the mid-follicular and the mid-luteal phases, respectively, of the cycle were used. The peristaltic frequency was normalised to the mean frequency in women without endometriosis as 100%. In women with endometriosis the grade according to the revised AFS classification (American Society for Reproductive Medicine) is indicated in addition.
**Figure 14****:** Representative scans obtained from hysterosalpingoscintigraphy in women without (left panel) and with endometriosis (right panel) 32 minutes following application of technetium-labelled macrospheres of sperm size in the posterior fornix of the vagina in six different women in the a) early follicular, b) mid-follicular phase and c) late follicular phases, respectively of the menstrual cycle. In normal women with normoperistalsis the particles usually remain at the site of application during the early follicular phase (left panel a). In women with endometriosis and hyperperistalsis there is in this phase already a massive transport of the particles through the uterine cavity in one of the tubes (right panel a). In the mid-follicular phase normal women show only a ascension of the particles into the uterine cavity and sometimes a trend of ascension into the tube ispsilateral to the dominant follicle (left panel b). In women with endometriosis the ascension dramatically increased and in this example the particles are transported through the tube into the peritoneal cavity. This was, however, the contralateral tube to the dominant follicle (right panel b). During the preovulatory phase of healthy women the particles are rapidly transported into the "dominant" tube (left panel c), while, due to dysperistalsis, there is a break down of directed sperm transport in women with endometriosis (right panel c). These scans show the enormous power of the uterine peristaltic pump during the early and mid-follicular phase of the cycle in women with hyperperistalsis and endometriosis. Continuous hyperperistalsis results in auto-traumatisation of the uterus. Modified from Kunz et al. (1996, Hum Reprod 11, 627-632) and from Leyendecker et al. (1996, Human Reproducion 11, 1542-1551).

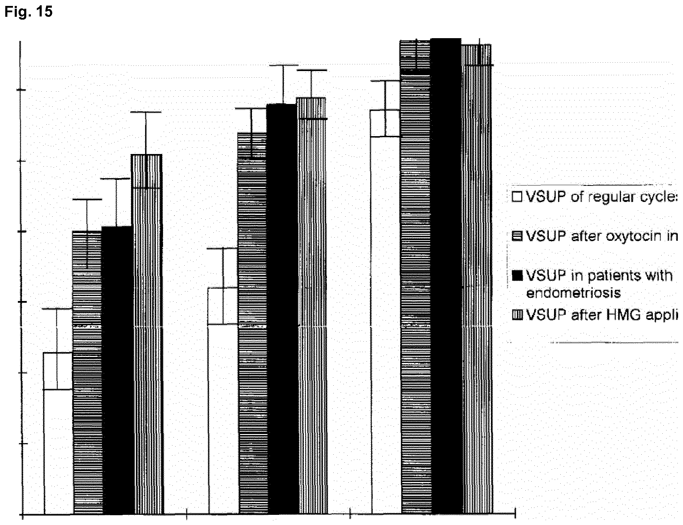

**Figure 15****:** Frequency of uterine peristaltic contractions during the follicular phase of the menstrual cycle in healthy women, in those with endometriosis, in those treated with HMG, resulting in unphysiologically high levels of oestradiol in serum and normal women treated with an iv bolus of oxytocin. The data show that high oestradiol levels and bolus injections of oxytocin, respectively, simulate the significantly increased uterine peristalsis in patients with endometriosis in comparison to healthy women. Values are mean ± SEM. Vaginal sonography of uterine peristalsis (VSUP). From Leyendecker et al.(1998 Human Reproduction Update 4: 752-762) with permission.
**Figure 16****:** Sperm transport in women with and without endomteriosis as shown by hysterosalpingoscintigraphy. Left: Representative scintigrams of the early and mid-follicular phases: Right: Histograms of the respective data obtained in the early follicular phase in these women. It is demonstrated that, in women with endometriosis, there is a significantly increased cervico-fundal transport activity in women with endometriosis.
**Figure 17****:** Recording of intrauterine pressure in an adolescent girl with extreme primary dysmenorrhea on the second day of the cycle (Courtesy L. Wildt and B. Böttcher)
**Figure 18****:** The longitudinal extension of adenomyotic lesions in the upper third (a), middle third (b) and lower third (c) of the uterine corpus . Adenomyotic lesions were localized predominantly in the upper two thirds of the uterine corpus and extended also over the whole length of the uterine corpus (a+b+c). They did rarely present in the lower two thirds (b+c) and never in the lower third (c).
**Figure 19****:** Showing examples of uterine adenomyosis in six patients as presented by magnetic resonance imaging (MRI). Representative sagittal and coronary scans are shown. In the infertile, non-parous women (a-e) (30 - 32 years of age) pelvic endometriosis of grade I-IV was demonstrated by laparoscopy. In the parous woman (f) (40 years of age) no laparoscopy was performed. In all scans preponderance of the adenomyotic lesions (expanded junctional zone) in the midline close to the fundo-cornual raphe of the archimyometrium can be demonstrated. In the first three scans (a-c) the diagnosis of adenomyosis would not meet the established radiologic criteria for MRI. In a scientific context, however, the irregularities of the junctional zone are characteristic of beginning adenomyosis. From Leyendecker et al. (2009 Archive of Gynecology and Obstetrics 280: 529-538) with permission.
**Figure 20****:** MRI findings of adenomyosis in three representative patients without a relation of the lesions with the fundo-cornual raphe. Top: Uterus bicornis with extensive adenomyosis in both cornua; adenomyosis in the right uterus in a patient with uterus duplex. Bottom: Cystic cornual angle adenomyosis.
**Figure 21****:** Nine examples of cystic cornual angle adenomyosis. These women suffered from extreme primary dysmenorrhea
**Figure 22****:** Schematic representation of uterine auto-traumatisation by the mechanism of 'archimetral compression due neometral contraction' at the onset of menstruation. N = neometra; E = endometrium; A = archimyometrium (a). Due to the high intrauterine pressure in consequence of the contraction of the neometra the archimyometrium ruptures in the cornual angles and fragments of basal endometrium are dislocated into myometrial wall, where they develop into endometriotic cysts (a and c). At the same time basal stromal cell at the fundo-cornual raphe are chronically overstretched resulting in the initiation of the TIAR mechanism and the development of an adenomyotic lesion.
**Figure 23****:** Adenomyosis in a 32 years old woman without dysmenorrhea. In the sagittal scan the hypointense area could be considered to result from a episodic neometral contraction (top). The coronal scan performed after a lapse of time reveals adenomyotic lesions with signs of sprouting of the lesion in various directions (bottom).
**Figure 24****:** Real-time PCR, comparison between women with endometriosis and controls. Patients with endometriosis are demonstrated with full line, whereas controls are demonstrated with dotted line. The figure demonstrates one representative sample out of 20 for women with endometriosis and one representative control sample out of 22.
**Figure 25****:** The basic aspects of the molecular biology of the physiological mechanism of 'tissue injury and repair' (TIAR) as demonstrates in mesenchymal tissue such as astrocytes, tendons and cartilage (Leyendecker et al., 2009)
**Figure 26****:** This is a schematic demonstration of the sites of the development of persisting and deeply infiltrating adenomyosis.
   (1) Uterine adenomyosis; (2) obvarian endometriosis; (3) intestinal endopmetriosis; (4) intestinal-uterine adhesion due to an endometriotic lesion; (5) lesion in sacro-uterine ligament; (6) retrocervical-vaginal endometriosis and in the cul de sac; (7) lesion atserosa of the urinary bladder and the anterior wall of the uterus; (8) umbilical endometriosis; (9) endometriosis in the abdominal wall; (10) inguinal endometriosis. These sites are characterized by chronic mechanical strain. Modified from Cullen 1920
**Figure 27****:** Implantation, miscarriage (left histogram) and ongoing pregnancies rates (right histogram), respectively, in patients with adenomyosis and in controls following ICSI and transfer of two embryos, respectively. Fertility Center Darmstadt. (Presented on the 2nd SEUD Congress, Barcelona, May 2016)
**Figure 28****:** Morphological and functional aspects of the mechanism of disease. Uterine auto-traumatisation is caused by hyperperistalsis and increased neometral compression of the archimetra.
Adenomyotic lesions develop near the upper uterine midline (MRI). Fragments of basal endometrium are detached and potentialy transmitted into the peritoneal cavity. Immunhistochemistry of estrodiol-receptor. While the fragment of the basdal endometrial layer is vital that of the functionalis is non-vital.
**Figure 29****:** Trauma and Müllerian tissue proliferation are organ-specific (A and C). With TIAR and the morphogenetic complex' (B) two non-organ specific systems are utilized for repair. The chronic proliferative process results in a complex morphological and functional destruction of the archimetra (MRI top right) resulting in dysperistalsis and infertility.
**Figure 30****:** The principle of the test. The specificity of the test with respect to the early diagnosis of endometriosis is attained by examining an aliquot of menstrual blood.

### EXAMPLES

The invention is further described by the following examples. These are not intended to limit the scope of the invention, but represent preferred embodiments of aspects of the invention provided for greater illustration of the invention described herein.

In order to illustrate a practical embodiment of the invention, the following is carried out:
A sandwich ELISA assay is carried out using a menstrual blood samples obtained from female subjects of ages 12 to 20.
   (1) A standard plastic ELISA plate is coated with a capture antibody directed against CXCL12; the surface is prepared with a known quantity of capture antibody. Any nonspecific binding sites on the surface are blocked. The plate is washed.
   (2) The menstrual blood sample is added, and any CXCL12 present in the sample binds to the capture antibody immobilized on the solid surface; The plate is washed to remove unbound components.
   (3) A secondary detecting antibody is added, which also binds to CXCL12, and which is labelled with a horseradish peroxidase enzyme; This enzyme-linked secondary antibody is applied as a detection antibody and binds to the already immobilized CXCL12 from the sample. The plate is washed to remove the unbound antibody-enzyme conjugates.
   (4) A substrate is added, and is converted by the enzyme of the secondary antibody to a detectable form. Essentially, a chemical is added to be converted by the enzyme into a color or fluorescent or electrochemical signal. The absorbance or fluorescence or electrochemical signal of the plate wells is measured in a standardized plate reader to determine the presence and quantity of CXCL12.

The above analysis is conducted using samples from multiple subjects who are diagnosed as healthy subjects after internal physical examination, or show symptoms of dysmenorrhea but prior to pathological indications of endometriosis. All subjects are monitored for further disease symptoms and are eventually categorized into patient groups with or without abnormal formation of endometrial tissue. Comparisons between the CXCL12 levels determined in the two subject groups indicate an elevated level of CXCL12 in subjects who went on to develop a medical condition of abnormal formation of endometrial tissue. Similar analysis on the basis of CXCR4 reveals a similar correlation.

### References

Abramovicz J.S., Archer D.F. (1990) Uterine endometrial peristalsis - a transvaginal ultrasound study. Fertil Steril 54:451-454
Absenger Y, Hess-Stumpp H, Kreft B, Kratzschmar J, Haendler B, Schutze N, Regidor PA, Winterhager E (2004) Cyr61, a deregulated gene in endometriosis. Mol Hum Reprod. 10, 399-407
Adamson GD and Pasta DJ (1994) Surgical treatment of endometriosis-associated infertility: analysis compared with survival analysis. Am J Obstet Gynecol 171, 1488-1505
Aghajanova L, Hamilton A, Kwintkiewicz J, Vo KC, Giudice LC. (2009) Steroidogenic enzyme and key decidualization marker dysregulation in endometrial stromal cells from women with versus without endometriosis. Biol Reprod Jan;80 (1):105-14. Epub 2008 Sep 24.
Alabiso et al, Endometriosis Treatment Italian Club. (2015) Adenomyosis: What the Patient Needs. J Minim Invasive Gynecol. 2016 May-Jun;23 (4):476-88. doi: 10.1016/j.jmig..12.017. Epub 2016 Jan 6 American Fertility Society (1985) Revised American Fertility Society classification of endometriosis. Fertil Steril 43,351-352
Anaf V1, Simon P, EI Nakadi I, Fayt I, Simonart T, Buxant F, Noel JC .(2002) Hyperalgesia, nerve infiltration and nerve growth factor expression in deep adenomyotic nodules, peritoneal and ovarian endometriosis. Hum Reprod 17(7):1895-900.
Attar E, Tokunaga H, Imir G, Yilmaz MB, Redwine D, Putman M, Gurates B, Attar R, Yaegashi N, Hales DB, Bulun SE. (2009) Prostaglandin E2 via steroidogenic factor-1 coordinately regulates transcription of steroidogenic genes necessary for estrogen synthesis in endometriosis. J Clin Endocrinol Metab 94: 623-631
Barcena de Arellano ML, Gericke J, Reichelt U, Ebert AD, Chiantera V, Schneider A, Mechsner S (2011) Immunohistochemical characterization of endometriosis- associated smooth muscle cells in human peritoneal endometriotic lesions. Hum Reprod 26:2721-2730
Barrier BF1, Malinowski MJ, Dick EJ Jr, Hubbard GB, Bates GW. (2004) Adenomyosis in the baboon is associated with primary infertility. Fertil Steril. Oct; 82 Suppl 3: 1091-1094
Bartelmez GW (1957) The form and the functions of the uterine blood vessels in the rhesus monkey. Carnegie Contrib Embryol 36: 153-182
Bartosik, D, Viscarello RR, Damjanov I (1984) Endometriosis as an autoimmune disease. Fertil Steril 41/2:21S
Batt RE (2011) A history of endometriosis, Springer New York
   24. Benagiano G, Brosens I. (2006) History of adenomyosis. Best Pract Res Clin Obstet Gynaecol. 20:449-463
Birnholz J. (1984) Ultrasonic visualization of endometrial movements. Fertil Steril 41:157-158
Blumenkrantz, M.J., Gallagher, N., Bashore, R.A., Tenckhoh, H. (1981) Retrograde menstruation in women undergoing chronic peritoneal dialysis. Obstet. Gynecol. 57: 667-672
Bombail V1, MacPherson S, Critchley HO, Saunders PT. (2008) Estrogen receptor related beta is expressed in human endometrium throughout the normal menstrual cycle. Hum Reprod. Dec; 23 (12):2782-90. Doi: 10.1093/humrep/den298. Epub 2008 Sep 4
Boudot A1, Kerdivel G, Habauzit D, Eeckhoute J, Le Dily F, Flouriot G, Samson M, Pakdel F. (2011) Differential estrogen-regulation of CXCL12 chemokine receptors, CXCR4 and CXCR7, contributes to the growth effect of estrogens in breast cancer cells. PLoS One.; 6 (6):e20898. doi: 10.1371/journal.pone.0020898. Epub 2011 Jun 10.
Bouzaffour M, Dufoursq P, Lecaudey V, Haas P, Vriz, S (2009) Fgf and Sdf-1 pathways interact during zebrafish fin regeneration Plos One 4: e5824
Brenner RM Slayden OD (1994) Cyclic changes in the primate oviduct and endometrium. In: E. Knobil, J.D. Neill (eds) The Physiology of Reproduction. Raven Press New York. P. 541-549
Brosens IA, Brosens JJ. (2000) Redefining endometriosis: is deep endometriosis a progressive disease? Hum Reprod. Jan;15(1):1-3. Review.
Bulletti C, De Ziegler D, Polli V, Del Ferro E, Palini S and Flamigni C. (2002) Characteristics of uterine contractility during menses in women with mild to moderate endometriosis. Fertil Steril 77: 156-1161.
Bulun SE. Endometriosis. (2009) N Engl J Med. Jan 15; 360 (3):268-79.
Burnett MA1, Antao V, Black A, Feldman K, Grenville A, Lea R, Lefebvre G, Pinsonneault O, Robert M. (2005) Prevalence of primary dysmenorrhea in Canada. J Obstet Gynaecol Can. Aug;27 (8):765-70
Cahil D. J.,Wardle P.G., Maile, L.A., Harlow, C.R., Hull, M. G. R. (1995) Pituitary ovarian dysfunction as a cause for endometriosis-associated and unexplained infertility. Hum Reprod 10: 3142-3146
Ceradini DJ1, Kulkarni AR, Callaghan MJ, Tepper OM, Bastidas N, Kleinman ME, Capla JM, Galiano RD, Levine JP, Gurtner GC. (2004) Progenitor cell trafficking is regulated by hypoxic gradients through HIF-1 induction of SDF-1. Nat Med. Aug; 10 (8):858-64. Epub 2004 Jul 4
Chapron C, Souza C, Borghese B, Lafay-Pillet MC, Santulli P, Bijaoui G, Goffinet F, de Ziegler, D (2011) Oral contraceptives and endometriosis: the past use of oral contraceptives for treating severe primary dysmenorrhea is associated with endometriosis, especially deep infiltrating endometriosis. Hum Reprod 26:2028-2035
Counseller VS (1938) Endometriosis. A clinical and surgical review. Am J Obstet Gynecol 36:877-886 Cullen TS (1896) Adenomyoma uteri diffusum benignum. Johns Hopkins Hosp. Rep 6:133-157
Cullen TS (1903) Adeno-Myome des Uterus. (Festschrift Johannes Orth) Verlag von August Hirschwald, Berlin
Cullen TS (1908) Adenomyoma of the uterus. W.B. Saunders Company. Philadelphia and London
Cullen TS (1920) The distribution of adenomyoma containing uterine mucosa. Arch Surgery 1:215-283
Dawood MY (2006) Primary dysmenorrhea. Advances in pathogenesis and management. Obstet. Gynecol 108: 428-440,
DeVries K, Lyons EA, Ballard G, Levi CS, Lindsay D (1990) Contractions of the inner third of the myometrium. Am J Obstet Gynecol 162:679-682.
Dotan I, Werner L, Vigodman S, Weiss S, Brazowski E, Maharshak N, Chen O, Tulchinsky H, Halpem Z, Guzner-Gur H (2010) CXCL12 is a constitutive and inflammatory chemokine in the intestinal immune system. Inflamm Bowel Dis 16: 583-592
Du H1, Taylor HS.. (2007) Contribution of bone marrow-derived stem cells to endometrium and endometriosis.Stem Cells Aug;25(8):2082-6. Epub 2007 Apr 26
Ebert AD, Fuhr N, David M, Schneppel L, Papadopoulos T. (2009) Histological confirmation of endometriosis in a 9-year-old girl suffering from unexplained cyclic pelvic pain since her eighth year of life. Gynecol Obstet Invest 67:158-161
Einer-Jensen N (1988) Countercurrent transfer in the ovarian pedicle and its physiological implications. Oxford Rev Reprod Biol 10,348-381
20. Emge LA (1962) The elusive adenomyosis of the uterus. It's historical past and it's present state of recognition. Am J Obstet Gynecol 83:1541-1563
ESHRE Capri Workshop Group (2008). Genetic aspects of female reproduction Hum Reprod Update. Jul-Aug;14(4):293-307.
Fazleabas AT, Brudney A, Chai D, Langoi D, Bulun SE (2003). Steroid receptor and aromatase expression in baboon endometriotic lesions. Fertil Steril. Sep;80 Suppl 2:820-7.
Franceschini I1, Lomet D, Cateau M, Delsol G, Tillet Y, Caraty A. (2006) Kisspeptin immunoreactive cells of the ovine preoptic area and arcuate nucleus co-express estrogen receptor alpha. Neurosci Lett. Jul 3;401(3):225-230. Epub 2006 Apr 18.
Freund WA (1896) Klinische Notizen zu den voluminösen Adenomyomen des Uterus. In: Recklinghausen von, F. Die Adenomyomata und Cystadenomyomata des Uterus und der Tubenwandung: ihre Abkunft von Resten des Wolff'schen Körpers. August Hirschwald Verlag, Berlin
Fuchs AR, Behrens O, Maschek H, Kupsch E, Einspanier A (1998) Oxytocin and vasopressin receptors in human nonpregnant endometrium, myometrium and uterine myomas during menstrual cycle and early pregnancy: characterisation, cellular localisation and comparison with rhesus monkey. Hum. Reprod. Update 4:594-604,
Fujii S, Konishi, I, Mori T (1989) Smooth muscle differentiation at endometrio-myometrial junction. An ultrastructural study. Virch Archiv A Pathal. Anat. 414: 105-112
Garcia-Segura LM, Wozniak A, Azcoitia I, Rodriguez JR, Hutchison RE, Hutchison AB (1999) Aromatase expression by astrocytes after brain injury: Implications for local estrogen formation in brain repair. Neuroscience 89: 567-578
Garcia-Segura LM (2008) Aromatase in the brain: not just for reproduction anymore.. J Neuroendocrinol. Jun;20(6):705-12. Links
Gargett CE, Schwab KE, Deane JA (2016) Endometrial stem/progenitor cells: the first 10 years. Hum Reprod Update Mar-Apr;22(2):137-63. doi: 10.1093/humupd/dmv051. Epub 2015 Nov 9.
Garbern JC1, Mummery CL, Lee RT. (2013) Model systems for cardiovascular regenerative biology. Cold Spring Harb Perspect Med. Apr 1;3(4):a014019.
Gashaw I, Hastings JM, Jackson KS, Winterhager E, Fazleabas AT. (2006) Induced endometriosis in the baboon (Papio anubis) increases the expression of the proangiogenic factor CYR61 (CCN1) in eutopic and ectopic endometria. Biol Reprod. Jun;74(6):1060-6. Epub 2006 Feb 15.
Gilliver SC, Ashworth JJ, Ashcroft GS. (2007) The hormonal regulation of cutaneous wound healing. Clin Dermatol. Jan-Feb;25(1):56-62. Review.
Godwin JW, Pinto AR, Rosenthal NA (2013) Macrophages are required for adult salamander limb regeneration, PNAS 110: 9415-9420
Goodall J. R (1944) A study of endometriosis, endosalpingiosis, endocervicosis and peritoneo-ovarian sclerosis. A clinical and pathologic study (2nd edition). JB Lippincott Company, Philadelphia,
Gui Y, Zhang J, Yuan L, Lessey BA. (1999) Regulation of HOXA-10 and its expression in normal and abnormal Endometrium Mol Hum Reprod 5:866-873
Guo C, Zhu H, Huang W, Li S, Qu W, Liu Y, Tan A. (2010) Side population cells in the human decidua of early pregnancy exhibit stem/progenitor cell-like characteristics. Reprod Biomed Online. Dec;21(6):783-93. doi: 10.1016/j.rbmo.2010.07.010. Epub 2010 Aug 1.
Guo S.-W, Mao X, Ma Q, Liu X. (2013).Dysmenorrhea and its severity are associated with increased contractility and over-expression of oxytocin receptor (OTR) in women with symptomatic adenomyosis. Fertil Steril 99:231-240
Gurates B, Bulun SE. (2003) Endometriosis: the ultimate hormonal disease. Semin Reprod Med. May;21(2):125-34. Review.
Hadfield R M, Yudkin P L, Coe C L, Scheffler, J., Uno, H., Barlow, D. H., Kemnitz, J. W. Kennedy S H (1997). Risk factors for endometriosis in the rhesus monkey (Macaca mulatta): a case-control study. Hum Reprod Update 3:109-15.
Hadjiargyrou M, Ahrens W, Rubin CT. Temporal expression of the chondrogenic and angiogenic growth factor CYR61 during fracture repair (2000). J Bone Miner Res. Jun;15(6):1014-23.
Halme J, Hammond MG, Hulka JF, Raj SG, Talbert LM. (1984) Retrograde menstruation in healthy women and in patients with endometriosis. Obstet Gynecol. Aug;64(2):151-4.
Halme J, Becker S, Wing R. Accentuated cyclic activation of peritoneal macrophages in patients with endometriosis (1984). Am J Obstet Gynecol. Jan 1;148(1):85-90
Han SJ, Jung SY, Wu S-P, Hawkins SM, Park MJ, KYO S, Qin J, Lydon JP, Tsai SY, Tsai M-J, DeMayop FJ, O'Malley BW (2015) Estrogen receptor β modulates apoptosis complexes and the inflammasome to drive pathogenesis of endometriosis Cell 163: 960-974
Hansen T, Vulgaris S, Siggelkow W, Kirkpatrick CJ (2006) Massive adenomyosis in a patient with uterus septus completus. Zentralbl Gynäkol 128:153-156
Hapangama DK, Kamal AM, Bulmer JN (2015) Estrogen receptor β: the guardian of the endometrium Hum Reprod. Update 21: 174-193
Hauksson A, Akerlund M, Melin P (1988) Uterine blood flow and myometrial activity at menstruation, and the action of vasopressin and a synthetic antagonist. Br. J. Obstet. Gynaecol. 95:898-904
Hocking AM (2015) The role od chemokines in mesenchymal stem cell homing to wounds.Adv Wound Care (New Rochelle). 4:623-630. Review.
Hricak H, Alpers C, Crooks LE and Sheldon PE (1983) Magnetic resonance imaging of the female pelvis: Initial experience. Am J Rad 141,119-1128
Huang CC1, Orvis GD, Wang Y, Behringer RR. (2012) Stromal-to-epithelial transition during postpartum endometrial regeneration. PLoS One;7(8):e44285. doi: 10.1371/journal.pone.0044285. Epub 2012 Aug 27.
Hudelist G, Czerwenka K, Keckstein J, Haas C, Fink-Retter A, Gschwantler-Kaulich D, Kubista E, Singer CF. (2007) Expression of Aromatase and Estrogen Sulfotransferase in Eutopic and Ectopic Endometrium: Evidence for Unbalanced Estradiol Production in Endometriosis. Reproductive Sc, Vol. 14, No. 8, 798-805
Hufnagel D, Li F, Cosar E, Krikun G, Taylor HS. (2015) The Role of Stem Cells in the Etiology and Pathophysiology of Endometriosis. Semin Reprod Med. 33:333-340.
Hull ME, Moghissi KS, Magyar DF, Hayes MF (1986) Comparison of different treatment modalities of endometriosis in infertile women. Fertil. Steril. 47:40
Ibrahim MG, Chiantera V, Frangini S, Younes S, Köhler C, Taube ET, Plendl J, Mechsner S (2015) Ultramicro-trauma in the endometrial-myometrial junctional zone and pale cell migration in adenomyosis Fertil Steril 104: 1475-1483
Janssen EB1, Rijkers AC, Hoppenbrouwers K, Meuleman C, D'Hooghe TM. (2013)Prevalence of endometriosis diagnosed by laparoscopy in adolescents with dysmenorrhea or chronic pelvic pain: a systematic review. Hum Reprod Update. 19(5):570-82. doi: 10.1093/humupd/dmt016. Epub 2013 May 31
Jeffrey JE, Aspden RM (2007). Cyclooxygenase inhibition lowers prostaglandin E2 release from articular cartilage and reduces apoptosis but not proteoglycan degradation following an impact load in vitro. Arthritis Res Ther.(6):R129.
Kaiserman-Abramof, I.R. and Padykula, H.A. (1989) Ultrastructural epithelial zonation of the primate endometrium (rhesus monkey). Am. J. Anat., 184, 13-30.
Kennedy S, Bergqvist A, Chapron C, D'Hooghe T, Dunselman G, Greb R, Hummelshoj L, Prentice A, Saridogan E. ESHRE Special Interest Group for Endometriosis and Endometrium Guideline Development Group (2005) ESHRE guideline for the diagnosis and treatment of endometriosis Hum Reprod 20:2698-704
Kindermann G (1988) Endometriose: Wesen ubd Entstehung. In: Käser O, Friedber V, Ober KG, Thomsen K, Zander J (Hsg.) Gynäkologie und Geburtshilfe Band III. Teil 2 13.1-13.27 Thieme, Stuttgart
Kissler S, Schmidt M, Keller N, Wiegratz T, Tonn, T, Roth KW,Seifried E, Baumann R, Siebzehnruebl E, Leyendecker G, Kaufmann M. (2005) Real-time PCR analysis for estrogen receptor beta and progesterone receptor in menstrual blood samples - a new approach to a non-invasive diagnosis for endometriosis. Hum Reprod. 20 (suppl.): i179 (P-496)
Kissler S, Schmidt M, Keller N, Wiegratz I, Kohl J, Baumann R, Kunz G, Kaufmann M, Leyendecker G (2007) Real-Time PCR-Analyse für Ostrogen-Rezeptor beta, Progesteronrezeptor und P-450-Aromatase im Menstrualblut - eine Pilotstudie über die Bedeutung des basalen Endometriums in der Pathogenese der Endometriose Geburtshilfe Frauenheilkd 67 - A24 DOI: 10.1055/s-2007-989163
Kitawaki J, Noguchi T, Amatsu T, Maeda K, Tsukamoto K, Yamamoto T, Fushiki S, Osawa Y, Honjo H (1997) Expression of aromatase cytochrome P450 protein and messenger ribonucleic acid in human endometriotic and adenomyotic tissues but not in normal endometrium. Biol. Reprod. 57: 514-519
Konstantzinopoulos PA, Kominea A, Vandoros G, Sykiotis GP, Andricopoulos P, Varakis I, Sotiropoulos-Bonikou G, Papavassiliou AG (2003) Oestrogen receptor beta (ERbeta) is abundantly expressed in normal colonic mucosa, but declines in colon adenocarcinoma pralleling the tumour's differentiation. Eur J Cancer 39: 1251-1258
Kossmann, R. (1897) Die Abstammung der Drüsenschläuche in dem Uterus und in den Tuben. Arch Gynak 54:359-381
Kunz G, Beil D, Deininger H, Wildt L, Leyendecker G (1996) The dynamics of rapid sperm transport through the female genital tract. Evidence from vaginal sonography of uterine peristalsis (VSUP) and hysterosalpingoscintigraphy (HSSG). Hum. Reprod. 11:627-632
Kunz G, Noe M, Herbertz M and Leyendecker G. (1998a) Uterine peristalsis during the follicular phase of the menstrual cycle. Effects of oestrogen, antioestrogen and oxytocin. Hum Reprod. Update 4: 647-654
Kunz G, Herbertz M, Noe M and Leyendecker G. (1998b) Sonographic evidence of a direct impact of the ovarian dominant structure on uterine function during the menstrual cycle. Hum Reprod. Update 4: 667-672
Kunz G, Beil D, Huppert P and Leyendecker G. (2000) Structural abnormalities of the uterine wall in women with endometriosis and infertility visualized by vaginal sonography and magnetic resonance imaging. Hum Reprod 15: 76-82
Kunz G, Beil D, Huppert P, Noe M, Kissler S, & Leyendecker G. (2005) Adenomyosis in endometriosis - prevalence and impact on fertility. Evidence from magnetic resonance imaging. Hum Reprod 20: 2309-2316
Kunz G, Herbertz M, Beil D, Huppert P, Leyendecker G. (2007) Adenomyosis as a disorder of the early and late human reproductive period. Reprod Biomed. Online Dec; 15: 681-685
Laird SM, Widowson R, El-Sheilkhi M, Hall AJ, Li TC (2011) Expression of CXCL12 and CXCR4 in human endometrium; effects of CXCL12 on MMP production by human endometrial cells. Hum Reprod 26: 1144-1152
Lander AD, Kimble J, Clevers H, Fuchs E, Montarras D, Buckingham M, Calof AL, Trumpp A, Oskarsson T (2012). What does the concept of the stem cell niche really mean today? BMC Biol. Mar 9;10:19. doi: 10.1186/1741-7007-10-19
Larsen SB, Lundorf E, Forman A, Dueholm M. (2011) Adenomyosis and junctional zone changes in patients with endometriosis. Eur J Obstet Gynecol Reprod Biol.157:206-11
Lavaque E, Sierra A, Azcoitia I, Garcia-Segura LM (2006). Steroidogenic acute regulatory protein in the brain. Neuroscience. 138(3):741-7.
Leiva MC, Hasty LA and Lyttle CR (1994) Inflammatory changes of the endometrium in patients with minimal-to-moderate endometriosis. Fertil. Steril. 62:967-972
Leyendecker G, Wildt L. (1983) Induction of ovulation with chronic intermittent (pulsatile) administration of Gn-RH in women with hypothalamic amenorrhoea. J Reprod Fertil. Sep;69(1):397-409.
Leyendecker, G. Kunz G, Wildt, L, Beil D, Deininger H (1996) Uterine hyperperistalsis and dysperistalsis as dysfunctions of the mechanism of rapid sperm transport in patients with endometriosis and infertility. Hum. Reprod. 11:1542-1551.
Leyendecker G, Kunz G, Noe M, Herbertz M and Mall G. (1998) Endometriosis: A dysfunction and disease of the archimetra. Hum Reprod Update 4: 752-762
Leyendecker G, Kunz G, Noe M, Herbertz M, Beil, D, Huppert P, Mall G. (1999) Die Archimetra als neues morphologisch-funktionelles Konzept des Uterus sowie als Ort der Primärerkrankung bei Endometriose Reproduktionsmedizin 15: 356-371
Leyendecker G. Endometriosis is an entity with extreme pleiomorphism. (2000) Hum Reprod 15: 4-7 Leyendecker G, Herbertz M, Kunz G and Mall G (2002) Endometriosis results from the dislocation of basal endometrium. Hum Reprod 17:2725-2736
.Leyendecker G, Kunz G, Kissler S, Wildt L. (2006) Adenomyosis and reproduction. Best Pract Res Clin Obstet Gynaecol. 20:523-46.
Leyendecker G, Wildt L, Mall G. (2009) The pathophysiology of endometriosis and adenomyosis: tissue injury and repair Arch Gynecol Obstet 280:529-538
Leyendecker G, Wildt L (2011) A new concept of endometriosis and adenomyosis: Tissue injury and repair (TIAR). Hum Mol Biol Clin Invest 5:125-142
Leyendecker G, Wildt L. (2012) Neue Erkenntnisse zur Pathophysiologie von Endometriose und Adenomyose: Tissue Injury and Repair (TIAR). Fortschritte in der Endometrioseforschung. forMed. Exzellenzforschung in der Medizin. 3:12-23. ISSN 2191-9003.
Leyendecker G1, Bilgicyildirim A, Inacker M, Stalf T, Huppert P, Mall G, Böttcher B, Wildt L. (2015) Adenomyosis and endometriosis. Re-visiting their association and further insights into the mechanisms of auto-traumatisation. An MRI study. Arch Gynecol Obstet. Apr;291(4):917-32. doi: 10.1007/s00404-014-3437-8. Epub 2014 Sep 21
Li X, Guo SW (2014) Clinical profiles of 710 premenopausal women with adenomyosis who underwent hysterectomy. J. Ocstet Gynaecol Res :40:485-494
Liu Y, Tal R, Pluchino N, Mamillapalli R, Taylor HS (2017) Systemic administration of bone marrow-derived cells leads to better uterine engraftment than use of uterine-derived cells or local injection. J Cell Mol Med. Aug 7. doi: 10.1111/jcmm.13294. [Epub ahead of print]
Lyons EA, Taylor PJ, Zheng, XH, Ballard G, Levi CS, Kredentser JV (1991) Characterization of subendometrial myometrial contractions throughout the menstrual cycle in normal fertile women. Fertil Steril 55:771-775
Mäkäräinen L, (1988) Uterine contractions in endometriosis: effects of operative and danazol treatment. J. Obstet Gynecol 9,134-138
Maggi M, Magini A, Fiscella A, Giannini S, Fantoni G, Toffoletti F, Massi G, Serio M (1992) Sexsteroid modulation of neurohypophysial hormone receptors in human nonpregnant myometrium. J clin Endocrinol Metab 74: 385-392
Mahmood TA, Templeton A. (1990) Pathophysiology of mild endometriosis: review of literature. Hum Reprod. Oct; 5(7):765-84. Review.
Malinak LR, Buttram VC, Elias S, Simpson JL (1980) Heritable aspects of endometriosis II. Clinical characteristics of familial endometriosis. Am J Obstet Gynecol 127:332
Marsh EE & Laufer MR (2005). Endometriosis in premenarcheal girls who do not have an obstructive anomaly. Fertil Steril 83: 758-760
Marcoux S, Maheux R and Berube S (1997) Laparoscopic surgery in infertile women with minimal or mild endometriosis. Canadian Collaborative Group on Endometriosis. N Engl J Med 337,217-22.
Martinez-Conjero JA, Morgan M, Montesinos M, Fortuno S, Meseguer M, Simon C, Horcajados JA, Pellicer A (2011) Adenomyosis does not affect implantation, but is associated with miscarriage in patients undergoing oocyte donation Fertil Steril 96: 943-950
Matsuzaki S, Murakami t, Uehara S, Canis M, Sasano H, (2001) Expression of estrogen receptor alpha and beta in peritoneal and ovarian endometriosis. Fertil Steril. Jun;75(6):1198-205.
Mechsner S1, Weichbrodt M, Riedlinger WF, Bartley J, Kaufmann AM, Schneider A, Köhler (2008) Estrogen and progestogen receptor positive endometriotic lesions and disseminated cells in pelvic sentinel lymph nodes of patients with deep infiltrating rectovaginal endometriosis: a pilot study. Hum Reprod. Oct;23(10):2202-9. doi: 10.1093/humrep/den259. Epub 2008 Jul 16
Meyer R (1919) Über den Stand der Frage der Adenomyositis und Adenome im allgemeinen und insbesondere über Adenomyositis seroepithelialis und Adenomyometritis sarcomatosa. Zbl Gynäkol 43,745-750
Meyer, R, (1930) Adenomyosis, Adenofibrosis und Adenomyom. In: W. Stoeckel (ed) Handbuch der Gynäkologie. Sechster Band / Erste Hälfte; p 356 - 669, J.F. Bergmann, München
Moen MH, Muus KM (1991) Endometriosis in pregnant and non-pregnant women at tubal sterilisation. Hum. Reprod. 6:699
Moen MH. (1991)Is a long period without childbirth a risk factor for developing endometriosis? Hum Reprod. 6:1404-1407
Montgomery GW, Nyholt DR, Zhao ZZ, Treloar SA, Painter JN, Missmer SA, Kennedy SH, Zondervan KT (2008). The search for genes contributing to endometriosis risk. Hum Reprod Update. Sep-Oct;14(5):447-57. Epub 2008 Jun 5. Review.
Moridi I, Mamillapalli R, Cosar E, Ersoy GS, Taylor HS (2017) Bone marrow stem cell chemotactic activity is induce by elevated CXCL12 in endometriosis. Reprod Sc 24:526-533
Mowa CN, Hoch R, Montavon CL, Jesmin S, Hindman G, Hou G (2008). Estrogen enhances wound healing in the penis of rats. Biomed Res. Oct;29(5):267-70.
Nisolle M, Donnez J. (1997) Peritoneal endometriosis, ovarian endometriosis, and adenomyotic nodules of the rectovaginal septum are three different entities. Fertil Steril. Oct;68(4):585-96
Noe M, Kunz G, Herbertz M, Mall G and Leyendecker G (1999). The cyclic pattern of the immunocytochemical expression of oestrogen and progesterone receptors in human myometrial and endometrial layers: Characterisation of the endometrial-subendometrial unit. Hum Reprod 14: 101-110 Oike K., Obata S., Tagaki K., Matsuo K., Ishihara K., and Kikuchi S. (1988) Observation of endometrial movement with transvaginal sonography. J Ultrasound Med. 7: 99
Okkels H, Engle ET (1938) Studies on the finer structure of the uterine blood vessels of the macacus monkey. Acta Patho Microbiol Scand 15: 150-168
Osteen KG, Rogers WH, Gaire M, Hargrove JT, Gorstein F, Matrisian LM (1994) Stromal epithelial interaction mediates steroidal regulation of metalloproteinase expression in human endometrium. Proc Natl Acad Sci USA 91, 10129-10133
Otto K (1957) Über Vorkommen und Ätiologie der Adenomyosis uteri mit Berichten über zwei atypische Fälle. Zentralbl Gynäk 79,471-480
Padykula, H.A., Coles, L.G., Okulicz, W.C., Rapaport, S.I., Mc Cracken, J.A., King, N.W. jr, Longcope, C. and Kaiserman-Abramof, I.R. (1989) The basalis of the primate endometrium: a bifunctional germinal compartment. Biol. Reprod., 40, 681-690.
Parazzini F, Vercellini P, Panazza S, Chatenoud L, Oldani S, Crosignani PG (1997) Risk factors for adenomyosis. Hum Reprod 12, 1275-1279
Peters H. (1977) The human ovary in childhood and early maturity. Eur J Obstet Gynec Reprod Biol 9:137-144
Philipp E, Huber H. (1939) Die Entstehung der Endometriose, gleichzeitig ein Beitrag zur Pathologie des interstitiellen Tuben Abschnittes Zbl Gyn 63: 7-40
Ramsey EM (1989) Vascular anatomy In: Wynn RM and Jollie WP. Biology of the Uterus (second edition) Plenum Medical Book Company, New York
Recklinghausen von F. (1896) Die Adenomyomata und Cystadenomyomata des Uterus und der Tubenwandung: ihre Abkunft von Resten des Wolff'schen Körpers. August Hirschwald Verlag, Berlin
Reinhold C, Tafazoli F and Wang L (1998) Imaging features of adenomyosis. Hum Reprod Update 4,337-349
Ridley JH (1968) The histogenesis of endometriosis. Obstet Gynec Surv 23,1-35
Revised American Fertility Society classification of endometriosis. (1985) Fertil Steril 43:351-352 Rokitansky von K (1960) Uber Uterusdrüsen-Neubildung. Z. Gesellschaft Ärzte 16:577-581
Rogers PAW (1996) Structure and function od endometrial blood vessels Hum Reprod Update 2 : 57-62
Rudolph-Owen LA, Slayden OVD, Matrisian LM, Brenner RM (1998) Matrix metalloproteinase expression in macaca mulatta endometrium: Evidence for zone-specific regulatory tissue gradients. Biol Reprod 59, 1349-1368
Ruiz A1, Salvo VA, Ruiz LA, Baez P, Garcia M, Flores I. (2010) Basal and steroid hormone-regulated expression of CXCR4 in human endometrium and endometriosis. Reprod Sci. Oct;17(10):894-903. doi: 10.1177/1933719110379920. Epub 2010 Aug 18
Sakr S, Naqvi H, Komm B, Taylor HS (2014) Endometriosis impairs bone marrow-derived stem cell recruitment to the uterus Whereas Bazedoxifene treatment leads to endometriosis regression and improved stem cell engraftment Endocrinology 155: 1489-1497
Salamanca A, Beltran E (1995). Subendometrial contractility in menstrual phase visualised by transvaginal sonography in patients with endometriosis. Fertl. Steril. 64: 193 - 195
Sampson, J.A. (1922) The life history of ovarian hematomas (hemorrhagic cysts) of endometrial (Müllerian) type. Am J Obstet Gynecol 4: 451-512
Sampson, J.A., (1927) Peritoneal endometriosis due to the menstrual dissemination of endometrial tissue into the peritoneal cavity. Am. J. Obstet. Gynecol. 14: 422-429
Schwalm H, Dubrauszky V (1966) The structure of the musculature of the human uterus - muscles and connective tissue. Am. J. Obstet. Gynecol. 94:391-404
Shioyama R, Aoki Y, Ito H, Matsuta Y, Nagase K, Oyama N, Miwa Y, Akino H, Imamura Y, Yokoyama O (2008). Long-lasting breaches in the bladder epithelium lead to storage dysfunction with increase in bladder PGE2 levels in the rat. Am J Physiol Regul Integr Comp Physiol. Aug;295(2):R714-8
Sierra A, Lavaque E, Perez-Martin M, Azcoitia I, Hales DB, Garcia-Segura LM (2003). Steroidogenic acute regulatory protein in the rat brain: cellular distribution, developmental regulation and overexpression after injury. Eur J Neurosci. Sep;18(6):1458-67.
Simpson JL, Elias S, Melinak LR, Buttram VC (1980) Heritable aspects of endometriosis: I genetic studies. Am J Obstet Gynecol 137:327
Su HY, Chen CH, Gao HW, Liu JY. (2005) A bicornuate uterus with a unilateral cornual adenomyosis. Obstet Gynecol. 105:1191-3.
Stappenbeck TS1, Miyoshi H. (2009) The role of stromal stem cells in tissue regeneration and wound repair. Science. Jun 26;324(5935): 1666-9. doi: 10.1126/science.1172687
Takahashi K, Nagata H and Kitao M (1989). Clinical usefulness of determination of estradiol levels in the menstrual blood for patients with endometriosis. Acta Obstet Gynecol Jpn 41,1849-1850. Takebayashi A1, Kimura F, Kishi Y, Ishida M, Takahashi A, Yamanaka A, Wu D, Zheng L, Takahashi K, Suginami H, Murakami T (2014). Subpopulations of macrophages within eutopic endometrium of endometriosis patients. Am J Reprod Immunol. 2015 Mar;73(3):221-31. doi: 10.1111/aji.12331. Epub Oct 25
Takemura M, Nomura S, Kimura T, Inoue T, Onoue H, Azuma C, Saji F, Kitamura Y, Tanzawa O (1993) Expression and localization of oxytocin receptor gene in human uterine endometrium in relation to the menstrual cycle. Endocrinology 132: 1830-1835
Taylor AH, Al -Azzawi F. (2000) Immunolocalisation of oestrogen receptor beta in human tissues. J Mol Endocrinol. 24:145-55.
Taylor HS, Vanden Heuvel GB, Igarashi P. (1997) A conserved HOX axis in the mouse and human female reproductive system: Late establishment and persistent adult expression of the HOXA cluster genes. Biol Reprod. 57:1338-1345
Teicher BA, Fricker SP (2010) CXCL12 (SDF-1)/CXCR4 pathway in cancer. Clin Cancer Res. 16:2927-2931
Tummon IS, Maclin VM, Rachwanska E, Binor Z, Dmowski PW (1988) Occult ovulatory dysfunction in women with minimal endometriosis or unexplained infertility. Fertil Steril 50:716
Van den Bosch T1, Dueholm M2, Leone FP3, Valentin L4, Rasmussen CK2, Votino A5, Van Schoubroeck D1, Landolfo C6, Installé AJ7,8, Guerriero S9, Exacoustos C10, Gordts S11, Benacerraf B12, D'Hooghe T13, De Moor B7,8, Brölmann H14, Goldstein S15, Epstein E16, Bourne T1,17, Timmerman D1. (2015) Terms, definitions and measurements to describe sonographic features of myometrium and uterine masses: a consensus opinion from the Morphological Uterus Sonographic Assessment (MUSA) group. Ultrasound Obstet Gynecol. Sep;46(3):284-98. doi: 10.1002/uog.14806. Epub 2015 Aug 10
Wada-Hiraike O1, Imamov O, Hiraike H, Hultenby K, Schwend T, Omoto Y, Warner M, Gustafsson JA (2006) Role of estrogen receptor beta in colonic epithelium Proc Natl Acad Sci U S A. 103:2959-2964. Epub 2006 Feb 13
Wang X, Mamillapalli R, Mutlu L, Du H, Taylor HS (2015) Chemoattraction of bone arrow-derived stem cells towards human endometrial stromal cells is mediated by estradiol regulated CXCL12 and CXCR4 expression. Stem Cell Res 15: 14-22
Werth R, Grusdew W (1898) Untersuchungen über die Entwicklung und Morphologie der menschlichen Uterusmuskulatur. Arch. Gynäkol. 55:325-409.
Wetzstein R (1965) Der Uterusmuskel: Morphologie. Arch Gynecol 202,1-13
Wierman ME, Kisiljak-Vassiliades K, DO, Tobert S, (2011) Gonadotropin releasing hormone (GnRH) neurn migration: initiation, maintenance and cessation a critical steps to ensure normal reproductive function Front Neuroendocrinol 32: 43-52
Wildt L, Kissler S, Licht P, Becker W (1998) Sperm transport in the human female genital tract and its modulation by oxytocin ass assessed by hysterosalpingography, hysterotonography, electrohysterography and Doppler sonography. Hum Reprod Update 4,655-666
Wu Y1, Wang J, Scott PG, Tredget EE (2007) Bone marrow-derived stem cells in wound healing: a review. Wound Repair Regen. Sep-Oct;15 Suppl 1:S18-26.
Xu Y1, Zhu H1, Zhao D1, Tan J1. (2015) Endometrial stem cells: clinical application and pathological roles. Int J Clin Exp Med. Dec 15;8(12):22039-44. eCollection 2015
Yamamoto T, Noguchi T, Tamura T, Kitiwaki J, Okada H (1993) Evidence for oestrogen synthesis in adenomyotic tissues. Am. J. Obstet. Gynecol. 169: 734-738
Yang G, Im HJ, Wang JH (2005). Repetitive mechanical stretching modulates IL-1beta induced COX-2, MMP-1 expression, and PGE2 production in human patellar tendon fibroblasts. Gene. Dec 19;363:166-72. Epub 2005 Oct 13. Links
Zervomanolakis I, Ott HW, Müller J, Seeber BE, Friess SC, Mattle V, Virgolini I, Heute D, Wildt L (2009). Uterine mechanisms of ipsilateral directed spermatozoa transport: Evidence for a contribution of the utero-ovarian countercurrent system. Eur J Obstet Gynecol Reprod BiolMay; 144 Suppl 1:S45-9.
Zhang LX1, Shen LL2, Ge SH3, Wang LM3, Yu XJ4, Xu QC5, Yang PS3, Yang CZ6. (2015) Systemic BMSC homing in the regeneration of pulp-like tissue and the enhancing effect of stromal cell-derived factor-1 on BMSC homing. Int J Clin Exp Pathol. Sep 1;8(9):10261-71. eCollection Zhou WH, Wu X, Hu WD, Du MR (2015) Co-expression of CXCR4 and CXCR7 in human endometrial stromal cells is modulated by steroid hormones. Int J Clin Exp Pathol. Mar 1;8(3):2449-60. eCollection 2015
Zhu W1, Pao GM, Satoh A, Cummings G, Monaghan JR, Harkins TT, Bryant SV, Randal Voss S, Gardiner DM, Hunter (2012). Activation of germline-specific genes is required for limb regeneration in the Mexican axolotl. Dev Biol. Oct 1;370(1):42-51. doi: 10.1016/j.ydbio.2012.07.021. Epub 2012 Jul 27 Zingg HH, Rosen F, Chu K, Larcher A, Arslan AM, Richard S, Lefebvre D (1995) Oxytocin and oxytocin receptor gene expression in the uterus. Recent Progr. Hormone Res. 50:255-273
Zgraggen S, Huggenberger R, Kerl K, Detmar M (2014) An important role of the SDF-1/CXCR4 axis in chronic skin inflammation.PLoS One. Apr 2;9(4):e93665. doi: 10.1371/journal.pone.0093665. eCollection 2014

## Claims

1. Method for determining the presence of a tissue injury and repair (TIAR) process in the uterus of a subject as a marker for the presence of a preliminary stage or increased risk of developing a medical disorder associated with abnormal formation of endometrial tissue, comprising:
a) providing a sample of said patient,
b) determining a level of CXCL12 and/or CXCR4 in said sample,
c) wherein the level of CXCL12 and/or CXCR4 correlates with the presence of tissue injury and repair (TIAR) processes in the uterus of a subject.

2. Method according to claim 1, wherein the tissue injury and repair (TIAR) process comprises uterine auto-traumatization.

3. Method according to any one of the preceding claims, wherein the recruitment of bone marrow-derived stem cells, preferably mesenchymal stem cells, is evident in the uterus of said subject.

4. Method according to any one of the preceding claims, wherein the subject exhibits symptoms of dysmenorrhea.

5. Method according to any one of the preceding claims, wherein the medical disorder associated with abnormal formation of endometrial tissue is endometriosis.

6. Method according to any one of the preceding claims, wherein the medical disorder associated with abnormal formation of endometrial tissue is adenomyosis.

7. Method according to any one of the preceding claims, wherein the level of CXCL12 and/or CXCR4 positively correlates with the presence of a preliminary stage or increased risk of developing a medical disorder associated with abnormal formation of endometrial tissue.

8. Method according to any one of the preceding claims, wherein the sample comprises or consists of menstrual fluid.

9. Method according to any one of the preceding claims, wherein the sample comprises a cervical test specimen or cervical test smear.

10. Method according to any one of the preceding claims, wherein the method comprises a polymerase chain reaction (PCR) to determine a level of CXCL12 and/or CXCR4 in said sample, wherein said PCR comprises primers that hybridize with CXCL12 and/or CXCR4-encoding nucleic acid molecules according to one or more of SEQ ID NO 8-17.

11. Method according to any one of the preceding claims, wherein the method comprises an immunoassay using one or more antibodies that bind CXCL12 and/or CXCR4 according to one or more of SEQ ID NO 1-7 to determine a level of CXCL12 and/or CXCR4 in said sample.

12. Method according to any one of the preceding claims, wherein the sample is a menstrual fluid sample and said levels of CXCL12 and/or CXCR4 are elevated in said menstrual blood sample in comparison to a peripheral blood sample.

13. Method according to any one of the preceding claims, wherein the determination of an increased risk of developing a medical disorder associated with abnormal formation of endometrial tissue is conducted prior to the occurrence of abnormal formation of endometrial tissue (such as in endometriosis and/or adenomyosis) in said subject.

14. Method according to any one of the preceding claims, wherein the subject has been menstruating for a period of 3 years or less, preferably 2 years or less.

15. Method according to any one of the preceding claims, wherein the subject is of an age of 12-20, preferably 12, 13, 14, 15, 16 or 17.


SEQUENCE LISTING

<110>  Leyendecker, Gerhard

<120>  METHOD FOR DETERMINING A TISSUE INJURY AND REPAIR (TIAR) PROCESS
       ASSOCIATED WITH ABNORMAL FORMATION OF ENDOMETRIAL TISSUE

<130>  XII 2221/17EP

<160>  17

<170>  PatentIn version 3.5

<210>  1
<211>  140
<212>  PRT
<213>  Homo sapiens

<400>  1

Met Asn Ala Lys Val Val Val Val Leu Val Leu Val Leu Thr Ala Leu
1               5                   10                  15

Cys Leu Ser Asp Gly Lys Pro Val Ser Leu Ser Tyr Arg Cys Pro Cys
            20                  25                  30

Arg Phe Phe Glu Ser His Val Ala Arg Ala Asn Val Lys His Leu Lys
        35                  40                  45

Ile Leu Asn Thr Pro Asn Cys Ala Leu Gln Ile Val Ala Arg Leu Lys
    50                  55                  60

Asn Asn Asn Arg Gln Val Cys Ile Asp Pro Lys Leu Lys Trp Ile Gln
65                  70                  75                  80

Glu Tyr Leu Glu Lys Ala Leu Asn Asn Leu Ile Ser Ala Ala Pro Ala
                85                  90                  95

Gly Lys Arg Val Ile Ala Gly Ala Arg Ala Leu His Pro Ser Pro Pro
            100                 105                 110

Arg Ala Cys Pro Thr Ala Arg Ala Leu Cys Glu Ile Arg Leu Trp Pro
        115                 120                 125

Pro Pro Glu Trp Ser Trp Pro Ser Pro Gly Asp Val
    130                 135                 140


<210>  2
<211>  21
<212>  PRT
<213>  Homo sapiens

<400>  2

Met Asn Ala Lys Val Val Val Val Leu Val Leu Val Leu Thr Ala Leu
1               5                   10                  15

Cys Leu Ser Asp Gly
            20

<210>  3
<211>  72
<212>  PRT
<213>  Homo sapiens

<400>  3

Lys Pro Val Ser Leu Ser Tyr Arg Cys Pro Cys Arg Phe Phe Glu Ser
1               5                   10                  15

His Val Ala Arg Ala Asn Val Lys His Leu Lys Ile Leu Asn Thr Pro
            20                  25                  30

Asn Cys Ala Leu Gln Ile Val Ala Arg Leu Lys Asn Asn Asn Arg Gln
        35                  40                  45

Val Cys Ile Asp Pro Lys Leu Lys Trp Ile Gln Glu Tyr Leu Glu Lys
    50                  55                  60

Ala Leu Asn Lys Arg Phe Lys Met
65                  70

<210>  4
<211>  70
<212>  PRT
<213>  Homo sapiens

<400>  4

Val Ser Leu Ser Tyr Arg Cys Pro Cys Arg Phe Phe Glu Ser His Val
1               5                   10                  15

Ala Arg Ala Asn Val Lys His Leu Lys Ile Leu Asn Thr Pro Asn Cys
            20                  25                  30

Ala Leu Gln Ile Val Ala Arg Leu Lys Asn Asn Asn Arg Gln Val Cys
        35                  40                  45

Ile Asp Pro Lys Leu Lys Trp Ile Gln Glu Tyr Leu Glu Lys Ala Leu
    50                  55                  60

Asn Lys Arg Phe Lys Met
65                  70

<210>  5

<211>  65
<212>  PRT
<213>  Homo sapiens

<400>  5

Val Ser Leu Ser Tyr Arg Cys Pro Cys Arg Phe Phe Glu Ser His Val
1               5                   10                  15

Ala Arg Ala Asn Val Lys His Leu Lys Ile Leu Asn Thr Pro Asn Cys
            20                  25                  30

Ala Leu Gln Ile Val Ala Arg Leu Lys Asn Asn Asn Arg Gln Val Cys
        35                  40                  45

Ile Asp Pro Lys Leu Lys Trp Ile Gln Glu Tyr Leu Glu Lys Ala Leu
    50                  55                  60

Asn
65

<210>  6
<211>  352
<212>  PRT
<213>  Homo sapiens

<400>  6

Met Glu Gly Ile Ser Ile Tyr Thr Ser Asp Asn Tyr Thr Glu Glu Met
1               5                   10                  15

Gly Ser Gly Asp Tyr Asp Ser Met Lys Glu Pro Cys Phe Arg Glu Glu
            20                  25                  30

Asn Ala Asn Phe Asn Lys Ile Phe Leu Pro Thr Ile Tyr Ser Ile Ile
        35                  40                  45

Phe Leu Thr Gly Ile Val Gly Asn Gly Leu Val Ile Leu Val Met Gly
    50                  55                  60

Tyr Gln Lys Lys Leu Arg Ser Met Thr Asp Lys Tyr Arg Leu His Leu
65                  70                  75                  80

Ser Val Ala Asp Leu Leu Phe Val Ile Thr Leu Pro Phe Trp Ala Val
                85                  90                  95

Asp Ala Val Ala Asn Trp Tyr Phe Gly Asn Phe Leu Cys Lys Ala Val
            100                 105                 110

His Val Ile Tyr Thr Val Asn Leu Tyr Ser Ser Val Leu Ile Leu Ala
        115                 120                 125

Phe Ile Ser Leu Asp Arg Tyr Leu Ala Ile Val His Ala Thr Asn Ser
    130                 135                 140

Gln Arg Pro Arg Lys Leu Leu Ala Glu Lys Val Val Tyr Val Gly Val
145                 150                 155                 160

Trp Ile Pro Ala Leu Leu Leu Thr Ile Pro Asp Phe Ile Phe Ala Asn
                165                 170                 175

Val Ser Glu Ala Asp Asp Arg Tyr Ile Cys Asp Arg Phe Tyr Pro Asn
            180                 185                 190

Asp Leu Trp Val Val Val Phe Gln Phe Gln His Ile Met Val Gly Leu
        195                 200                 205

Ile Leu Pro Gly Ile Val Ile Leu Ser Cys Tyr Cys Ile Ile Ile Ser
    210                 215                 220

Lys Leu Ser His Ser Lys Gly His Gln Lys Arg Lys Ala Leu Lys Thr
225                 230                 235                 240

Thr Val Ile Leu Ile Leu Ala Phe Phe Ala Cys Trp Leu Pro Tyr Tyr
                245                 250                 255

Ile Gly Ile Ser Ile Asp Ser Phe Ile Leu Leu Glu Ile Ile Lys Gln
            260                 265                 270

Gly Cys Glu Phe Glu Asn Thr Val His Lys Trp Ile Ser Ile Thr Glu
        275                 280                 285

Ala Leu Ala Phe Phe His Cys Cys Leu Asn Pro Ile Leu Tyr Ala Phe
    290                 295                 300

Leu Gly Ala Lys Phe Lys Thr Ser Ala Gln His Ala Leu Thr Ser Val
305                 310                 315                 320

Ser Arg Gly Ser Ser Leu Lys Ile Leu Ser Lys Gly Lys Arg Gly Gly
                325                 330                 335

His Ser Ser Val Ser Thr Glu Ser Glu Ser Ser Ser Phe His Ser Ser
            340                 345                 350

<210>  7
<211>  356
<212>  PRT
<213>  Homo sapiens

<400> 7

Met Ser Ile Pro Leu Pro Leu Leu Gln Ile Tyr Thr Ser Asp Asn Tyr
1               5                   10                  15

Thr Glu Glu Met Gly Ser Gly Asp Tyr Asp Ser Met Lys Glu Pro Cys
            20                  25                  30

Phe Arg Glu Glu Asn Ala Asn Phe Asn Lys Ile Phe Leu Pro Thr Ile
        35                  40                  45

Tyr Ser Ile Ile Phe Leu Thr Gly Ile Val Gly Asn Gly Leu Val Ile
    50                  55                  60

Leu Val Met Gly Tyr Gln Lys Lys Leu Arg Ser Met Thr Asp Lys Tyr
65                  70                  75                  80

Arg Leu His Leu Ser Val Ala Asp Leu Leu Phe Val Ile Thr Leu Pro
                85                  90                  95

Phe Trp Ala Val Asp Ala Val Ala Asn Trp Tyr Phe Gly Asn Phe Leu
            100                 105                 110

Cys Lys Ala Val His Val Ile Tyr Thr Val Asn Leu Tyr Ser Ser Val
        115                 120                 125

Leu Ile Leu Ala Phe Ile Ser Leu Asp Arg Tyr Leu Ala Ile Val His
    130                 135                 140

Ala Thr Asn Ser Gln Arg Pro Arg Lys Leu Leu Ala Glu Lys Val Val
145                 150                 155                 160

Tyr Val Gly Val Trp Ile Pro Ala Leu Leu Leu Thr Ile Pro Asp Phe
                165                 170                 175

Ile Phe Ala Asn Val Ser Glu Ala Asp Asp Arg Tyr Ile Cys Asp Arg
            180                 185                 190

Phe Tyr Pro Asn Asp Leu Trp Val Val Val Phe Gln Phe Gln His Ile
        195                 200                 205

Met Val Gly Leu Ile Leu Pro Gly Ile Val Ile Leu Ser Cys Tyr Cys
    210                 215                 220

Ile Ile Ile Ser Lys Leu Ser His Ser Lys Gly His Gln Lys Arg Lys
225                 230                 235                 240

Ala Leu Lys Thr Thr Val Ile Leu Ile Leu Ala Phe Phe Ala Cys Trp

                245                 250                 255

Leu Pro Tyr Tyr Ile Gly Ile Ser Ile Asp Ser Phe Ile Leu Leu Glu
            260                 265                 270

Ile Ile Lys Gln Gly Cys Glu Phe Glu Asn Thr Val His Lys Trp Ile
        275                 280                 285

Ser Ile Thr Glu Ala Leu Ala Phe Phe His Cys Cys Leu Asn Pro Ile
    290                 295                 300

Leu Tyr Ala Phe Leu Gly Ala Lys Phe Lys Thr Ser Ala Gln His Ala
305                 310                 315                 320

Leu Thr Ser Val Ser Arg Gly Ser Ser Leu Lys Ile Leu Ser Lys Gly
                325                 330                 335

Lys Arg Gly Gly His Ser Ser Val Ser Thr Glu Ser Glu Ser Ser Ser
            340                 345                 350

Phe His Ser Ser
        355

<210> 8
<211> 1940
<212> DNA
<213> Homo sapiens

<400> 8
gccgcacttt cactctccgt cagccgcatt gcccgctcgg cgtccggccc ccgacccgcg 60

ctcgtccgcc cgcccgcccg cccgcccgcg ccatgaacgc caaggtcgtg gtcgtgctgg 120

tcctcgtgct gaccgcgctc tgcctcagcg acgggaagcc cgtcagcctg agctacagat 180

gcccatgccg attcttcgaa agccatgttg ccagagccaa cgtcaagcat ctcaaaattc 240

tcaacactcc aaactgtgcc cttcagattg tagcccggct gaagaacaac aacagacaag 300

tgtgcattga cccgaagcta aagtggattc aggagtacct ggagaaagct ttaaacaagt 360

aagcacaaca gccaaaaagg actttccgct agacccactc gaggaaaact aaaaccttgt 420

gagagatgaa agggcaaaga cgtgggggag ggggccttaa ccatgaggac caggtgtgtg 480

tgtggggtgg gcacattgat ctgggatcgg gcctgaggtt tgccagcatt tagaccctgc 540

atttatagca tacggtatga tattgcagct tatattcatc catgccctgt acctgtgcac 600

gttggaactt ttattactgg ggtttttcta agaaagaaat tgtattatca acagcatttt 660

caagcagtta gttccttcat gatcatcaca atcatcatca ttctcattct cattttttaa 720

atcaacgagt acttcaagat ctgaatttgg cttgtttgga gcatctcctc tgctcccctg 780

gggagtctgg gcacagtcag gtggtggctt aacagggagc tggaaaaagt gtcctttctt 840

cagacactga ggctcccgca gcagcgcccc tcccaagagg aaggcctctg tggcactcag 900

ataccgactg gggctgggcg ccgccactgc cttcacctcc tctttcaacc tcagtgattg 960

gctctgtggg ctccatgtag aagccactat tactgggact gtgctcagag acccctctcc 1020

cagctattcc tactctctcc ccgactccga gagcatgctt aatcttgctt ctgcttctca 1080

tttctgtagc ctgatcagcg ccgcaccagc cgggaagagg gtgattgctg gggctcgtgc 1140

cctgcatccc tctcctccca gggcctgccc cacagctcgg gccctctgtg agatccgtct 1200

ttggcctcct ccagaatgga gctggccctc tcctggggat gtgtaatggt ccccctgctt 1260

acccgcaaaa gacaagtctt tacagaatca aatgcaattt taaatctgag agctcgcttt 1320

gagtgactgg gttttgtgat tgcctctgaa gcctatgtat gccatggagg cactaacaaa 1380

ctctgaggtt tccgaaatca gaagcgaaaa aatcagtgaa taaaccatca tcttgccact 1440

accccctcct gaagccacag cagggtttca ggttccaatc agaactgttg gcaaggtgac 1500

atttccatgc ataaatgcga tccacagaag gtcctggtgg tatttgtaac tttttgcaag 1560

gcattttttt atatatattt ttgtgcacat ttttttttac gtttctttag aaaacaaatg 1620

tatttcaaaa tatatttata gtcgaacaat tcatatattt gaagtggagc catatgaatg 1680

tcagtagttt atacttctct attatctcaa actactggca atttgtaaag aaatatatat 1740

gatatataaa tgtgattgca gcttttcaat gttagccaca gtgtattttt tcacttgtac 1800

taaaattgta tcaaatgtga cattatatgc actagcaata aaatgctaat tgtttcatgg 1860

tataaacgtc ctactgtatg tgggaattta tttacctgaa ataaaattca ttagttgtta 1920

gtgatggagc ttaaaaaaaa 1940

<210> 9
<211> 3549
<212> DNA
<213> Homo sapiens

<400> 9
gccgcacttt cactctccgt cagccgcatt gcccgctcgg cgtccggccc ccgacccgcg 60

ctcgtccgcc cgcccgcccg cccgcccgcg ccatgaacgc caaggtcgtg gtcgtgctgg 120

tcctcgtgct gaccgcgctc tgcctcagcg acgggaagcc cgtcagcctg agctacagat 180

gcccatgccg attcttcgaa agccatgttg ccagagccaa cgtcaagcat ctcaaaattc 240

tcaacactcc aaactgtgcc cttcagattg tagcccggct gaagaacaac aacagacaag 300

tgtgcattga cccgaagcta aagtggattc aggagtacct ggagaaagct ttaaacaaga 360

ggttcaagat gtgagagggt cagacgcctg aggaaccctt acagtaggag cccagctctg 420

aaaccagtgt tagggaaggg cctgccacag cctcccctgc cagggcaggg ccccaggcat 480

tgccaagggc tttgttttgc acactttgcc atattttcac catttgatta tgtagcaaaa 540
tacatgacat ttatttttca tttagtttga ttattcagtg tcactggcga cacgtagcag 600
cttagactaa ggccattatt gtacttgcct tattagagtg tctttccacg gagccactcc 660
tctgactcag ggctcctggg ttttgtattc tctgagctgt gcaggtgggg agactgggct 720
gagggagcct ggccccatgg tcagccctag ggtggagagc caccaagagg gacgcctggg 780
ggtgccagga ccagtcaacc tgggcaaagc ctagtgaagg cttctctctg tgggatggga 840
tggtggaggg ccacatggga ggctcacccc cttctccatc cacatgggag ccgggtctgc 900
ctcttctggg agggcagcag ggctaccctg agctgaggca gcagtgtgag gccagggcag 960
agtgagaccc agccctcatc ccgagcacct ccacatcctc cacgttctgc tcatcattct 1020
ctgtctcatc catcatcatg tgtgtccacg actgtctcca tggccccgca aaaggactct 1080
caggaccaaa gctttcatgt aaactgtgca ccaagcagga aatgaaaatg tcttgtgtta 1140
cctgaaaaca ctgtgcacat ctgtgtcttg tttggaatat tgtccattgt ccaatcctat 1200
gtttttgttc aaagccagcg tcctcctctg tgaccaatgt cttgatgcat gcactgttcc 1260
ccctgtgcag ccgctgagcg aggagatgct ccttgggccc tttgagtgca gtcctgatca 1320
gagccgtggt cctttggggt gaactacctt ggttccccca ctgatcacaa aaacatggtg 1380
ggtccatggg cagagcccaa gggaattcgg tgtgcaccag ggttgacccc agaggattgc 1440
tgccccatca gtgctccctc acatgtcagt accttcaaac tagggccaag cccagcactg 1500
cttgaggaaa acaagcattc acaacttgtt tttggttttt aaaacccagt ccacaaaata 1560
accaatcctg gacatgaaga ttctttccca attcacatct aacctcatct tcttcaccat 1620
ttggcaatgc catcatctcc tgccttcctc ctgggccctc tctgctctgc gtgtcacctg 1680
tgcttcgggc ccttcccaca ggacatttct ctaagagaac aatgtgctat gtgaagagta 1740
agtcaacctg cctgacattt ggagtgttcc ccttccactg agggcagtcg atagagctgt 1800
attaagccac ttaaaatgtt cacttttgac aaaggcaagc acttgtgggt ttttgttttg 1860
tttttcattc agtcttacga atacttttgc cctttgatta aagactccag ttaaaaaaaa 1920
ttttaatgaa gaaagtggaa aacaaggaag tcaaagcaag gaaactatgt aacatgtagg 1980
aagtaggaag taaattatag tgatgtaatc ttgaattgta actgttcttg aatttaataa 2040
tctgtagggt aattagtaac atgtgttaag tattttcata agtatttcaa attggagctt 2100
catggcagaa ggcaaaccca tcaacaaaaa ttgtccctta aacaaaaatt aaaatcctca 2160
atccagctat gttatattga aaaaatagag cctgagggat ctttactagt tataaagata 2220
cagaactctt tcaaaacctt ttgaaattaa cctctcacta taccagtata attgagtttt 2280
cagtggggca gtcattatcc aggtaatcca agatatttta aaatctgtca cgtagaactt 2340
ggatgtacct gcccccaatc catgaaccaa gaccattgaa ttcttggttg aggaaacaaa 2400

catgacccta aatcttgact acagtcagga aaggaatcat ttctatttct cctccatggg 2460

agaaaataga taagagtaga aactgcaggg aaaattattt gcataacaat tcctctacta 2520

acaatcagct ccttcctgga gactgcccag ctaaagcaat atgcatttaa atacagtctt 2580

ccatttgcaa gggaaaagtc tcttgtaatc cgaatctctt tttgctttcg aactgctagt 2640

caagtgcgtc cacgagctgt ttactaggga tccctcatct gtccctccgg gacctggtgc 2700

tgcctctacc tgacactccc ttgggctccc tgtaacctct tcagaggccc tcgctgccag 2760

ctctgtatca ggacccagag gaaggggcca gaggctcgtt gactggctgt gtgttgggat 2820

tgagtctgtg ccacgtgttt gtgctgtggt gtgtccccct ctgtccaggc actgagatac 2880

cagcgaggag gctccagagg gcactctgct tgttattaga gattacctcc tgagaaaaaa 2940

ggttccgctt ggagcagagg ggctgaatag cagaaggttg cacctccccc aaccttagat 3000

gttctaagtc tttccattgg atctcattgg acccttccat ggtgtgatcg tctgactggt 3060

gttatcaccg tgggctccct gactgggagt tgatcgcctt tcccaggtgc tacacccttt 3120

tccagctgga tgagaatttg agtgctctga tccctctaca gagcttccct gactcattct 3180

gaaggagccc cattcctggg aaatattccc tagaaacttc caaatcccct aagcagacca 3240

ctgataaaac catgtagaaa atttgttatt ttgcaacctc gctggactct cagtctctga 3300

gcagtgaatg attcagtgtt aaatgtgatg aatactgtat tttgtattgt ttcaattgca 3360

tctcccagat aatgtgaaaa tggtccagga gaaggccaat tcctatacgc agcgtgcttt 3420

aaaaaataaa taagaaacaa ctctttgaga aacaacaatt tctactttga agtcatacca 3480

atgaaaaaat gtatatgcac ttataatttt cctaataaag ttctgtactc aaatgtagcc 3540

accaacagt 3549

<210> 10
<211> 517
<212> DNA
<213> Homo sapiens

<400> 10
gccgcacttt cactctccgt cagccgcatt gcccgctcgg cgtccggccc ccgacccgcg 60

ctcgtccgcc cgcccgcccg cccgcccgcg ccatgaacgc caaggtcgtg gtcgtgctgg 120

tcctcgtgct gaccgcgctc tgcctcagcg acgggaagcc cgtcagcctg agctacagat 180

gcccatgccg attcttcgaa agccatgttg ccagagccaa cgtcaagcat ctcaaaattc 240

tcaacactcc aaactgtgcc cttcagattg tagcccggct gaagaacaac aacagacaag 300

tgtgcattga cccgaagcta aagtggattc aggagtacct ggagaaagct ttaaacaagg 360

ggcgcagaga agaaaaagtg gggaaaaaag aaaagatagg aaaaaagaag cgacagaaga 420

agagaaaggc tgcccagaaa aggaaaaact agttatctgc cacctcgaga tggaccacag 480

ttcacttgct ctcggcgctt tgtaaatttg ctcgatc 517

<210> 11
<211> 1216
<212> DNA
<213> Homo sapiens

<400> 11
gccgcacttt cactctccgt cagccgcatt gcccgctcgg cgtccggccc ccgacccgcg 60

ctcgtccgcc cgcccgcccg cccgcccgcg ccatgaacgc caaggtcgtg gtcgtgctgg 120

tcctcgtgct gaccgcgctc tgcctcagcg acgggaagcc cgtcagcctg agctacagat 180

gcccatgccg attcttcgaa agccatgttg ccagagccaa cgtcaagcat ctcaaaattc 240

tcaacactcc aaactgtgcc cttcagattg tagcccggct gaagaacaac aacagacaag 300

tgtgcattga cccgaagcta aagtggattc aggagtacct ggagaaagct ttaaacaacc 360

tgatcagcgc cgcaccagcc gggaagaggg tgattgctgg ggctcgtgcc ctgcatccct 420

ctcctcccag ggcctgcccc acagctcggg ccctctgtga gatccgtctt tggcctcctc 480

cagaatggag ctggccctct cctggggatg tgtaatggtc cccctgctta cccgcaaaag 540

acaagtcttt acagaatcaa atgcaatttt aaatctgaga gctcgctttg agtgactggg 600

ttttgtgatt gcctctgaag cctatgtatg ccatggaggc actaacaaac tctgaggttt 660

ccgaaatcag aagcgaaaaa atcagtgaat aaaccatcat cttgccacta ccccctcctg 720

aagccacagc agggtttcag gttccaatca gaactgttgg caaggtgaca tttccatgca 780

taaatgcgat ccacagaagg tcctggtggt atttgtaact ttttgcaagg cattttttta 840

tatatatttt tgtgcacatt tttttttacg tttctttaga aaacaaatgt atttcaaaat 900

atatttatag tcgaacaatt catatatttg aagtggagcc atatgaatgt cagtagttta 960

tacttctcta ttatctcaaa ctactggcaa tttgtaaaga aatatatatg atatataaat 1020

gtgattgcag cttttcaatg ttagccacag tgtatttttt cacttgtact aaaattgtat 1080

caaatgtgac attatatgca ctagcaataa aatgctaatt gtttcatggt ataaacgtcc 1140

tactgtatgt gggaatttat ttacctgaaa taaaattcat tagttgttag tgatggagct 1200

taaaaaaaac tcctcc 1216

<210> 12
<211> 3139
<212> DNA
<213> Homo sapiens

<400> 12
gccgcacttt cactctccgt cagccgcatt gcccgctcgg cgtccggccc ccgacccgcg 60

ctcgtccgcc cgcccgcccg cccgcccgcg ccatgaacgc caaggtcgtg gtcgtgctgg 120

tcctcgtgct gaccgcgctc tgcctcagcg acgggaagcc cgtcagcctg agctacagat 180

gcccatgccg attcttcgaa agccattatt gtacttgcct tattagagtg tctttccacg 240

gagccactcc tctgactcag ggctcctggg ttttgtattc tctgagctgt gcaggtgggg 300

agactgggct gagggagcct ggccccatgg tcagccctag ggtggagagc caccaagagg 360

gacgcctggg ggtgccagga ccagtcaacc tgggcaaagc ctagtgaagg cttctctctg 420

tgggatggga tggtggaggg ccacatggga ggctcacccc cttctccatc cacatgggag 480

ccgggtctgc ctcttctggg agggcagcag ggctaccctg agctgaggca gcagtgtgag 540

gccagggcag agtgagaccc agccctcatc ccgagcacct ccacatcctc cacgttctgc 600

tcatcattct ctgtctcatc catcatcatg tgtgtccacg actgtctcca tggccccgca 660

aaaggactct caggaccaaa gctttcatgt aaactgtgca ccaagcagga aatgaaaatg 720

tcttgtgtta cctgaaaaca ctgtgcacat ctgtgtcttg tttggaatat tgtccattgt 780

ccaatcctat gtttttgttc aaagccagcg tcctcctctg tgaccaatgt cttgatgcat 840

gcactgttcc ccctgtgcag ccgctgagcg aggagatgct ccttgggccc tttgagtgca 900

gtcctgatca gagccgtggt cctttggggt gaactacctt ggttccccca ctgatcacaa 960

aaacatggtg ggtccatggg cagagcccaa gggaattcgg tgtgcaccag ggttgacccc 1020

agaggattgc tgccccatca gtgctccctc acatgtcagt accttcaaac tagggccaag 1080

cccagcactg cttgaggaaa acaagcattc acaacttgtt tttggttttt aaaacccagt 1140

ccacaaaata accaatcctg gacatgaaga ttctttccca attcacatct aacctcatct 1200

tcttcaccat ttggcaatgc catcatctcc tgccttcctc ctgggccctc tctgctctgc 1260

gtgtcacctg tgcttcgggc ccttcccaca ggacatttct ctaagagaac aatgtgctat 1320

gtgaagagta agtcaacctg cctgacattt ggagtgttcc ccttccactg agggcagtcg 1380

atagagctgt attaagccac ttaaaatgtt cacttttgac aaaggcaagc acttgtgggt 1440

ttttgttttg tttttcattc agtcttacga atacttttgc cctttgatta aagactccag 1500

ttaaaaaaaa ttttaatgaa gaaagtggaa aacaaggaag tcaaagcaag gaaactatgt 1560

aacatgtagg aagtaggaag taaattatag tgatgtaatc ttgaattgta actgttcttg 1620

aatttaataa tctgtagggt aattagtaac atgtgttaag tattttcata agtatttcaa 1680

attggagctt catggcagaa ggcaaaccca tcaacaaaaa ttgtccctta aacaaaaatt 1740

aaaatcctca atccagctat gttatattga aaaaatagag cctgagggat ctttactagt 1800

tataaagata cagaactctt tcaaaacctt ttgaaattaa cctctcacta taccagtata 1860

attgagtttt cagtggggca gtcattatcc aggtaatcca agatatttta aaatctgtca 1920

cgtagaactt ggatgtacct gcccccaatc catgaaccaa gaccattgaa ttcttggttg 1980

aggaaacaaa catgacccta aatcttgact acagtcagga aaggaatcat ttctatttct 2040

cctccatggg agaaaataga taagagtaga aactgcaggg aaaattattt gcataacaat 2100

tcctctacta acaatcagct ccttcctgga gactgcccag ctaaagcaat atgcatttaa 2160

atacagtctt ccatttgcaa gggaaaagtc tcttgtaatc cgaatctctt tttgctttcg 2220

aactgctagt caagtgcgtc cacgagctgt ttactaggga tccctcatct gtccctccgg 2280

gacctggtgc tgcctctacc tgacactccc ttgggctccc tgtaacctct tcagaggccc 2340

tcgctgccag ctctgtatca ggacccagag gaagggccca gaggctcgtt gactggctgt 2400

gtgttgggat tgagtctgtg ccacgtgttt gtgctgtggt gtgtccccct ctgtccaggc 2460

actgagatac cagcgaggag gctccagagg gcactctgct tgttattaga gattacctcc 2520

tgagaaaaaa ggttccgctt ggagcagagg ggctgaatag cagaaggttg cacctccccc 2580

aaccttagat gttctaagtc tttccattgg atctcattgg acccttccat ggtgtgatcg 2640

tctgactggt gttatcaccg tgggctccct gactgggagt tgatcgcctt tcccaggtgc 2700

tacacccttt tccagctgga tgagaatttg agtgctctga tccctctaca gagcttccct 2760

gactcattct gaaggagccc cattcctggg aaatattccc tagaaacttc caaatcccct 2820

aagcagacca ctgataaaac catgtagaaa atttgttatt ttgcaacctc gctggactct 2880

cagtctctga gcagtgaatg attcagtgtt aaatgtgatg aatactgtat tttgtattgt 2940

ttcaattgca tctcccagat aatgtgaaaa tggtccagga gaaggccaat tcctatacgc 3000

agcgtgcttt aaaaaataaa taagaaacaa ctctttgaga aacaacaatt tctactttga 3060

agtcatacca atgaaaaaat gtatatgcac ttataatttt cctaataaag ttctgtactc 3120

aaatgtagcc accaacagt 3139

<210> 13
<211> 1904
<212> DNA
<213> Homo sapiens

<400> 13
cttccctcta gtgggcgggg cagaggagtt agccaagatg tgactttgaa accctcagcg 60

tctcagtgcc cttttgttct aaacaaagaa ttttgtaatt ggttctacca aagaaggata 120

taatgaagtc actatgggaa aagatgggga ggagagttgt aggattctac attaattctc 180

ttgtgccctt agcccactac ttcagaattt cctgaagaaa gcaagcctga attggttttt 240

taaattgctt taaaaatttt ttttaactgg gttaatgctt gctgaattgg aagtgaatgt 300

ccattccttt gcctcttttg cagatataca cttcagataa ctacaccgag gaaatgggct 360

caggggacta tgactccatg aaggaaccct gtttccgtga agaaaatgct aatttcaata 420

aaatcttcct gcccaccatc tactccatca tcttcttaac tggcattgtg ggcaatggat 480

tggtcatcct ggtcatgggt taccagaaga aactgagaag catgacggac aagtacaggc 540

tgcacctgtc agtggccgac ctcctctttg tcatcacgct tcccttctgg gcagttgatg 600

ccgtggcaaa ctggtacttt gggaacttcc tatgcaaggc agtccatgtc atctacacag 660

tcaacctcta cagcagtgtc ctcatcctgg ccttcatcag tctggaccgc tacctggcca 720

tcgtccacgc caccaacagt cagaggccaa ggaagctgtt ggctgaaaag gtggtctatg 780

ttggcgtctg gatccctgcc ctcctgctga ctattcccga cttcatcttt gccaacgtca 840

gtgaggcaga tgacagatat atctgtgacc gcttctaccc caatgacttg tgggtggttg 900

tgttccagtt tcagcacatc atggttggcc ttatcctgcc tggtattgtc atcctgtcct 960

gctattgcat tatcatctcc aagctgtcac actccaaggg ccaccagaag cgcaaggccc 1020

tcaagaccac agtcatcctc atcctggctt tcttcgcctg ttggctgcct tactacattg 1080

ggatcagcat cgactccttc atcctcctgg aaatcatcaa gcaagggtgt gagtttgaga 1140

acactgtgca caagtggatt tccatcaccg aggccctagc tttcttccac tgttgtctga 1200

accccatcct ctatgctttc cttggagcca aatttaaaac ctctgcccag cacgcactca 1260

cctctgtgag cagagggtcc agcctcaaga tcctctccaa aggaaagcga ggtggacatt 1320

catctgtttc cactgagtct gagtcttcaa gttttcactc cagctaacac agatgtaaaa 1380

gacttttttt tatacgataa ataacttttt tttaagttac acatttttca gatataaaag 1440

actgaccaat attgtacagt ttttattgct tgttggattt ttgtcttgtg tttctttagt 1500

ttttgtgaag tttaattgac ttatttatat aaattttttt tgtttcatat tgatgtgtgt 1560

ctaggcagga cctgtggcca agttcttagt tgctgtatgt ctcgtggtag gactgtagaa 1620

aagggaactg aacattccag agcgtgtagt gaatcacgta aagctagaaa tgatccccag 1680

ctgtttatgc atagataatc tctccattcc cgtggaacgt ttttcctgtt cttaagacgt 1740

gattttgctg tagaagatgg cacttataac caaagcccaa agtggtatag aaatgctggt 1800

tttcagtttt tcaggagtgg gttgatttca gcacctacag tgtacagtct tgtattaagt 1860

tgttaataaa agtacatgtt aaacttaaaa aaaaaaaaaa aaaa 1904

<210> 14
<211> 1904
<212> DNA
<213> Homo sapiens

<400> 14
aacttcagtt tgttggctgc ggcagcaggt agcaaagtga cgccgagggc ctgagtgctc 60

cagtagccac cgcatctgga gaaccagcgg ttaccatgga ggggatcagt gaaaatgccc 120

cgctccctaa cgtcccaaac gcgccaagtg ataaacacga ggatggcaag agacccacac 180

accggaggag cgcccgcttg ggggaggagg tgccgtttgt tcattttctg acactcccgc 240

ccaatatacc ccaagcaccg aagggccttc gttttaagac cgcattctct ttacccacta 300

caagttgctt gaagcccaga atgatataca cttcagataa ctacaccgag gaaatgggct 360

caggggacta tgactccatg aaggaaccct gtttccgtga agaaaatgct aatttcaata 420

aaatcttcct gcccaccatc tactccatca tcttcttaac tggcattgtg ggcaatggat 480

tggtcatcct ggtcatgggt taccagaaga aactgagaag catgacggac aagtacaggc 540

tgcacctgtc agtggccgac ctcctctttg tcatcacgct tcccttctgg gcagttgatg 600

ccgtggcaaa ctggtacttt gggaacttcc tatgcaaggc agtccatgtc atctacacag 660

tcaacctcta cagcagtgtc ctcatcctgg ccttcatcag tctggaccgc tacctggcca 720

tcgtccacgc caccaacagt cagaggccaa ggaagctgtt ggctgaaaag gtggtctatg 780

ttggcgtctg gatccctgcc ctcctgctga ctattcccga cttcatcttt gccaacgtca 840

gtgaggcaga tgacagatat atctgtgacc gcttctaccc caatgacttg tgggtggttg 900

tgttccagtt tcagcacatc atggttggcc ttatcctgcc tggtattgtc atcctgtcct 960

gctattgcat tatcatctcc aagctgtcac actccaaggg ccaccagaag cgcaaggccc 1020

tcaagaccac agtcatcctc atcctggctt tcttcgcctg ttggctgcct tactacattg 1080

ggatcagcat cgactccttc atcctcctgg aaatcatcaa gcaagggtgt gagtttgaga 1140

acactgtgca caagtggatt tccatcaccg aggccctagc tttcttccac tgttgtctga 1200

accccatcct ctatgctttc cttggagcca aatttaaaac ctctgcccag cacgcactca 1260

cctctgtgag cagagggtcc agcctcaaga tcctctccaa aggaaagcga ggtggacatt 1320

catctgtttc cactgagtct gagtcttcaa gttttcactc cagctaacac agatgtaaaa 1380

gacttttttt tatacgataa ataacttttt tttaagttac acatttttca gatataaaag 1440

actgaccaat attgtacagt ttttattgct tgttggattt ttgtcttgtg tttctttagt 1500

ttttgtgaag tttaattgac ttatttatat aaattttttt tgtttcatat tgatgtgtgt 1560

ctaggcagga cctgtggcca agttcttagt tgctgtatgt ctcgtggtag gactgtagaa 1620

aagggaactg aacattccag agcgtgtagt gaatcacgta aagctagaaa tgatccccag 1680

ctgtttatgc atagataatc tctccattcc cgtggaacgt tttcctgttt cttaagacgt 1740

gattttgctg tagaagatgg cacttataac caaagcccaa agtggtatag aaatgctggt 1800

ttttcagttt tcaggagtgg gttgatttca gcacctacag tgtacagtct tgtattaagt 1860

tgttaataaa agtacatgtt aaacttaaaa aaaaaaaaaa aaaa 1904

<210> 15
<211> 1790
<212> DNA
<213> Homo sapiens

<400> 15
aacttcagtt tgttggctgc ggcagcaggt agcaaagtga cgccgagggc ctgagtgctc 60

cagtagccac cgcatctgga gaaccagcgg ttaccatgga ggggatcagt gaaaatgccc 120

cgctccctaa cgtcccaaac gcgccaagtg ataaacacga ggatggcaag agacccacac 180

accggaggag cgcccgcttg ggggaggaga tatacacttc agataactac accgaggaaa 240

tgggctcagg ggactatgac tccatgaagg aaccctgttt ccgtgaagaa aatgctaatt 300

tcaataaaat cttcctgccc accatctact ccatcatctt cttaactggc attgtgggca 360

atggattggt catcctggtc atgggttacc agaagaaact gagaagcatg acggacaagt 420

acaggctgca cctgtcagtg gccgacctcc tctttgtcat cacgcttccc ttctgggcag 480

ttgatgccgt ggcaaactgg tactttggga acttcctatg caaggcagtc catgtcatct 540

acacagtcaa cctctacagc agtgtcctca tcctggcctt catcagtctg gaccgctacc 600

tggccatcgt ccacgccacc aacagtcaga ggccaaggaa gctgttggct gaaaaggtgg 660

tctatgttgg cgtctggatc cctgccctcc tgctgactat tcccgacttc atctttgcca 720

acgtcagtga ggcagatgac agatatatct gtgaccgctt ctaccccaat gacttgtggg 780

tggttgtgtt ccagtttcag cacatcatgg ttggccttat cctgcctggt attgtcatcc 840

tgtcctgcta ttgcattatc atctccaagc tgtcacactc caagggccac cagaagcgca 900

aggccctcaa gaccacagtc atcctcatcc tggctttctt cgcctgttgg ctgccttact 960

acattgggat cagcatcgac tccttcatcc tcctggaaat catcaagcaa gggtgtgagt 1020

ttgagaacac tgtgcacaag tggatttcca tcaccgaggc cctagctttc ttccactgtt 1080

gtctgaaccc catcctctat gctttccttg gagccaaatt taaaacctct gcccagcacg 1140

cactcacctc tgtgagcaga gggtccagcc tcaagatcct ctccaaagga aagcgaggtg 1200

gacattcatc tgtttccact gagtctgagt cttcaagttt tcactccagc taacacagat 1260

gtaaaagact tttttttata cgataaataa ctttttttta agttacacat ttttcagata 1320

taaaagactg accaatattg tacagttttt attgcttgtt ggatttttgt cttgtgtttc 1380

tttagttttt gtgaagttta attgacttat ttatataaat tttttttgtt tcatattgat 1440

gtgtgtctag gcaggacctg tggccaagtt cttagttgct gtatgtctcg tggtaggact 1500

gtagaaaagg gaactgaaca ttccagagcg tgtagtgaat cacgtaaagc tagaaatgat 1560

ccccagctgt ttatgcatag ataatctctc cattcccgtg gaacgttttt cctgttctta 1620

agacgtgatt ttgctgtaga agatggcact tataaccaaa gcccaaagtg gtatagaaat 1680

gctggttttt cagttttcag gagtgggttg atttcagcac ctacagtgta cagtcttgta 1740

ttaagttgtt aataaaagta catgttaaac ttaaaaaaaa aaaaaaaaaa 1790

<210> 16
<211> 1699
<212> DNA

<213>  Homo sapiens

<400>  16
gagttacatt gtctgaattt agaggcggag ggcggcgtgc ctgggctgag ttcccaggag      60

gagattgcgc ccgctttaac ttcggggtta agcgcctggt gactgttctt gacactggat     120

atacacttca gataactaca ccgaggaaat gggctcaggg gactatgact ccatgaagga     180

accctgtttc cgtgaagaaa atgctaattt caataaaatc ttcctgccca ccatctactc     240

catcatcttc ttaactggca ttgtgggcaa tggattggtc atcctggtca tgggttacca     300

gaagaaactg agaagcatga cggacaagta caggctgcac ctgtcagtgg ccgacctcct     360

ctttgtcatc acgcttccct tctgggcagt tgatgccgtg gcaaactggt actttgggaa     420

cttcctatgc aaggcagtcc atgtcatcta cacagtcaac ctctacagca gtgtcctcat     480

cctggccttc atcagtctgg accgctacct ggccatcgtc cacgccacca acagtcagag     540

gccaaggaag ctgttggctg aaaaggtggt ctatgttggc gtctggatcc ctgccctcct     600

gctgactatt cccgacttca tctttgccaa cgtcagtgag gcagatgaca gatatatctg     660

tgaccgcttc taccccaatg acttgtgggt ggttgtgttc cagtttcagc acatcatggt     720

tggccttatc ctgcctggta ttgtcatcct gtcctgctat tgcattatca tctccaagct     780

gtcacactcc aagggccacc agaagcgcaa ggccctcaag accacagtca tcctcatcct     840

ggctttcttc gcctgttggc tgccttacta cattgggatc agcatcgact ccttcatcct     900

cctggaaatc atcaagcaag ggtgtgagtt tgagaacact gtgcacaagt ggatttccat     960

caccgaggcc ctagctttct tccactgttg tctgaacccc atcctctatg ctttccttgg    1020

agccaaattt aaaacctctg cccagcacgc actcacctct gtgagcagag ggtccagcct    1080

caagatcctc tccaaaggaa agcgaggtgg acattcatct gtttccactg agtctgagtc    1140

ttcaagtttt cactccagct aacacagatg taaaagactt ttttttatac gataaataac    1200

ttttttttaa gttacacatt tttcagatat aaaagactga ccaatattgt acagttttta    1260

ttgcttgttg gatttttgtc ttgtgtttct ttagtttttg tgaagtttaa ttgacttatt    1320

tatataaatt ttttttgttt catattgatg tgtgtctagg caggacctgt ggccaagttc    1380

ttagttgctg tatgtctcgt ggtaggactg tagaaaaggg aactgaacat tccagagcgt    1440

gtagtgaatc acgtaaagct agaaatgatc cccagctgtt tatgcataga taatctctcc    1500

attcccgtgg aacgtttttc ctgttcttaa gacgtgattt tgctgtagaa gatggcactt    1560

ataaccaaag cccaaagtgg tatagaaatg ctggtttttc agttttcagg agtgggttga    1620

tttcagcacc tacagtgtac agtcttgtat taagttgtta ataaaagtac atgttaaact    1680

taaaaaaaaa aaaaaaaaa                                                 1699

<210>  17

<211>  1691
<212>  DNA
<213>  Homo sapiens

<400>  17
aacttcagtt tgttggctgc ggcagcaggt agcaaagtga cgccgagggc ctgagtgctc      60

cagtagccac cgcatctgga gaaccagcgg ttaccatgga ggggatcagt atatacactt     120

cagataacta caccgaggaa atgggctcag gggactatga ctccatgaag gaaccctgtt     180

tccgtgaaga aaatgctaat ttcaataaaa tcttcctgcc caccatctac tccatcatct     240

tcttaactgg cattgtgggc aatggattgg tcatcctggt catgggttac cagaagaaac     300

tgagaagcat gacggacaag tacaggctgc acctgtcagt ggccgacctc ctctttgtca     360

tcacgcttcc cttctgggca gttgatgccg tggcaaactg gtactttggg aacttcctat     420

gcaaggcagt ccatgtcatc tacacagtca acctctacag cagtgtcctc atcctggcct     480

tcatcagtct ggaccgctac ctggccatcg tccacgccac caacagtcag aggccaagga     540

agctgttggc tgaaaaggtg gtctatgttg gcgtctggat ccctgccctc ctgctgacta     600

ttcccgactt catctttgcc aacgtcagtg aggcagatga cagatatatc tgtgaccgct     660

tctaccccaa tgacttgtgg gtggttgtgt tccagtttca gcacatcatg gttggcctta     720

tcctgcctgg tattgtcatc ctgtcctgct attgcattat catctccaag ctgtcacact     780

ccaagggcca ccagaagcgc aaggccctca agaccacagt catcctcatc ctggctttct     840

tcgcctgttg gctgccttac tacattggga tcagcatcga ctccttcatc ctcctggaaa     900

tcatcaagca agggtgtgag tttgagaaca ctgtgcacaa gtggatttcc atcaccgagg     960

ccctagcttt cttccactgt tgtctgaacc ccatcctcta tgctttcctt ggagccaaat    1020

ttaaaacctc tgcccagcac gcactcacct ctgtgagcag agggtccagc ctcaagatcc    1080

tctccaaagg aaagcgaggt ggacattcat ctgtttccac tgagtctgag tcttcaagtt    1140

ttcactccag ctaacacaga tgtaaaagac ttttttttat acgataaata actttttttt    1200

aagttacaca tttttcagat ataaaagact gaccaatatt gtacagtttt tattgcttgt    1260

tggatttttg tcttgtgttt ctttagtttt tgtgaagttt aattgactta tttatataaa    1320

ttttttttgt ttcatattga tgtgtgtcta ggcaggacct gtggccaagt tcttagttgc    1380

tgtatgtctc gtggtaggac tgtagaaaag ggaactgaac attccagagc gtgtagtgaa    1440

tcacgtaaag ctagaaatga tccccagctg tttatgcata gataatctct ccattcccgt    1500

ggaacgtttt tcctgttctt aagacgtgat tttgctgtag aagatggcac ttataaccaa    1560

agcccaaagt ggtatagaaa tgctggtttt tcagttttca ggagtgggtt gatttcagca    1620

cctacagtgt acagtcttgt attaagttgt taataaaagt acatgttaaa cttaaaaaaa    1680

aaaaaaaaaa a                                                          1691